# EUROPEAN PATENT APPLICATION

(11) **EP 4 647 771 A1**
(43) Date of publication of application: **12.11.2025**
(21) Application number: 23912600.6
(22) Date of filing: 24.11.2023
(51) Int. Cl.: G01N 33/573, G01N 33/544, G01N 33/543, C07K 16/40, C12N 15/10, C12Q 1/6806

(54) **ANTIBODY OR PEPTIDE BINDING SPECIFICALLY TO RNASE A, IMMUNOASSAY DEVICE AND METHOD FOR DETECTING RNASE A, AND METHOD FOR COLLECTING RNASE A**

(30) Priority: 30.12.2022 KR 20220190498; 20.01.2023 KR 20230008774
(71) Applicant: UIF (University Industry Foundation), Yonsei University, Seoul 16499 (KR)
(72) Inventor: PYUN, Jae Chul, Seoul 06521 (KR)
(74) Representative: Laine IP Oy
(86) International application number: PCT/KR2023/019083
(87) International publication number: WO 2024/143925

(57) **Abstract**

The present invention relates to an antibody or peptide that specifically binds to RNase A. The invention can effectively inhibit RNase A and can therefore be usefully applied in various fields such as RNA extraction methods, RNA purification methods, PCR, cDNA synthesis methods, and DNA expression methods. An RNase detection immunoassay device is disclosed. The RNase detection immunoassay device includes a substrate; a binding antibody; a peptide compound and a switching peptide; and a quenching material, wherein the binding antibody specifically binds to RNase A and includes one selected from the group consisting of CDR3 comprising the amino acid sequence of SEQ ID NO: 5, CDR3 comprising the amino acid sequence of SEQ ID NO: 6, and CDR3 comprising the amino acid sequence of SEQ ID NO: 7.

## Description

### [Technical Field]

The present invention relates to an antibody or peptide that specifically binds to RNase A.

The present invention relates to an immunoassay device and method for detecting RNase A from a sample using an antibody or peptide that specifically binds to RNase A, as well as a method for capturing RNase A from the sample.

### [Background Art]

Bovine pancreatic RNase A (RNase A, EC 3.1.27.5) is a pancreatic enzyme that cleaves single-stranded RNA. This enzyme is a basic protein (pI=9.63) composed of 124 amino acids (13.7 kDa). RNase A has a two-layer protein structure consisting of an alpha-helix (from the N-terminal half) and a beta-hairpin (from the C-terminal half) with a deep cleft for binding RNA substrates. Additionally, four disulfide bonds create an essential structural fold for RNase activity. The RNA cleavage mechanism has been reported to involve RNase A hydrolyzing the 3' end of pyrimidine residues in RNA and specifically cleaving the phosphodiester bond to adjacent nucleotides. The first step is known to be carried out through a nucleophilic attack by the 2'-oxygen on the electrophilic phosphorus atom, leading to the formation of a pentavalent intermediate. The second step involves RNA cleavage through the decomposition of the 2',3'-cyclic phosphate intermediate. Both steps are facilitated by electron transfer mediated by histidine residues (His12 and His119). RNase inhibitors (hereinafter referred to as "RI") that inhibit RNase activity have been developed using small molecules based on modified nucleotides and nucleotide mimics, as well as proteins. As a protein-based RI, a leucine-rich protein (49 kDa) has been used to protect cytoplasmic RNA from RNase activity. Uridine 2',3'-cyclic vanadate (U>v) and adenosine 5'-pyrophosphate (ppA-3'-p, ppA-2'-p) dinucleotide derivatives are well-known nucleotide-based RIs. It is reported that over 18,000 compounds, such as those in Patent Document 1, have been registered with the National Cancer Institute as non-nucleotide-based RIs.

### [Prior art literature]

### [Patent Document]

(Patent Document 1) U.S. Patent No. 9693999 B2 (July 4, 2017)

### [Detailed Description of the Invention]

### [Technical Problem]

The problem to be solved by the present invention is to provide antibodies and peptides that can efficiently inhibit RNase A.

The problem to be solved by the present invention is to discover and provide RNase A inhibitors by screening an Fv-antibody library.

The problem to be solved by the present invention is to provide an immunoassay device and method capable of quantitatively analyzing RNase A contained in a sample.

The problem to be solved by the present invention is to provide a method for capturing RNase A contained in a sample.

### [Technical Solution]

The antibody or an antigen-binding fragment that specifically binds to RNase A according to the present invention includes those selected from the group consisting of CDR3 comprising the amino acid sequence of SEQ ID NO: 5, CDR3 comprising the amino acid sequence of SEQ ID NO: 6, and CDR3 comprising the amino acid sequence of SEQ ID NO: 7 as a technical feature.

The peptide that specifically binds to RNase A according to the present invention is characterized in that it comprises an amino acid sequence selected from SEQ ID NO: 5, SEQ ID NO: 6, and SEQ ID NO: 7.

In addition, the present invention is characterized by comprising a peptide consisting of an amino acid sequence selected from SEQ ID NO: 5, SEQ ID NO: 6, and SEQ ID NO: 7 as an RNase A inhibitor.

An RNase detection immunoassay device according to an embodiment of the present invention comprises: a substrate having a reaction space that can accommodate a detection sample solution; a binding antibody located within the reaction space and fixed to the substrate; a switching peptide comprising a peptide compound reversibly bound to the Fab region of the binding antibody and a fluorescent label bound to the peptide compound; and a quenching material arranged adjacent to the fluorescent label to quench the fluorescence from the fluorescent label, wherein the binding antibody can specifically bind to RNase A and includes any one selected from the group consisting of CDR3 comprising the amino acid sequence of SEQ ID NO: 5, CDR3 comprising the amino acid sequence of SEQ ID NO: 6, and CDR3 comprising the amino acid sequence of SEQ ID NO: 7.

An RNase detection immunoassay device according to another embodiment of the present invention comprises: a substrate having a reaction space that can accommodate a detection sample solution; a support located within the reaction space; a binding antibody coupled to the support; a switching peptide comprising a peptide compound reversibly bound to the Fab region of the binding antibody and a fluorescent label bound to the peptide compound; and a quenching material arranged adjacent to the fluorescent label to quench the fluorescence from the fluorescent label. The binding antibody can specifically bind to RNase A and includes any one selected from the group consisting of CDR3 comprising the amino acid sequence of SEQ ID NO: 5, CDR3 comprising the amino acid sequence of SEQ ID NO: 6, and CDR3 comprising the amino acid sequence of SEQ ID NO: 7.

In one embodiment, the binding antibody may comprise a heavy chain variable region that includes CDR1 comprising the amino acid sequence of SEQ ID NO: 3, CDR2 comprising the amino acid sequence of SEQ ID NO: 4, and CDR3 comprising the amino acid sequence of SEQ ID NO: 5; a heavy chain variable region that includes CDR1 comprising the amino acid sequence of SEQ ID NO: 3, CDR2 comprising the amino acid sequence of SEQ ID NO: 4, and CDR3 comprising the amino acid sequence of SEQ ID NO: 6; and a heavy chain variable region that includes CDR1 comprising the amino acid sequence of SEQ ID NO: 3, CDR2 comprising the amino acid sequence of SEQ ID NO: 4, and CDR3 comprising the amino acid sequence of SEQ ID NO: 7, selected from the group consisting of any one of these.

In one embodiment, the peptide compound may have an amino acid sequence that can specifically and reversibly bind to one or more of the first to fourth framework regions of the light chain or heavy chain of the binding antibody.

In one embodiment, the binding antibody may comprise one selected from the group consisting of a heavy chain variable region that includes the amino acid sequence of SEQ ID NO: 8; a heavy chain variable region that includes the amino acid sequence of SEQ ID NO: 9; and a heavy chain variable region that includes the amino acid sequence of SEQ ID NO: 10.

In one embodiment, the binding antibody may be in the form of a single-domain antibody.

In one embodiment, the RNase detection immunoassay device may further comprise a light source that irradiates the sample solution accommodated in the reaction space of the substrate; and an image analysis device that receives the fluorescence generated from the switching peptide liberated from the binding antibody to produce an image thereof, and analyzes the image to quantify the amount of the switching peptide liberated from the binding antibody.

In one embodiment, the fluorescent label may comprise one or more selected from the group consisting of rhodamine and its derivatives, fluorescein and its derivatives, coumarin and its derivatives, acridine and its derivatives, pyrene and its derivatives, erythrosin and its derivatives, eosin and its derivatives, 4-acetamido-4'-isothiocyanatostilbene-2,2'-disulfonic acid, fluorescein isothiocyanate (FITC), Oregon Green, Alex Fluor, carboxyfluorescein (FAM), 6-carboxy-4',5'-dichloro-2',7'-dimethoxyfluorescein (JOE), carboxy-X-rhodamine (ROX), 6-carboxy-2',4,4',5',7,7'-hexachlorofluorescein (HEX), Texas Red (sulforhodamine 101 acid chloride), 6-carboxy-2',4,7',7-tetrachlorofluorescein (TET), tetramethylrhodamine isothiocyanate (TRITC), carboxytetramethylrhodamine (TAMRA), cyanine series dyes, and cyadicarbocyanine dyes.

In one embodiment, the quenching material may comprise one or more selected from the group consisting of 4-(dimethylamino)azobenzene-4-carboxylic acid (DABCYL), 4-(dimethylamino)azobenzenesulfonic acid (DABSYL), blackhole quencher (BHQ), blackberry quencher (BBQ), ECLIPSE quencher, Tide quencher, carbon nanomaterials, and manganese dioxide nanomaterials.

The RNase detection immunoassay method according to an embodiment of the present invention includes a first step of fixing a binding antibody, which is coupled to a switching peptide, to a substrate, or fixing the binding antibody to the substrate and then coupling the switching peptide to the binding antibody, and binding a quenching material to the binding antibody or the substrate; a second step of treating the binding antibody with a detection sample solution; and a third step of irradiating the detection sample solution after the treatment and quantitatively analyzing the target antigen in the detection sample solution through the fluorescence generated from the fluorescent label of the switching peptide released from the binding antibody. The binding antibody includes one selected from the group consisting of CDR3 comprising the amino acid sequence of SEQ ID NO: 5, CDR3 comprising the amino acid sequence of SEQ ID NO: 6, and CDR3 comprising the amino acid sequence of SEQ ID NO: 7, which specifically binds to RNase A, and the switching peptide may include a peptide compound having an amino acid sequence that can selectively and reversibly bind to the Fab (Fragment antigen-binding) region of the binding antibody, and a fluorescent label material that binds to it and emits fluorescence.

The RNase detection immunoassay method according to another embodiment of the present invention includes a first step of fixing a binding antibody, which is coupled to a switching peptide, to a support, or fixing the binding antibody to the support and then coupling the switching peptide to the binding antibody, and coupling a quenching material to the binding antibody or the support; a second step of injecting the support with the bound binding antibody and the detection sample solution into the reaction space of a substrate; and a third step of quantitatively analyzing the target antigen in the detection sample solution through the fluorescence generated from the fluorescent label of the switching peptide released from the binding antibody after irradiating the detection sample solution with light. The binding antibody includes one selected from the group consisting of CDR3 comprising the amino acid sequence of SEQ ID NO: 5, CDR3 comprising the amino acid sequence of SEQ ID NO: 6, and CDR3 comprising the amino acid sequence of SEQ ID NO: 7, which specifically binds to RNase A, and the switching peptide may include a peptide compound having an amino acid sequence that can selectively and reversibly bind to the Fab (Fragment antigen-binding) region of the binding antibody, and a fluorescent label material that binds to it and emits fluorescence.

In one embodiment, when RNase A is present in the detection sample solution in the second step, the switching peptide can be quantitatively released from the binding antibody depending on the amount of RNase A that has reacted with the binding antibody.

The RNase A capturing method according to an embodiment of the present invention comprises the step of mixing beads on which an antibody or peptide that specifically binds to RNase A is fixed on the surface with a sample containing RNase A to induce a reaction between the RNase A and the antibody or peptide; and the step of collecting the beads from the sample. The binding antibody may include any one selected from the group consisting of CDR3 comprising the amino acid sequence of SEQ ID NO: 5, CDR3 comprising the amino acid sequence of SEQ ID NO: 6, and CDR3 comprising the amino acid sequence of SEQ ID NO: 7, and can specifically bind to RNase A.

In one embodiment, the beads may include magnetic particles containing nickel (Ni), copper (Cu), zinc (Zn), or cobalt (Co) with chelating groups such as NTA (nitrilotriacetic acid) or IDA (iminodiacetic acid) modified on their surface. In this case, the beads can be collected from the sample using a magnetic field.

### [Effects of the Invention]

The present invention can effectively inhibit RNase A, and accordingly, it can be usefully applied in various fields such as RNA extraction methods, RNA purification methods, PCR (polymerase chain reaction), cDNA (complementary DNA) synthesis methods, and DNA expression methods.

According to the RNase detection immunoassay device and method of the present invention, by using an antibody that specifically binds to RNase A and a switching peptide bound to it, it is possible to perform quantitative analysis of RNase A in a one-step manner after reacting the detection sample solution with the binding antibody, without requiring a washing process to remove unreacted RNase A, etc.

According to the RNase A capturing method of the present invention, RNase A contained in a sample can be simply and easily captured or removed from the sample through an antibody or peptide that is fixed to a bead and specifically binds to RNase A.

### [Brief Description of the Drawings]

FIGs. 1 and 3 are schematic diagrams illustrating the screening process of Fv-antibodies that specifically bind to RNaseA.
FIG. 2 shows the FITC results confirming the binding of the fluorescently labeled RNase.

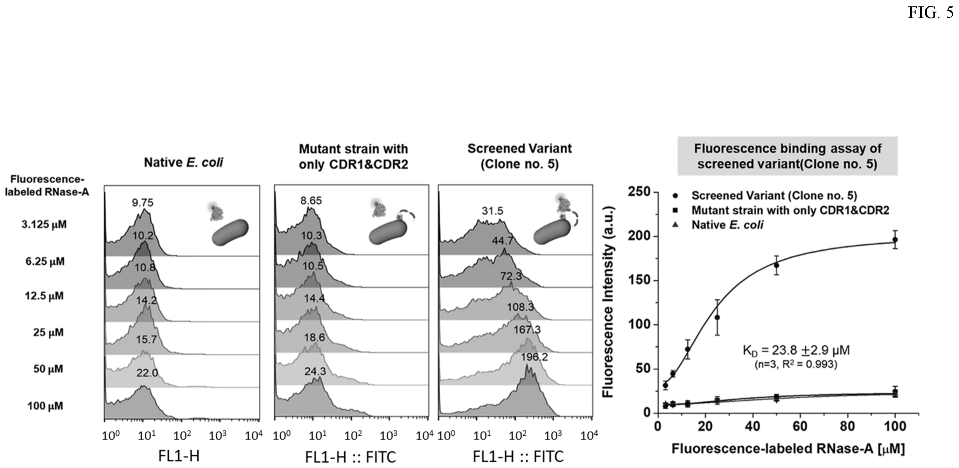

FIGs. 4 to 7 show the results of flow cytometric analysis of the screened antibodies.
FIG. 8 is a schematic diagram illustrating the antibody expression process using a recombinant plasmid.
FIG. 9 shows the electrophoresis results of the screened Fv-antibodies (clones 5, 23, and 39 from left to right).
FIG. 10 is a schematic diagram illustrating a part of the SPR process of the peptide.
FIGs. 11 and 12 show the SPR results of the screened peptides (Peptide-1, Peptide-2, Peptide-3).
FIG. 13 is a schematic diagram illustrating the hydrolysis reaction of 2',3'-cyclic CMP (cCMP).
FIGs. 14 to 17 show the results of cCMP analysis to confirm the RNase A inhibitory activity of the Fv antibody.
FIG. 18 is a schematic diagram of probe analysis for RNase inhibition analysis.
FIGs. 19 to 21 show the results of probe analysis for RNase inhibition analysis.
FIGs. 22 to 24 show the electrophoresis results of RNA for RNase inhibition analysis.
FIG. 25 shows the estimated IC₅₀ values based on the electrophoresis results of RNA.
FIG. 26 shows the electrophoresis results of RNA for RNase inhibition analysis.
FIG. 27 shows the estimated IC₅₀ values based on the electrophoresis results of RNA.
FIGs. 28 to 30 show the docking simulation results between the peptide and RNase A.
FIG. 31 shows the cCMP analysis results for actual samples of serum, pig swab, and saliva swab.
FIGs. 32A and 32B show the cCMP analysis results for serum real samples treated with peptide-1 to peptide-3 and Fv-1 to Fv-3.
FIGs. 33A and 33B show the cCMP analysis results for the Pig swap actual samples treated with Peptide-1 to Peptide-3 and Fv-1 to Fv-3.
FIGs. 34A and 34B show the cCMP analysis results for saliva swap actual samples treated with peptide-1 to peptide-3 and Fv-1 to Fv-3.
FIG. 35 is a conceptual diagram for explaining the RNase A capturing method according to an embodiment of the present invention.
FIGs. 36 to 38 show the results of electrophoretic analysis according to Example 1.
FIG. 39 shows the results of electrophoretic analysis according to Examples 2 to 4.
FIG. 40A illustrates the RNase detection immunosensor according to one embodiment of the present invention and the RNase detection mechanism thereof, and FIG. 40C is a diagram for explaining the switching peptide depicted in FIG. 40A.
FIG. 41 is a diagram for explaining an RNase detection immunosensor according to another embodiment of the present invention.
FIG. 42 shows the fluorescence measurement results according to Examples 1 to 3.

### [Best Mode for Carrying Out the Invention]

Unless otherwise defined, all technical and scientific terms used in this specification shall have the same meaning as commonly understood by a person skilled in the art to which the present invention pertains. Generally, the nomenclature used in this specification and the experimental methods described herein are well-known and commonly used in the technical field.

The term "antibody" as used in this specification encompasses all forms of antibodies known to date in the broadest sense. That is, as long as an antibody exhibits antigen-binding activity, it includes, without limitation, monoclonal antibodies, polyclonal antibodies, multispecific antibodies (for example, bispecific antibodies), full-length antibodies, and various antibody structures including their antigen-binding fragments. The term "antibody" also includes conventional tetravalent antibodies, single-domain antibodies, and their antigen-binding fragments.

The antibody according to the present invention may be in the form of a single-domain antibody (sdAb). A single-domain antibody can include a heavy chain variable domain derived from a heavy-chain-only antibody (for example, VHH from the Camelidae family), a light chain derived from a conventional four-chain antibody, a binding molecule that naturally lacks a single domain (for example, VH or VL), a humanized heavy-chain-only antibody, a human single-domain antibody produced by gene-engineered mice or rats expressing human heavy chain segments, and manipulated domains and single-domain scaffolds other than those derived from antibodies, without limitation. The sdAb can be derived from any species, including but not limited to mouse, rat, human, camel, llama, eel, fish, shark, goat, rabbit, and bovine. It may also include naturally occurring single-domain antibodies from species other than the Camelidae family.

Furthermore, the single-domain antibody is derived from a known naturally occurring single-domain antigen-binding molecule that is a heavy-chain antibody lacking a light chain. Such single-domain molecules are disclosed, for example, in WO 94/04678 and Hamers-Casterman et al., (1993) Nature 363:446-448. The variable domain derived from a heavy-chain molecule that naturally lacks a light chain is referred to as VHH in this specification, distinguishing it from the conventional VH of a 4-chain immunoglobulin. Such VHH molecules can be derived from antibodies produced by camelids, such as camels, llamas, vicuñas, Bactrian camels, alpacas, and guanacos. Other species outside of camelids may also produce heavy-chain molecules that naturally lack a light chain, and such VHHs are within the scope of the present application.

Furthermore, the single-domain antibody according to the present invention may be a chimeric antibody. Specific chimeric antibodies are described, for example, in patent US4,816,567; and Morrison et al., Proc. Natl. Acad. Sci. USA, 81:6851-6855 (1984). In some embodiments, the chimeric antibody may include a non-human variable region (for example, a variable region derived from a camelid species, such as a llama) and a human constant region. Additionally, the chimeric antibody may be humanized. Typically, non-human antibodies are humanized to reduce immunogenicity in humans while maintaining the specificity and affinity of the parent non-human antibody. Generally, humanized antibodies include one or more variable domains in which the CDRs (or portions thereof) are derived from the non-human antibody, and the FRs (or portions thereof) are derived from human antibody sequences. Humanized antibodies will also arbitrarily include at least a portion of the human constant region. In some embodiments, certain FR residues in the humanized antibody may be substituted with corresponding residues from the non-human antibody (for example, the antibody from which the CDR residues are derived) to restore or improve antibody specificity or affinity.

The term "Fv (Variable region fragment)" used in this specification refers to the Fv as the antigen-binding region of immunoglobulin G, which consists of three CDR regions (CDR1, CDR2, and CDR3) and a framework region (FR) between the CDR regions, making it the minimal antibody fragment that contains only the heavy chain variable region and/or the light chain variable region. The recombinant techniques for producing Fv are described in international patent application WO88/10649, among others.

The term "CDR (complementarity determining region)" used in this specification refers to the amino acid residues of the antibody variable domain that are necessary for antigen binding. Each variable domain typically has three CDR regions identified as CDR1, CDR2, and CDR3. Additionally, the CDR of the heavy chain variable region (heavy chain) may be denoted as HCDR.

The term "auto display" used in this specification refers to a technique for expressing (displaying) variant polypeptides as fusion proteins with at least a portion of a coat protein on the surface of an organism, such as phages or Escherichia coli. The utility of surface display lies in its ability to rapidly and efficiently sort sequences that bind to target antigens with high affinity, based on a large library of randomized protein variants. The expression of peptide and protein libraries on the surface of organisms has been used to screen millions of polypeptides to identify those with specific binding characteristics. In particular, it is important to generate various libraries of antibodies or antigen-binding proteins for the isolation of high-affinity antibodies. The CDR3 region has been found to often participate in antigen binding. The CDR3 region on the heavy chain is quite diverse in terms of size, sequence, and structural conformation, allowing for the production of various libraries using this region.

"Nucleic acid" encompasses DNA (gDNA and cDNA) and RNA molecules, and the basic building blocks of nucleic acids, nucleotides, include not only natural nucleotides but also analogues with modified sugar or base moieties. The sequence of the nucleic acid coding for the heavy chain and light chain variable regions of the present invention may be modified. Such modifications include additions, deletions, or non-conservative or conservative substitutions of nucleotides.

The amino acid sequence of the antibody or an antigen-binding fragment of the present invention, or the nucleic acid coding therefor, is interpreted to include sequences that exhibit substantial identity with the sequence listed in SEQ ID NO:. The term "substantial identity" refers to a sequence that, when aligned with any other sequence to maximize correspondence, shows at least 90% homology, and more preferably at least 95% homology, with sequences showing 96% or more, 97% or more, 98% or more, and 99% or more homology, as analyzed using algorithms commonly employed in the art.

Based on this, the antibody or its antigen-binding fragment of the present invention may have 90%, 91%, 92%, 93%, 94%, 95%, 96%, 97%, 98%, 99%, or greater homology compared to the specified sequence or the entirety described in the specification. Such homology can be determined by sequence comparison and/or alignment using methods known in the art. For example, the percentage sequence homology of the nucleic acid or protein of the present invention can be determined using sequence comparison algorithms (i.e., BLAST or BLAST 2.0), manual alignment, or visual inspection.

The DNA encoding the antibody is easily isolated or synthesized using conventional methods (for example, by using an oligonucleotide probe that can specifically bind to the DNA encoding the heavy and light chains of the antibody). Many vectors are available. The vector components generally include one or more of the following, but are not limited to: signal sequences, replication origins, one or more marker genes, enhancer elements, promoters, and transcription termination sequences.

The term "vector" used in this specification refers to a means for expressing a target gene in a host cell, including plasmid vectors; cosmid vectors; bacteriophage vectors; adenoviral vectors; retroviral vectors; and adeno-associated viral vectors, among others. The nucleic acid coding for the antibody in the vector is operatively linked to a promoter.

"Functionally linked" refers to a functional connection between a nucleic acid expression regulatory sequence (e.g., a promoter, signal sequence, or an array of transcription factor binding sites) and another nucleic acid sequence, whereby the regulatory sequence regulates the transcription and/or translation of the other nucleic acid sequence.

In the case of using prokaryotic cells as hosts, it is common to include a strong promoter capable of driving transcription (for example, tac promoter, lac promoter, lacUV5 promoter, 1pp promoter, rac5 promoter, amp promoter, recA promoter, SP6 promoter, trp promoter, and T7 promoter), a ribosome binding site for the initiation of translation, and transcription/translation termination sequences. Additionally, for example, when using eukaryotic cells as hosts, promoters derived from the genome of mammalian cells (e.g., metallothionein promoter, actin promoter, human hemoglobin promoter, and human muscle creatine promoter) or promoters derived from mammalian viruses (e.g., adenovirus late promoter, vaccinia virus 7.5K promoter, SV40 promoter, cytomegalovirus (CMV) promoter, HSV tk promoter, mouse mammary tumor virus (MMTV) promoter, HIV LTR promoter, Moloney virus promoter, Epstein-Barr virus (EBV) promoter, and Rous sarcoma virus (RSV) promoter) can be utilized, and they generally have a polyadenylation sequence as a transcription termination sequence.

In some cases, the vector may be fused with another sequence to facilitate the purification of the antibody expressed therefrom. The fused sequence may include, for example, glutathione S-transferase (Pharmacia, USA), maltose-binding protein (NEB, USA), FLAG (IBI, USA), and 6x His (hexahistidine; Quiagen, USA).

The vector includes an antibiotic resistance gene that is commonly used in the industry as a selectable marker, such as resistance genes for ampicillin, gentamicin, cabenicillin, chloramphenicol, streptomycin, kanamycin, geniticin, neomycin, and tetracycline.

The present invention, from another perspective, relates to transformed cells using the aforementioned vector. The cells used to generate the antibodies of the present invention may be prokaryotic, yeast, or higher eukaryotic cells, but are not limited to these.

Prokaryotic host cells such as strains of the genus *Bacillus,* including *Escherichia coli, Bacillus subtilis,* and *Bacillus thuringiensis,* as well as *Streptomyces, Pseudomonas,* Proteus mirabilis, and *Staphylococcus,* can be used.

The present invention relates to a method for producing an antibody or its antigen-binding fragment, comprising (a) a step of culturing cells; and (b) a step of recovering the antibody or its antigen-binding fragment from the culture medium and/or the cells.

The cells can be cultured in various media. Any commercially available medium can be used as a culture medium without limitation. Additionally, all other necessary supplements known to those skilled in the art may be included at appropriate concentrations. Culture conditions, such as temperature and pH, have already been used in conjunction with the selected host cells for expression, which will be apparent to those skilled in the art.

The term "transformation" as used in this specification refers to the introduction of a vector containing nucleic acid that encodes a target protein into a host cell, allowing the protein encoded by the nucleic acid to be expressed within the host cell. Transformed nucleic acid includes all forms, whether inserted into the chromosome of the host cell or located outside the chromosome, as long as it can be expressed within the host cell. Furthermore, the nucleic acid includes DNA and RNA that encode the target protein. The nucleic acid can be introduced into the host cell in any form as long as it can be expressed. For example, the nucleic acid may be introduced into the host cell in the form of an expression cassette, which is a gene construct that contains all the elements necessary for its own expression. The expression cassette typically includes a promoter, transcription termination signal, ribosome binding site, and translation termination signal that are operably linked to the nucleic acid. The expression cassette may be in the form of a self-replicating expression vector. Additionally, the nucleic acid may be introduced into the host cell in its own form, operably linked to the sequences necessary for expression in the host cell.

The recovery of the antibody or its antigen-binding fragment can be achieved by removing impurities, for example, through centrifugation or ultrafiltration, and the resulting product can be purified using methods such as affinity chromatography. Additional purification techniques, such as anion or cation exchange chromatography, hydrophobic interaction chromatography, and hydroxylapatite chromatography, may also be employed.

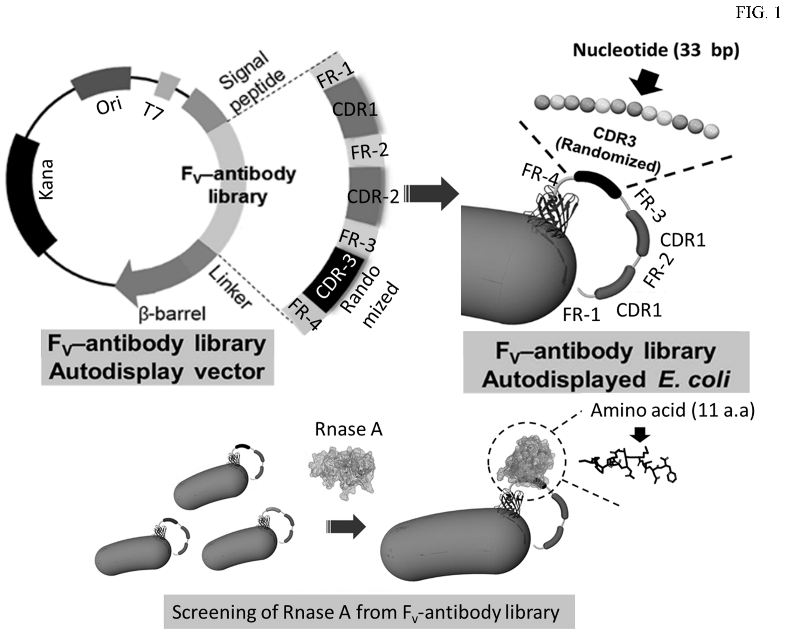

In the present invention, the RI with specific binding affinity for the active site of RNase A was screened from an Fv-antibody library that is surface-expressed on the outer membrane of Escherichia coli. As shown in FIG. 1, the Fv-antibody consists of the variable region of the IgG heavy chain, which is composed of three complementarity-determining regions (CDRs) and a framework region (FR) between the CDRs. To form the Fv-antibody library, the CDR3 region, which has 11 amino acid residues, was randomized by site-directed mutagenesis.

As previously reported, the Fv-antibody library was expressed on the outer membrane of *E. coli* using surface display technology, achieving a diversity level of over 10⁶ clones/library. The surface display technology transports the translated protein through the beta-barrel of AIDA-1 to the outer membrane of *E. coli* to express the target protein, and the expression yield of surface display is estimated to be over 10⁵ proteins/E. *coli.* This surface display technology has been used for the expression of Z-domain, streptavidin, lipase, Ro/SSA antigen, casein, and others. Using surface display technology, the Fv-antibody library was densely expressed on the outer membrane of *E. coli (>* 10⁵ proteins/E. *coli).* When fluorescently labeled antigens (target antigens) are treated with the surface-displayed Fv-antibody library, *E. coli* with target Fv variants generate sufficiently strong fluorescence to be efficiently isolated using flow cytometry without the need for repetitive panning processes.

In the present invention, the surface-expressed Fv-antibody library was used for the screening of Fv-variants that specifically bind to RNase A. The antibody library was prepared by introducing site-specific mutations in the CDR3 region of the Fv-antibody. Subsequently, fluorescently labeled RNase A was treated to screen *E. coli* for the target Fv-variants, and then the specific binding affinity of the Fv-variants on the *E. coli* outer membrane was estimated using flow cytometry. The screened Fv-variants were then expressed as soluble Fv antibodies and synthesized into peptides to have the amino acid sequence of the CDR3 region of the screened Fv-variants. The binding affinity constant (KD) of the synthesized peptides containing the CDR3 region of the expressed Fv-antibody and the screened Fv-variants was estimated using an SPR biosensor that included immobilized RNase A. The activity of the Fv-antibody and the RI generated from the synthesized peptides was measured through a cleavage assay of the phosphodiester bonds of RNA and an RNase activity assay using RNA probes labeled with a fluorophore and a quencher, and compared with two commercial RIs. Finally, the activity of the RI made from the expressed Fv-antibody and the synthesized peptides was demonstrated by an RNA cleavage assay using total RNA from HeLa cells.

### Example 1. Materials

Ribonuclease A (Type II-A) from bovine pancreas, cytidine 2':3'-cyclic monophosphate monosodium salt (2',3'-cCMP), and bovine serum albumin were purchased from Sigma-Aldrich Korea (Seoul, Korea). Luria-Bertani (LB) broth and agar media were obtained from Duchefa (Haarlem, Netherlands). Synthetic peptides (Peptide-1, 2, and 3) with 90% purity (Table 2) were purchased from Peptron Co. (Daejeon, Korea). RNaseOUT^{™} recombinant ribonuclease inhibitor, dynabeads^{™} M-280 tosylactivated, Phusion High-Fidelity DNA polymerase, and other PCR reagents were obtained from Thermo Fisher Scientific Inc. (Waltham, MA, USA). Biofact^{™} RNase inhibitor was purchased from Biofact Co. (Daejeon, Korea). Primers for the FV-antibody library construction were custom synthesized by Bionics (Seoul, Korea). Klenow DNA polymerase and NEBuffer^{™} 2 were obtained from New England Biolabs (Ipswich, MA, USA). PCR purification mini kits and plasmid extraction kits were purchased from Favorgen Biotech Corp. (Pingtung, Taiwan). A 100 kDa Amicon ultra centrifugal filter was obtained from Millipore (MA, USA). BK7 glass for SPR analysis was purchased from iCLUEBiO (Gyeonggi-do, Korea). TdTomato-labeled Fv-antibody fusion protein plasmids were custom synthesized by Cosmogenetech (Seoul, Korea).

### Example 2. Preparation of Fv-Antibody Library

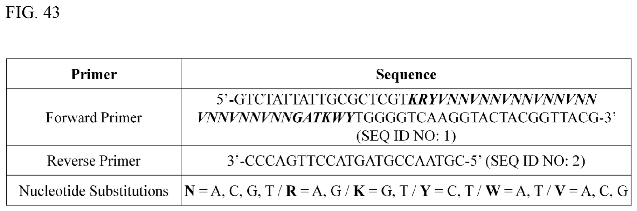

Table 1 is shown in FIG. 43.

A synthesized single-stranded DNA primer containing a randomized CDR3 sequence (75 bp, 0.5 µM) was first mixed with a reverse primer (22 bp) and NEBuffer2TM, followed by heating (95°C for 5 minutes) and cooling (36°C for 1 minute) to perform primer hybridization. The primer sequences used for constructing the Fv-antibody library are summarized in Table 1. After hybridization, primer polymerization was performed as follows: Klenow (exo-) DNA polymerase (3 µL), NEBuffer2TM (10 µL), dNTPs (10 mM, 8 µL), and the hybridized primers were mixed with deionized water (DW) to a total volume of 200 µL. Primer polymerization was carried out (37°C for 15 minutes) followed by enzyme inactivation (75°C for 20 minutes). Subsequently, the Fv-antibody library primers with the randomized CDR3 sequence were collected using a PCR purification mini kit. The Fv-antibody library plasmids containing the randomized CDR3 sequence were synthesized through PCR. Template plasmid (pST009, 150 ng), dNTPs (10 mM, 8 µL), HF buffer (10 µL), purified Fv-antibody library primers (150 ng), and Phusion high-fidelity DNA polymerase (0.5 µL) were mixed and PCR was performed under the following conditions: (1) initial denaturation (98°C, 1 minute), (2) denaturation (98°C, 30 seconds), (3) annealing (68°C, 1 minute), (4) elongation (72°C, 5 minutes), (5) 30 cycles (repeating steps (2)-(4)), and (6) final extension (72°C, 10 minutes). After PCR, DpnI enzyme was added to digest the template plasmid. The Fv-antibody library plasmids with the randomized CDR3 sequence were finally purified using a 100 kDa Amicon Ultra centrifugal filter. The surface expression of the Fv-antibody library on the outer membrane of *E. coli* BL21(DE3) was prepared by transforming the purified Fv-antibody library plasmids into electrocompetent cells. Electroporation was performed for bacterial transformation. The electroporated *E. coli* cells were cultured overnight in LB medium (10 mL) containing 50 µg/mL kanamycin (37°C, 16 h, 150 rpm). For the expression of the Fv-antibody library with the randomized CDR3 sequence on the outer membrane of *E. coli* cells, the overnight cultured cells were re-cultured in LB medium (10 mL) containing kanamycin (50 µg/mL), 99% β-mercaptoethanol (8 µL), and ethylenediaminetetraacetic acid (5 µM) (37°C, 2.5 h, 150 rpm). Isopropyl thio-β-galactoside (IPTG) induction was performed as follows for the expression of the Fv-antibody library with the randomized CDR3 sequence on the outer membrane of *E. coli* cells (Fv-variants): 1 mM IPTG was added and cultured (30°C, 3 hours, 120 rpm).

### Example 3. Screening of Fv-antibodies that specifically bind to RNase A

Before screening for FV antibodies against RNase A, RNase was first immobilized on magnetic beads. The immobilization of RNase on the magnetic beads was performed by mixing M-280 tosylactivated magnetic beads (5 mg, 165 µL), RNase A (100 µg), 0.1 M borate buffer (pH 9.5, 150 µL), and 3 M ammonium sulfate (100 µL) (37°C, 16 hours, 120 rpm). The washing step with PBS was carried out using an external magnet, and the immobilized RNase A-magnetic beads were collected and resuspended in a PBS solution.

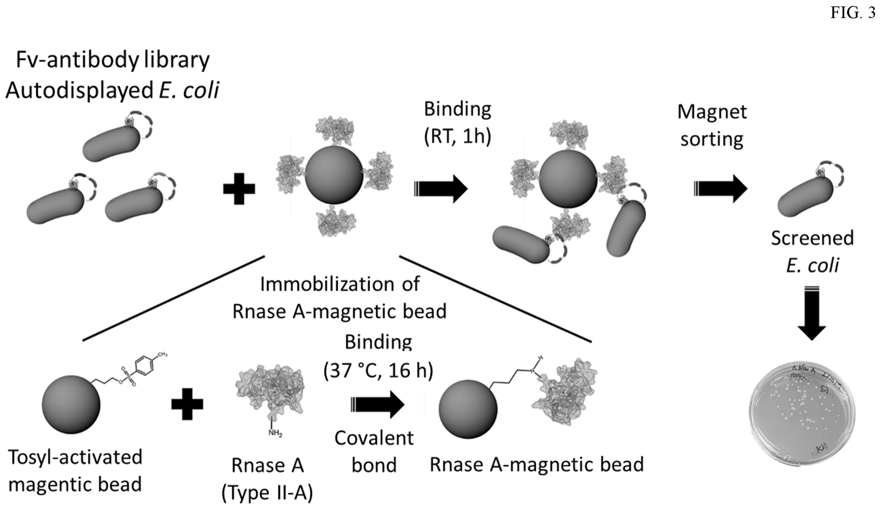

The screening of Fv variants specifically binding to RNase A was performed as follows: (1) Fv variants (200 µL, OD600 nm = 0.5) and immobilized RNase A-magnetic beads (10 mg/mL, 10 µL) were mixed at room temperature and 15 rpm for 1 hour, (2) using an external magnet, Fv variants specifically binding to the RNase A-immobilized magnetic beads were collected and subjected to 10 washing steps with 0.01% PBST and PBS, (3) the isolated Fv variant-bound RNase A magnetic beads were resuspended in 100 µL PBS, and (4) the resuspended magnetic beads were spread on an agar plate to obtain Fv variants specifically binding to RNase A, as illustrated in FIG. 3.

### Example 4. Flow Cytometry

To select Fv variants that specifically bind to RNase A, 44 Fv variant clones selected on an agar plate were cultured and mixed with 5 µM of FITC-labeled RNase A (RT, 1h, 15 rpm).

FITC-labeled RNase A was synthesized by mixing FITC (0.5 mg) and RNase A (1 mg) in 2 ml of PBS buffer under incubation conditions (RT, 24h, 15 rpm), and the FITC-labeled RNase A was purified using a 10 kDa Amicon Ultra centrifugal filter. After binding the Fv variant to the FITC-labeled RNase A, the Fv variant cells were washed three times with PBS buffer by centrifugation (3,000 x g, 3 minutes) and finally resuspended in PBS. The fluorescence intensity of the Fv variant was measured using a FACSCalibur flow cytometer (Becton-Dickinson, Franklin Lakes, NJ, USA) at an excitation wavelength of 485 nm and an emission wavelength of 535 nm, as shown in FIG. 3.

### Example 5. Expression of Antibody

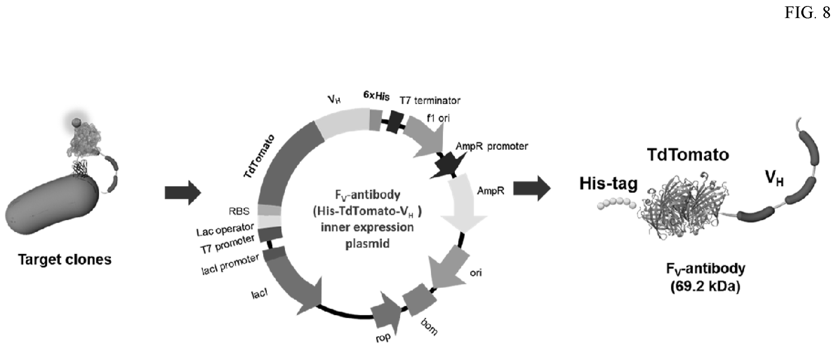

The recombinant plasmids (pJS001, pJS002, and pJS003) encoding the open reading frames of the screened Fv variants with binding activity to RNase A, TdTomato, and His-tag fusion proteins were custom synthesized by Cosmogenetech as shown in FIG. 8. The three plasmids were transformed into electrocompetent cells via electroporation, and Fv antibodies (His-tag-TdTomato-VH) were expressed in the transformed *E. coli* cells. The three transformed *E. coli* cells were cultured overnight in 20 ml of high-salt LB medium containing 1 mM IPTG and 30 µg/ml carbenicillin (30°C, 16 hours, 150 rpm). The cultured *E. coli* pellet was collected by centrifugation (3,000 xg, 3 minutes) and then resuspended in 30 ml of 3M urea containing binding buffer (1M NaCl, 5 mM Tris-HCl, 0.5 mM EDTA). The resuspended *E. coli* cells were lysed using an ultrasonic processor (Vibra cell VCX-130, Sonics, USA), and the lysate containing the Fv antibodies was collected by centrifugation (25,000 xg, 10 minutes, 4°C). The TdTomato-labeled Fv antibodies were purified using a His-tag purification column (Roche, Basel, Switzerland) and elution buffer (3M urea binding buffer with 100 mM imidazole). Dialysis (4°C, 16 hours, 100 rpm) was performed to remove urea and imidazole from the purified Fv antibody protein solution.

### Example 6. SPR Biosensor Analysis

The analysis of RNase A activity inhibition based on 2',3'-cyclic CMP (cCMP) was performed by measuring the absorbance of cCMP at a wavelength of 286 nm. A mixture was prepared by combining Tris-acetate buffer (500 mM, pH = 6.5, 200 µL), EDTA (5 mM, 200 µL), 2',3'-cCMP (10 mM, 100 µL), RNase A (10 µg/mL, 100 µL), Fv variant (or synthetic peptides in the concentration range of 0.1 nM - 1 µM), and DW (filled up to 1000 µL). The absorbance at 286 nm was measured using a Lamda35 UV-VIS spectrophotometer (Perkin-Elmer, MA, USA). The reaction was initiated by adding RNase A to the mixture, and the absorbance was measured every 3 minutes for a total of 30 minutes.

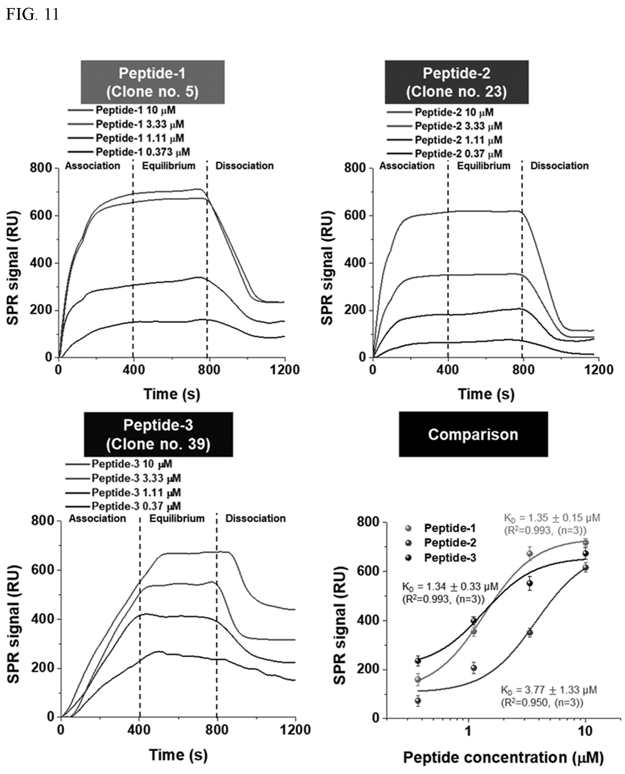

Binding analysis of the expressed FV antibody and synthesized peptides against RNase A was performed using an SPR (Surface Plasmon Resonance) biosensor from I-Cluebio (Seongnam, Korea). RNase A was immobilized on the gold surface of the SPR chip used in the SPR biosensor to conduct the binding analysis. For the manufacture of the SPR chip, a 2 nm thick titanium adhesive layer and a 48 nm thick gold surface were consecutively sputter-coated onto BK-7 glass (1×1 cm²). As shown in FIG. 11, RNase A (100 µg/ml) from bovine pancreas was incubated on the gold surface SPR chip at 4°C for 16 hours. To analyze the binding of the expressed Fv antibody to RNase A, Fv antibodies in the concentration range of 0.37 - 100 nM were reacted using PBS buffer as the cell through association (10 minutes), equilibrium (5 minutes), and dissociation (10 minutes) steps. The washing buffer and total SPR measurements were performed at a flow rate of 15 µL/min. For the binding analysis of the synthesized peptides against RNase A, peptides in the concentration range of 0.37 - 100 nM were reacted using PBS buffer as the washing buffer through association (10 minutes), equilibrium (5 minutes), and dissociation (10 minutes) steps, and total SPR measurements were conducted at a flow rate of 15 µL/min.

The analysis of RNase inhibitory activity of Fv antibodies and synthetic peptides was performed using RNA samples extracted from HeLa cells as follows: (1) Mixing the RNA sample extracted from HeLa cells (600 ng) with 87.5 nM - 5.6 µM under conditions of 37°C for 30 minutes, (2) Electrophoresis on a 1% agarose gel at 90 V for 40 minutes. The estimation of RNase inhibitory activity was carried out by gel imaging of the cleaved RNA using a ChemiDoc XPS (Bio-Rad, CA, USA).

### Example 7. Screening of Fv antibodies with affinity for RNase

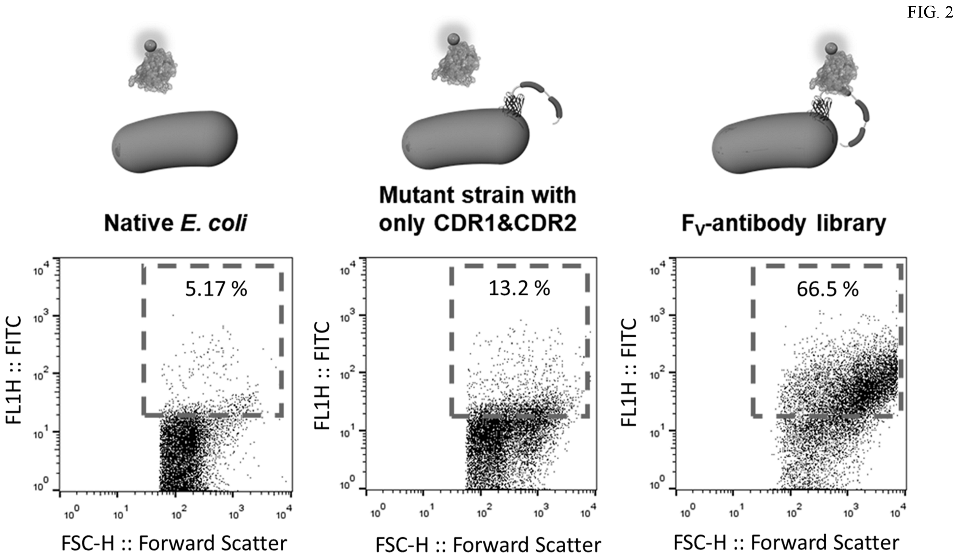

Target Fv variants with specific affinity for RNase A were screened from an Fv library that is surface-expressed in the outer membrane of *E. coli.* The Fv-antibody library consisted of three CDR regions (CDR1, 2, 3), and the CDR3 region, which has 11 amino acid residues, was designed to have a randomized amino acid sequence as shown in FIG. 1. After surface expression of the Fv-antibody library, the diversity of the Fv-antibody library was estimated to be over 10⁶ clones/library through random clone selection and genetic analysis of the CDR3 region. To screen for targeted Fv variants with affinity for RNase A, fluorescently labeled RNase A was treated with the Fv-antibody library. As illustrated in FIG. 2, Fv variants were observed to have significantly higher fluorescence intensity compared to the wild-type *E. coli,* where the Fv-antibody was not expressed on the surface, and the mutant strain with Fv-antibodies consisting only of CDR1 & 2. The Fv variants in the dotted rectangular area of the Fv-antibody library (red) were marked as target clones with Fv-antibodies that have specific affinity for RNase A. A significantly higher proportion of clones was observed at the same fluorescence level, compared to the wild-type *E. coli* (5.2%) and the mutant strain (13.2%) versus the Fv-antibody library (66.5% of the total population). These results indicate that the Fv-antibody library contains targeted Fv variants with high affinity for RNase A and that these targeted variants were isolated from the Fv-antibody library using magnetic beads with immobilized RNase A.

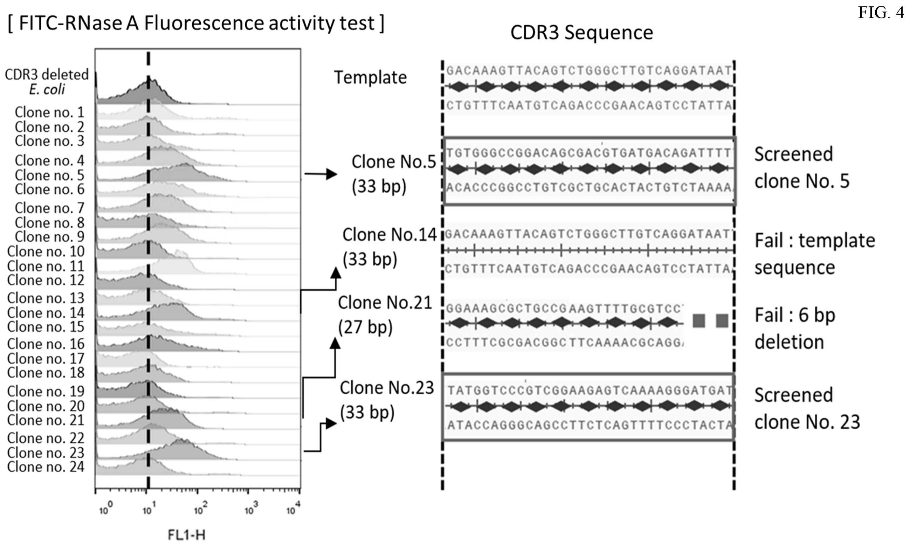

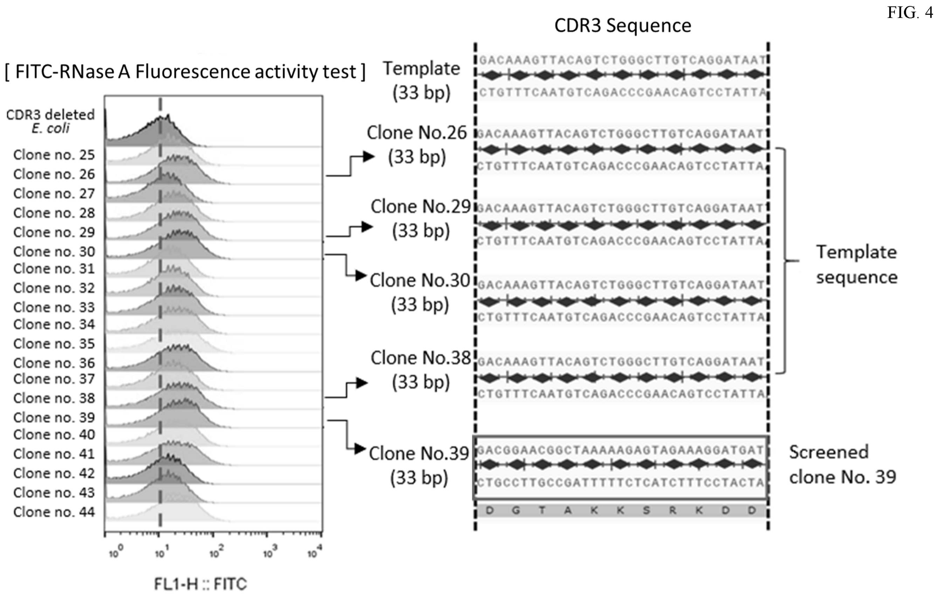

To isolate the target Fv variants against RNase A, RNase A was covalently immobilized on tosyl-activated magnetic beads, and the Fv library was cultured with the magnetic beads as shown in FIG. 3. The target clones were separated from the Fv library using an external magnet, and the magnetic beads were cultured on an agar plate. Randomly selected clones were analyzed using a flow cytometer with fluorescently labeled RNase A. As illustrated in FIG. 4, three clones and nine variants (clones 5, 14, 21, 23, 26, 29, 30, 39) exhibited significantly high fluorescence signals in the flow cytometry analysis using fluorescently labeled RNase A. Among these nine variants, six variants were confirmed to have non-targeted CDR3 sequences. Finally, three variants were determined to be target variants with CDR3 sequences different from the template sequence. From these results, clones 5, 23, and 39 were selected as target Fv antibodies.

The sequence of the heavy chain variable region of clone 5 (Fv-1) is as follows.
CDR1: TYGIQ (SEQ ID NO: 3)
CDR2: WIHAGTGGTKYSRKFQG (SEQ ID NO: 4)
CDR3: CGPDSDVMTDF (SEQ ID NO: 5)
VH:

EVQLVESAAEVRRPGASVKITCKASGYSFSTYGIQWMRQAPGQRPEWLGWIHAGTGGT
KYSRKFQGRITITRDTSANTVYLDLNSLTSEDTAVYYCARCGPDSDVMTDFWGQGTTVT
VSS (SEQ ID NO: 8)

The sequence of the heavy chain variable region of clone 23 (Fv-2) is as follows.
CDR1: TYGIQ (SEQ ID NO: 3)
CDR2: WIHAGTGGTKYSRKFQG (SEQ ID NO: 4)
CDR3: YGPVGRVKRDD (SEQ ID NO: 6)
VH:

EVQLVESAAEVRRPGASVKITCKASGYSFSTYGIQWMRQAPGQRPEWLGWIHAGTGGT
KYSRKFQGRITITRDTSANTVYLDLNSLTSEDTAVYYCARYGPVGRVKRDDWGQGTTVT
VSS (SEQ ID NO: 9)

The sequence of the heavy chain variable region of clone 39 (Fv-3) is as follows.
CDR1: TYGIQ (SEQ ID NO: 3)
CDR2: WIHAGTGGTKYSRKFQG (SEQ ID NO: 4)
CDR3: DGTAKKSRKDD (SEQ ID NO: 7)
VH:

EVQLVESAAEVRRPGASVKITCKASGYSFSTYGIQWMRQAPGQRPEWLGWIHAGTGGT
KYSRKFQGRITITRDTSANTVYLDLNSLTSEDTAVYYCARDGTAKKSRKDDWGQGTTVT
VSS (SEQ ID NO: 10)

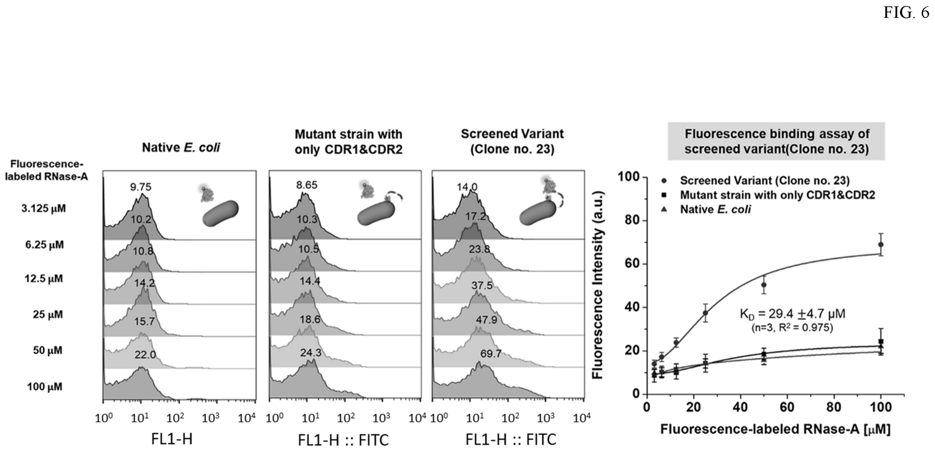

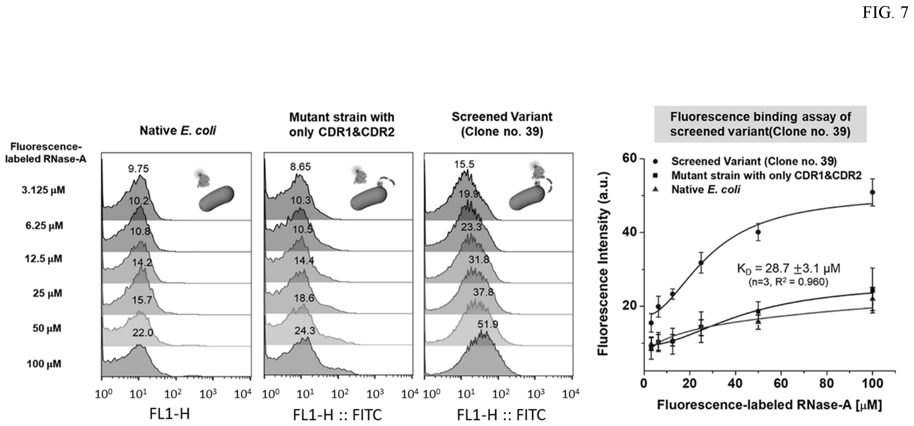

The affinity of the Fv antibodies screened from clone 5, clone 23, and clone 39 was analyzed by treatment with RNase A at varying concentrations: the screened variant (clone 5), a mutant strain with only CDR1 & CDR2, and a natural *E. coli* strain. In the case of the variant with only CDR1 & CDR2 and the natural *E. coli* strain, no significant differences were observed in the baseline and fluorescence signals. For the screened Fv variant (clone 5), a quantitative increase in fluorescence signal was observed according to the concentration of fluorescently labeled RNase A, indicating specific binding of RNase A to the screened Fv antibody. The binding affinity (KD) of the Fv antibody for clone 5 was estimated to be 23.8±2.9 (n=3) µM based on fitting analysis using an adsorption model. The same experiment was conducted for other screened clones (clone 23 and clone 39), and a quantitative increase in fluorescence signal was also observed according to the concentration of fluorescently labeled RNase A. In FIGs. 6 and 7, there was also no significant difference in the baseline and fluorescence signals for the mutant strain with only CDR1 & CDR2 and the natural *E. coli* strain. These results indicate that the specific binding of RNase A occurred with the Fv antibodies of the screened clones (clone 23 and clone 39), and the binding affinities (KD) of the Fv antibodies for clone 23 and clone 39 were estimated to be 29.4±4.7 µM (n=3) and 28.7±3.1 µM (n=3), respectively. From these results, it was inferred that the binding of fluorescently labeled RNase A to the screened clones occurs in the CDR3 region of the screened Fv antibodies, as the mutant strain with only CDR1 and CDR2 and the natural *E. coli* strain were determined not to have specific binding affinity. Furthermore, while CDR1 and CDR2 do not directly participate in the binding of RNase, it was confirmed that they cause steric hindrance within the active site of RNase. Additionally, this effect was found to occur even in cases other than SEQ ID NO: 3 and SEQ ID NO: 4. In other words, since CDR3 was directly involved in binding to RNase, CDR1 and CDR2 acted similarly in the case of other amino acid sequences.

Example 8. Inhibitory activity of synthesized peptides and expressed Fv-antibodies on RNase A.

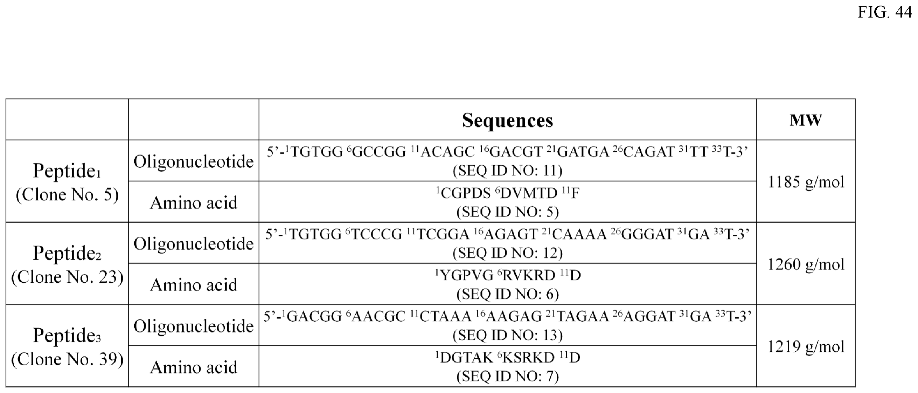

Table 2 is shown in FIG. 44.

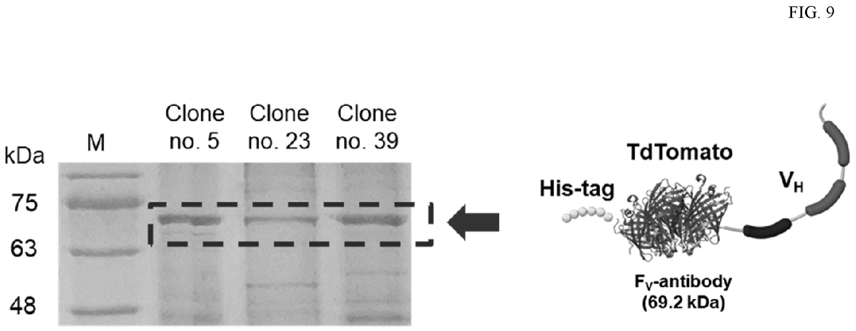

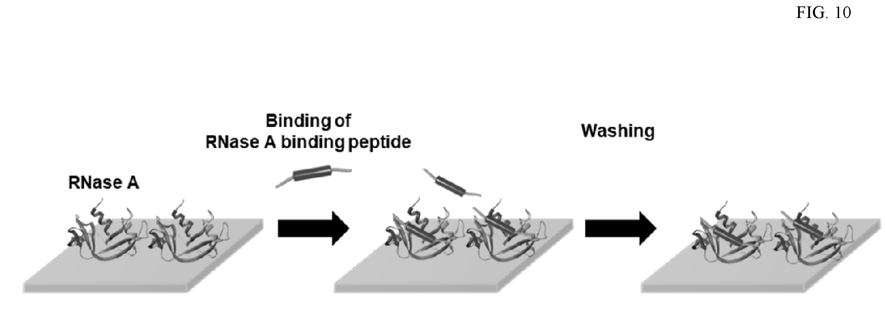

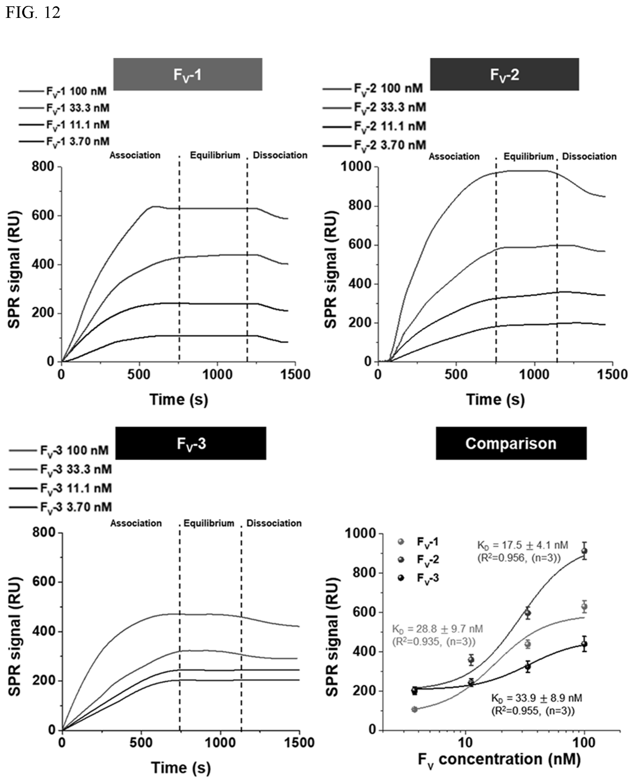

Using the screened CDR3 amino acid sequences, the CDR3 regions of the screened Fv antibodies were synthesized as peptides, and the amino acid sequences were determined from the nucleotide sequences of the CDR3 regions as summarized in Table 2. The clones were internally expressed using recombinant plasmids as shown in FIG. 8. These synthetic peptides (Peptide-1, Peptide-2, Peptide-3) and Fv antibodies (Fv-1, Fv-2, Fv-3) were identical to the amino acid sequences of the CDR3 regions of the screened clones. The expression of the Fv antibodies was confirmed using SDS-PAGE, and the molecular weight of the Fv antibodies was estimated to be 69.2 kDa as shown in FIG. 9. The affinity constants (KD) of these synthetic peptides and the expressed Fv antibodies against RNase A were estimated using an SPR biosensor. As shown in FIG. 10, RNase was immobilized on the Au chip of the SPR biosensor, and as shown in FIG. 11, synthetic peptides in the concentration range of 0.37-10 µM were reacted through binding, equilibrium, and dissociation steps. The SPR signal was plotted according to the concentration of the synthetic peptides, and the affinity constants (KD) of these synthetic peptides were calculated through a fitting process. The affinity constants (KD) were calculated to be 1.3± 0.1 µM for Peptide-1, 1.3± 0.3 µM for Peptide-2, and 3.7± 1.1 µM for Peptide-3. These results demonstrated that the three synthetic peptides could specifically bind to the active site of RNase A. Using the Au chip with immobilized RNase A, the same SPR measurements were performed for the expressed Fv antibodies in the concentration range of 3.70 - 100 nM. As shown in FIG. 12, the affinity constants (KD) were calculated to be 17.5± 4.10 nM for Fv-1, 28.8± 9.7 nM for Fv-2, and 33.9± 8.9 nM for Fv-3. These results indicated that the expressed Fv antibodies could have significantly higher binding affinity for RNase A compared to the synthetic peptides with the same amino acid sequences in the CDR3 regions.

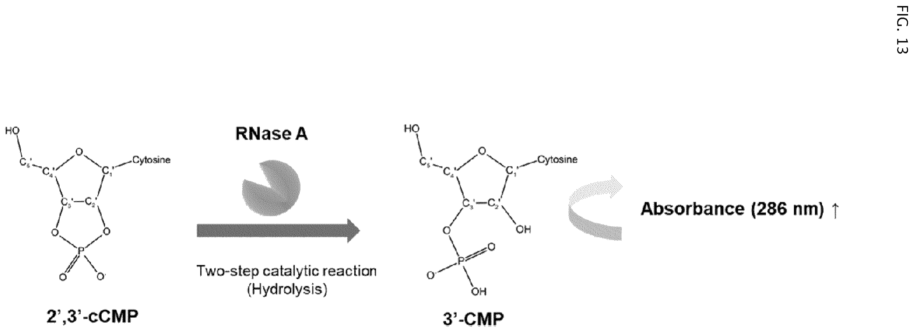

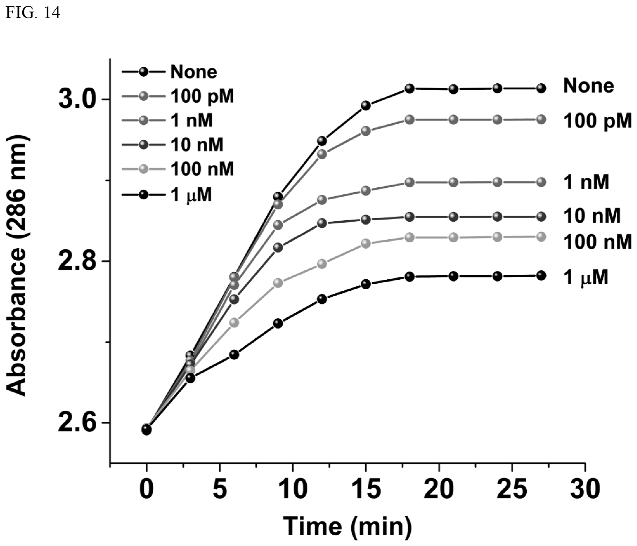

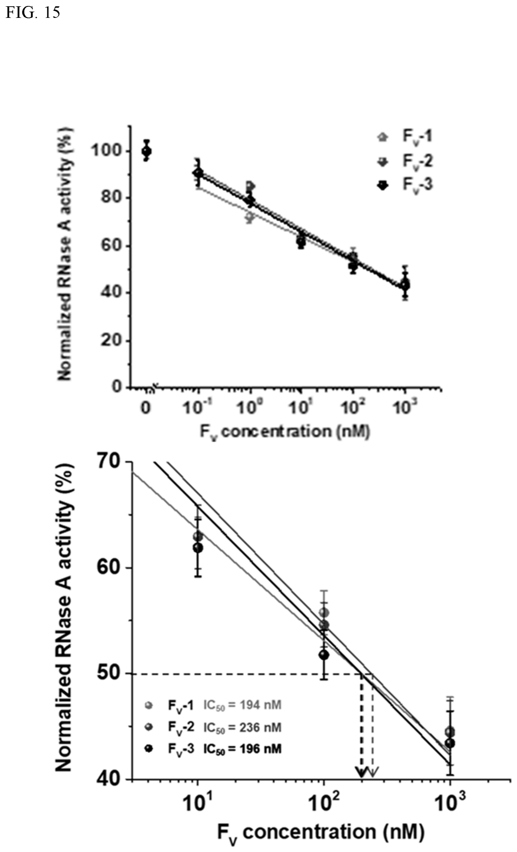

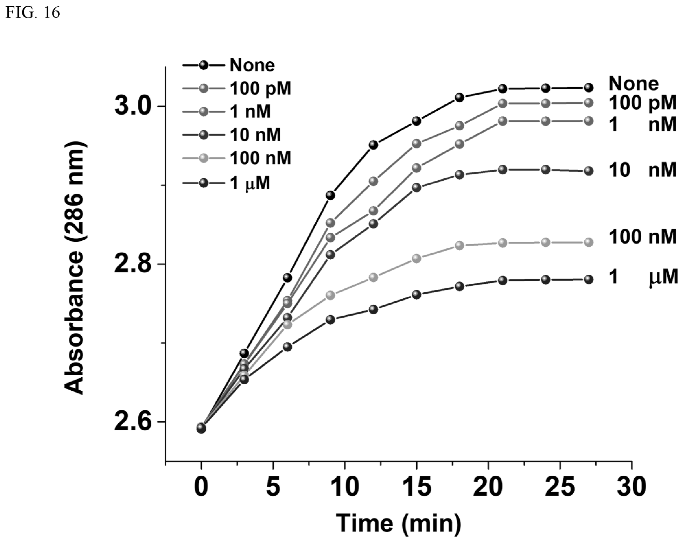

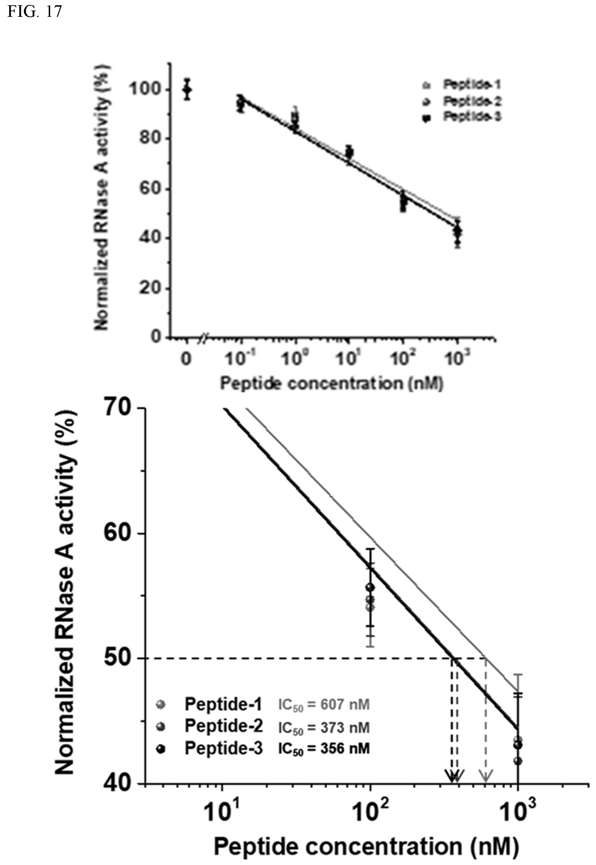

RNase A is known to degrade RNA by cleaving the phosphodiester bonds between nucleotides. The cleavage of the phosphodiester bonds occurs through a two-step reaction: (1) the formation of a 5'-phosphate transition state and (2) the subsequent degradation of a 2',3'-cyclic phosphate intermediate. During this RNA cleavage reaction, histidine residues (His12 and His119) are known to participate in the electron transfer reaction. In the homology of this RNA cleavage reaction, the hydrolysis reaction of 2',3'-cyclic CMP (cCMP) to 3'-CMP has been used to estimate RNase A activity. Specifically, it was utilized that the absorbance at 286 nm quantitatively increases as 3'-CMP increases due to the hydrolysis of cCMP, as shown in FIG. 13. In this invention, the active unit of the inhibitor (1 unit) is defined as the 50% inhibition of RNase A (5 ng) using 0.2 mM cCMP at 100 nM Tris-acetate (pH 6.5) for 10 minutes at 25°C. To estimate the RNase A inhibitory activity of the expressed Fv antibody using the cCMP assay, the expressed Fv antibody (Fv-1) was used as an inhibitor of RNase A in a concentration range of 0.1 nM to 1 µM, and cCMP was observed at 286 nm using a Lamda35 UV-VIS spectrophotometer (Perkin-Elmer, MA, USA). As shown in FIG. 14, the absorbance increased with reaction time, reaching saturation after 18 minutes, confirming that the inhibitory activity increased quantitatively with the concentration of the Fv antibody. The same experiment was performed for other Fv antibodies (Fv-2 and Fv-3) to observe the inhibitory activity of the Fv antibodies. The inhibition curves of the Fv antibodies were plotted linearly, and the IC₅₀ values were calculated by linear regression, as shown in FIG. 15. The IC₅₀ values were estimated to be 194 nM for Fv-1, 236 nM for Fv-2, and 196 nM for Fv-3. The same cCMP-based analysis was performed on synthesized peptides in the concentration range of 0.1 nM to 1 µM. As shown in FIG. 16, the absorbance also increased with reaction time, reaching saturation after 18 minutes, confirming that the inhibitory activity increased quantitatively with the concentration of the Fv antibody. The IC₅₀ values were estimated to be 607 nM for peptide-1, 373 nM for peptide-2, and 356 nM for peptide-3, as shown in FIG. 17. The RNase inhibition constants (IC₅₀) for the synthesized peptides and expressed Fv antibodies are summarized in Table 3.

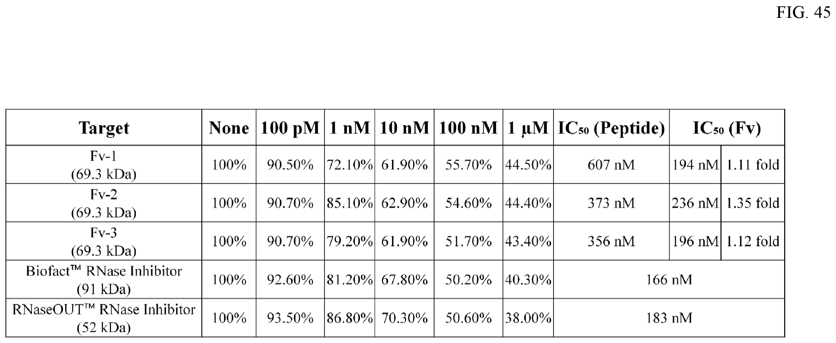

Table 3 is shown in FIG. 45.

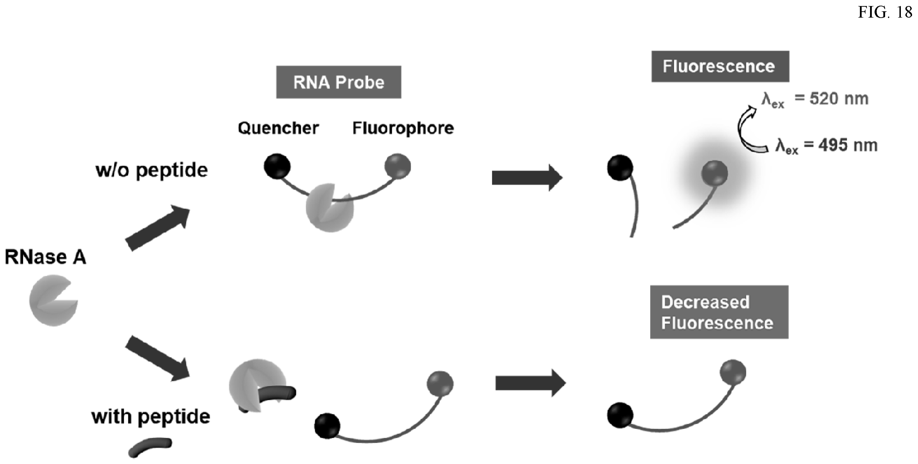

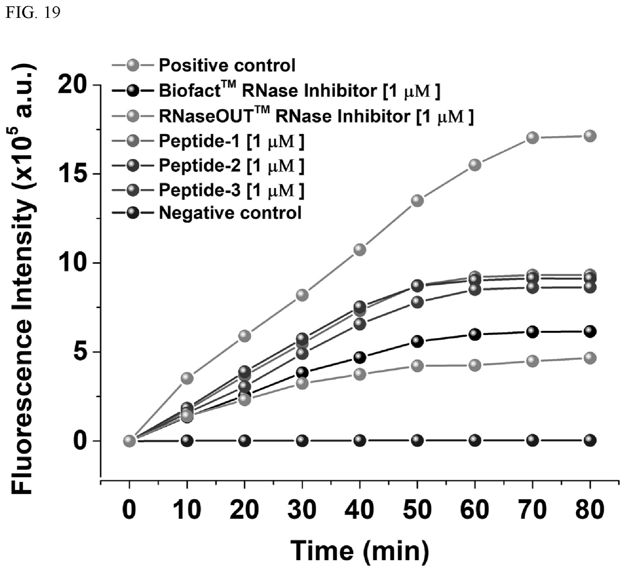

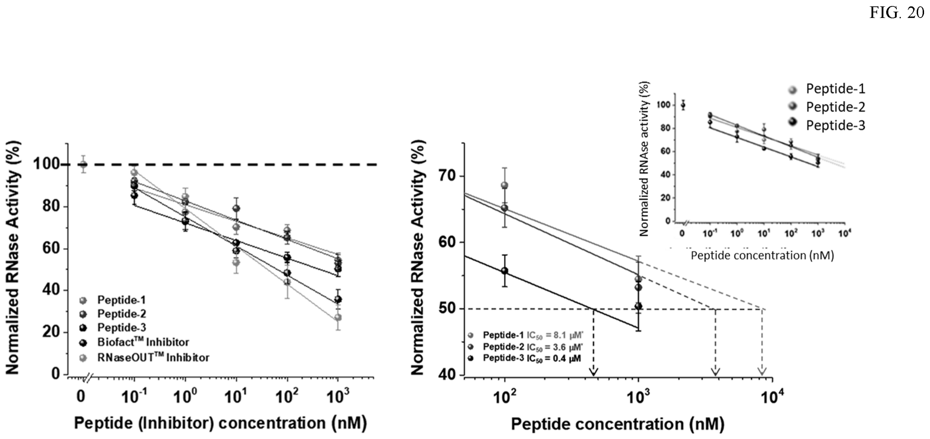

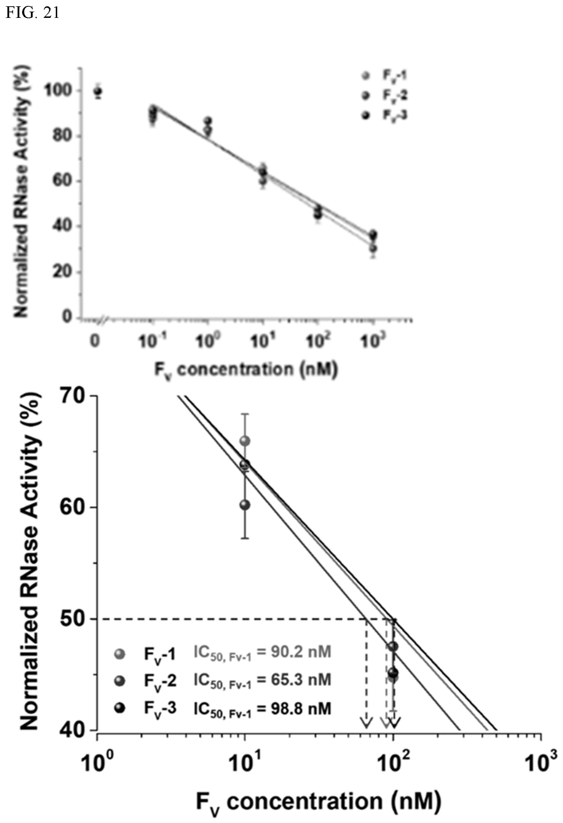

The activity of RNase A, which degrades RNA, was evaluated using specially designed RNA probes. This RNase A analysis was performed using a commercial RNase activity test kit based on a modified Taq-man probe analysis (ab273299) from Abcam (Cambridge, UK). To compare inhibitory activity, three synthetic peptides (peptide-1, peptide-2, peptide-3) and three expressed Fv-antibodies (Fv-1, Fv-2, and Fv-3) were compared using two types of commercially available RNase inhibitors from BiofactTM (Daejeon, South Korea) and two types of RNaseOUTTM from Thermofisher (USA). The probes for the RNase inhibition analysis contained RNA probes with fluorescent dyes and quenching materials at each end of the RNA sequence. As shown in FIG. 18, after the degradation of the RNA backbone, the fluorescent dye was released, generating a fluorescent signal. When RNase A was completely inhibited, the RNA backbone was maintained, and the fluorescent signal was completely absorbed by the quenching material. To estimate the RNase A inhibitory activity by the three synthesized peptides, each peptide was treated at a concentration of 1 µM using the analysis kit, and RNase activity was measured. The positive control was prepared to have an RNase reaction without an inhibitor, and the negative control was prepared to have an RNase reaction without the RNA probe. As shown in FIG. 19, an increase in fluorescent signal was observed over time in the RNase reactions with the peptides and the positive control, reaching saturation after approximately 60 minutes. When the fluorescent signal of the positive control was set to 100%, the relative inhibition levels after 60 minutes were estimated to be 54.4% for peptide-1, 53.2% for peptide-2, and 50.3% for peptide-3. Additionally, the inhibition levels of the commercial inhibitors were estimated to be 35.8% for Inhibitor-1 (Biofact Co) and 27.2% for Inhibitor-2 (RNaseOUTTM RNase inhibitor). These results indicate that the optimal time for the inhibition test was determined to be 60 minutes and that the relative inhibitory activity of the synthesized peptides could be evaluated by comparing them to the positive control (no inhibitor). The RNase A inhibitory activity of the three synthesized peptides was analyzed after treating each peptide at a concentration range of 100 pM to 1 µM in the assay kit. As shown in FIG. 20, in the non-inhibitory condition (positive control) where no RNase A inhibitor was applied, a fluorescent signal of 100% was observed. In the case of treatment with peptide-1 (peptide-2 and peptide-3), a quantitative decrease in the fluorescent signal was observed, indicating that RNase A activity was inhibited according to the concentration of peptide-1 (peptide-2 and peptide-3). Compared to the non-inhibitory condition (100%), RNase A activity was reduced by 54.4% for peptide-1 (53.2% for peptide-2, 50.3% for peptide-3) at a concentration of 1 µM. For the commercial RNase inhibitors, RNase A activity was reduced by 35.8% for Inhibitor-1 (Biofact Co) and 27.2% for Inhibitor-2 (referred to as RNaseOUTTM RNase inhibitor) at the same concentration of 1 µM (see FIG. 21). The IC₅₀ values estimated by linear regression were 8.1 µM for peptide-1, 3.6 µM for peptide-2, and 0.4 µM for peptide-3. These results demonstrated that the three synthetic peptides could specifically bind to the active site of RNase A and exhibited similar inhibition levels compared to commercial RNase inhibitors. The RNase A inhibitory activity of the three expressed Fv-antibodies was analyzed after treating each peptide at a concentration range of 100 pM to 1 µM in the analysis kit. In the RNase activity analysis, the IC₅₀ values were calculated to be 90.2 nM for Fv-1, 65.3 nM for Fv-2, and 98.8 nM for Fv-3. The RNase inhibition constants (IC₅₀) for the synthetic peptides and expressed Fv antibodies are summarized in Table 4. These results showed that the Fv antibodies could have significantly increased inhibitory activity against RNase A compared to the same synthetic peptides. As demonstrated in the RNase A binding analysis using the Fv-antibody library (FIG. 2) and the affinity constant (KD) from SPR analysis (FIG. 12), the amino acid sequence of the CDR3 region was confirmed to have high affinity for RNase A. Additionally, mutant strains with CDR1 and CDR2 did not show high affinity for RNase A (FIG. 2). These results indicate that inhibition of RNase requires specific affinity for the active site of RNase A along with steric effects.

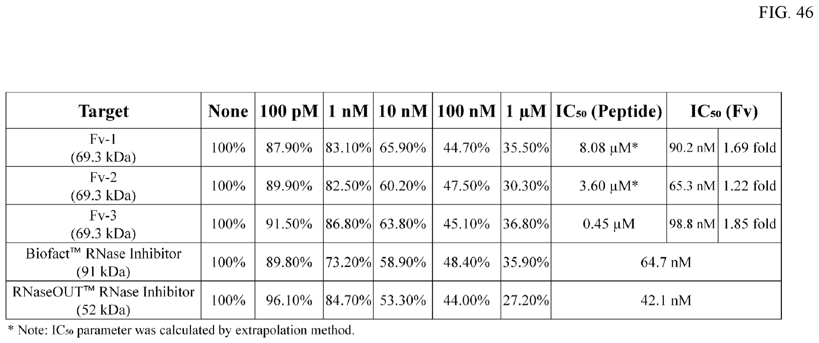

Table 4 is shown in FIG. 46.

### Example 9. Application of synthetic peptides and Fv-antibodies as RNase A inhibitors.

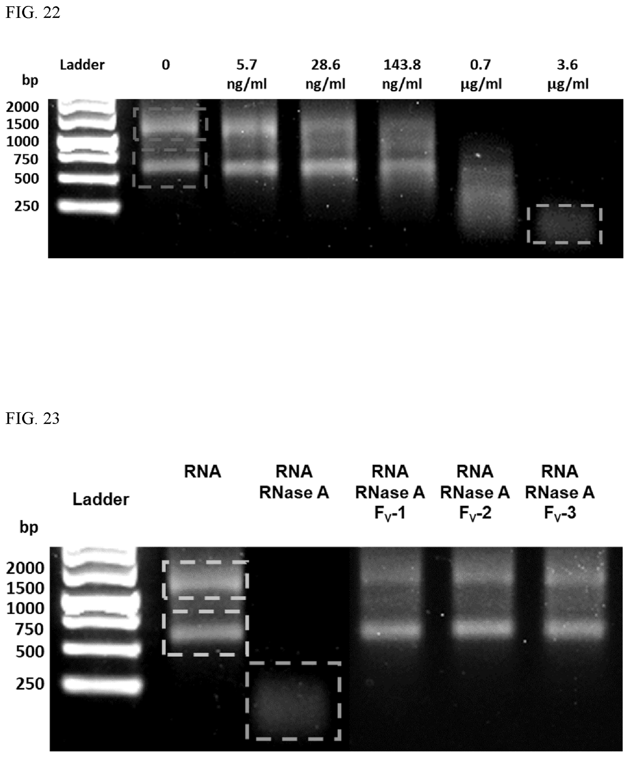

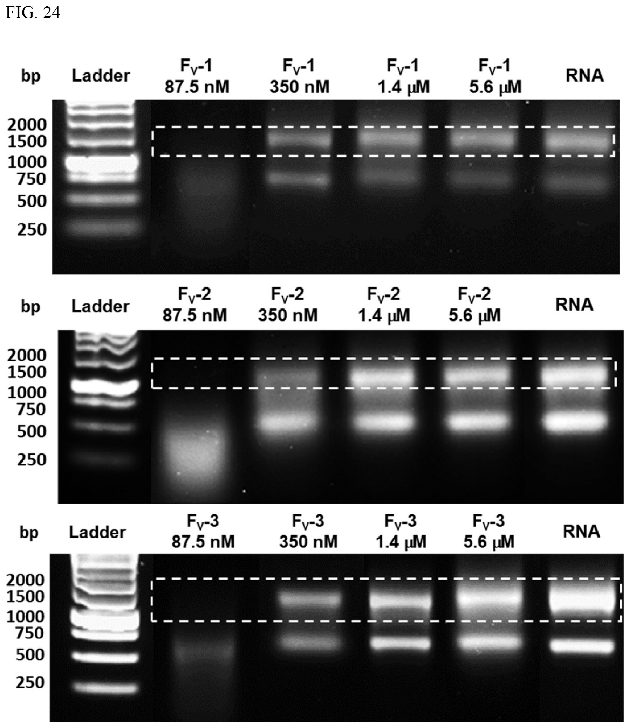

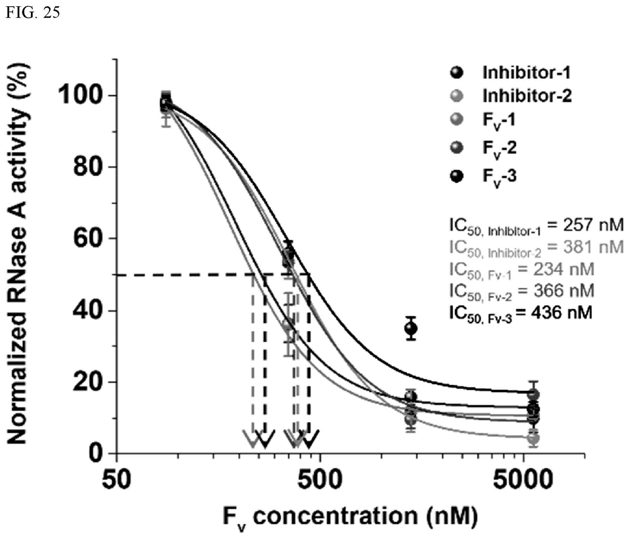

Using RNA samples extracted from HeLa cells, the RNase inhibitory activity of the synthesized peptides was estimated. The RNase inhibitory activity was assessed using agarose gel imaging of the cleaved RNA. To optimize the RNase concentration for the RNase inhibition assay, total RNA was treated with RNase A (600 ng), with RNase A concentrations ranging from 5.7 ng/ml to 3.6 µg/ml, and the cleaved RNA bands were observed after treatment with ETBR (ethidium bromide). As shown in FIG. 22, it was observed that the entire RNA band was cleaved at RNase concentrations of 0.7 µg/ml or higher, confirming that the full RNA bands (1500 bp and 600 bp) were completely cleaved into smaller bands of 1500 bp and 600 bp. The RNase concentration for inhibition analysis was determined to be 0.7 µg/ml, with RNase concentrations above 3.6 µg/ml. The inhibition analysis was performed by mixing total RNA from HeLa cells with RNase (0.7 µg/ml) and Fv-antibody at a concentration of 10 µM, resulting in the observation that the entire RNA was completely cleaved into small RNA bands in the absence of the Fv-antibody. However, as shown in FIG. 23, when Fv antibody (at a concentration of 10 µM) and two types of commercial inhibitors (at a concentration of 10 µM) were mixed with the total RNA, it was observed that the cleavage reaction of RNase A was completely inhibited. The optimal concentration of the Fv-antibody for RNase inhibition was determined by the cleavage reaction with total RNA in the concentration range of 87.5 nM to 5.6 µM for Fv-antibodies (Fv-1, Fv-2, Fv-3). As shown in FIG. 24, the RNase inhibitory activity was estimated to be over 50% at Fv-antibody concentrations of 350 nM or higher. From the analysis of agarose gel images, the estimated IC₅₀ values for RNase A activity inhibition by dose-response curve fitting were 234 µM for Fv-1, 366 µM for Fv-2, and 436 µM for Fv-3. As shown in FIG. 25, these values are similar to those of commercial inhibitors (257 nM for Inhibitor-1 and 381 nM for Inhibitor-2).

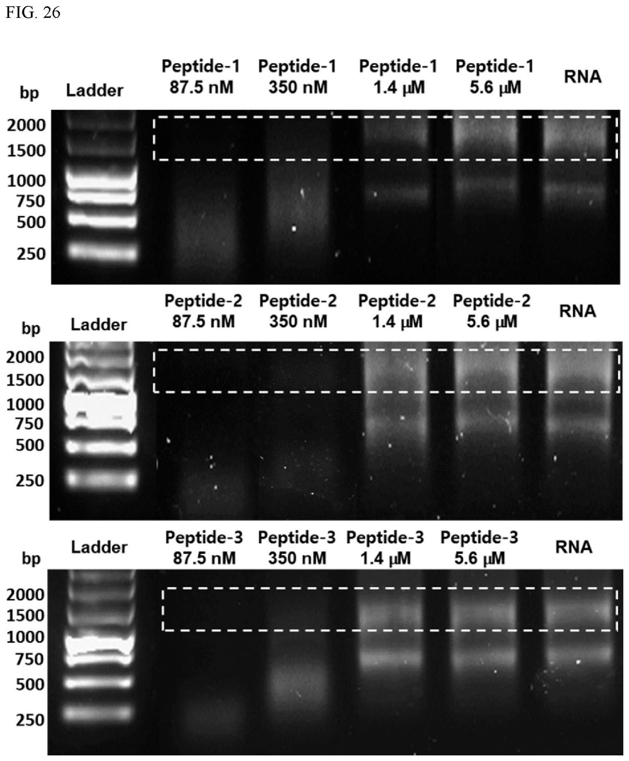

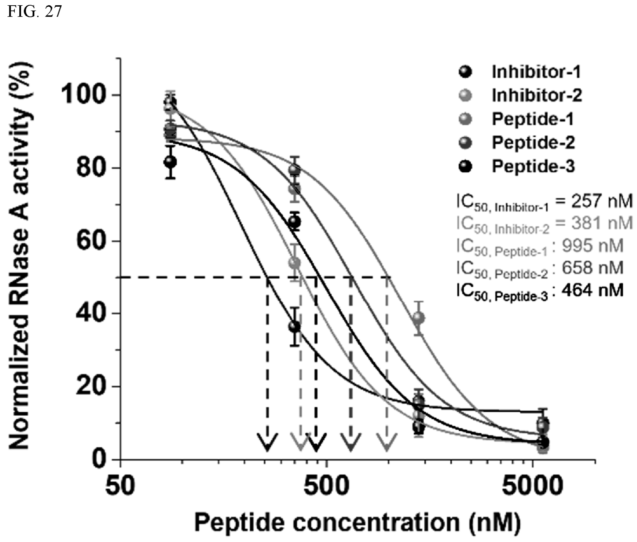

The same RNase inhibition analysis was performed using three synthetic peptides. As shown in FIG. 26, the optimal concentration of the Fv-antibody for RNase inhibition was determined by the cleavage reaction with total RNA for the three synthetic peptides (Peptide-1, Peptide-2, Peptide-3) in the concentration range of 87.5 nM to 5.6 µM. It was estimated from the agarose gel image that the RNase inhibition activity was over 50% at a concentration of synthetic peptides greater than 1.0 µM. As shown in FIG. 27, the IC₅₀ values for the inhibition of RNase A activity were estimated to be 995 nM for Peptide-1, 658 nM for Peptide-2, and 464 nM for Peptide-3, which showed results relatively similar to commercial inhibitors (257 nM for Inhibitor-1 and 381 nM for Inhibitor-2). These results demonstrated that the Fv-antibody can be effectively used to inhibit RNase activity, and it was estimated that the inhibitory activity of the Fv-antibody is similar to that of commercial RNase A inhibitors.

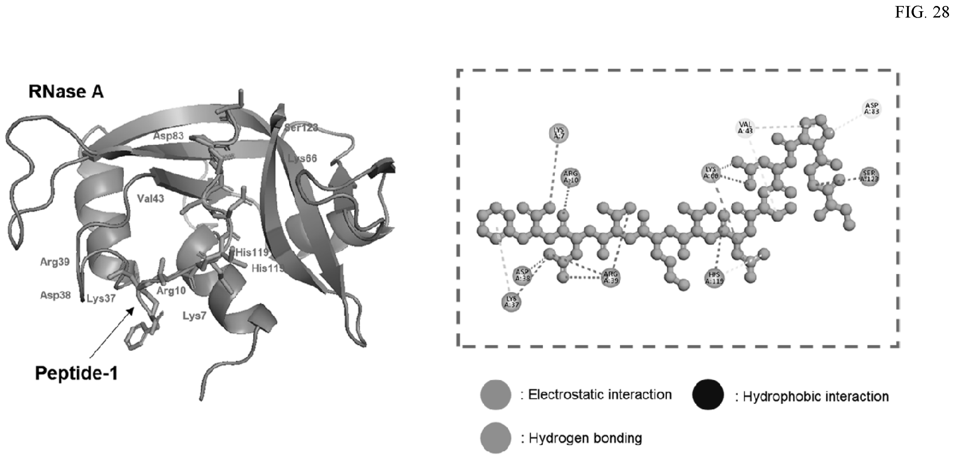

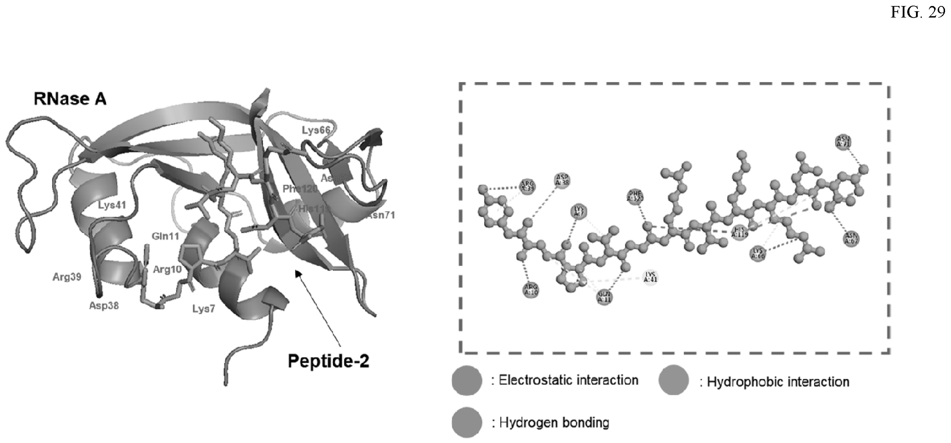

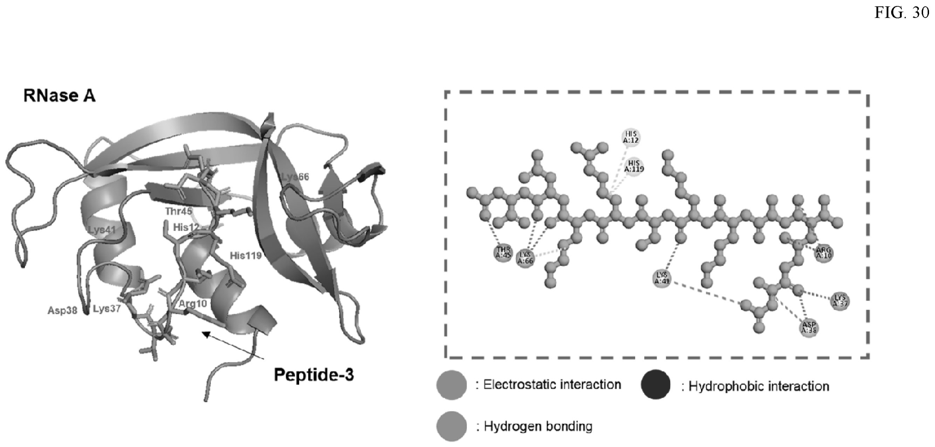

To investigate the interaction between two synthetic peptides (Peptide-1 and Peptide-2) and RNase A, docking simulations were performed using the AutoDock Vina software from Scripps Research. The interactions were visualized and analyzed using version 0.99rc6 of the Pymol software from DeLano Scientific LLC (South San Francisco, CA, USA) and version v21 of the Discovery Studio Visualizer. The structure of RNase A (PDB ID: 1jvt) was obtained from the PDB (www.rcsb.org), and the interactions of Peptide-1 were analyzed as shown in FIG. 28. The docking energies between the three synthetic peptides (Peptide-1, Peptide-2, and Peptide-3) and RNase A were estimated using the AutoDock Vina docking program. The docking energy was estimated by the change in Gibbs free energy (ΔG), which is one of the thermodynamic parameters. The docking results showed that the inhibitor binds to the active site of RNase A, blocking interactions with the RNA substrate. The amino acids of RNase A that interact hydrophobically with Peptide-1 were Lys³⁷ and Val⁴³, while Arg¹⁰, Lys³⁷, Asp³⁸, Arg³⁹, Val⁴³, Asp⁸³, His¹¹⁹, and Ser¹²³ of RNase A formed hydrogen bonds with Peptide-1. Additionally, Lys⁷, Lys⁶⁶, and Lys¹⁰⁴ of RNase A formed electrostatic interactions with Peptide-1. The change in Gibbs free energy (ΔG) between Peptide-1 and RNase A was estimated to be -7.1 kcal/mol. When Peptide-2 interacted with RNase A, the docking results indicated that Peptide-2 also binds to the active site of RNase A (FIG. 29). The amino acids of RNase A that interact hydrophobically with Peptide-2 were Lys⁷, Arg³⁹, Lys⁴¹, Lys⁶⁶, and His¹¹⁹, and Lys⁷, Arg¹⁰, Gln¹¹, Arg³⁹, Lys⁶⁶, Asn⁶⁷, Asn⁷¹, His¹¹⁹, and Phe¹²⁰ of RNase A formed hydrogen bonds with Peptide-2. Furthermore, Asp³⁸ and His¹¹⁹ of RNase A formed electrostatic interactions with Peptide-2. The binding energy (ΔG) between Peptide-2 and RNase A was estimated to be -6.9 kcal/mol. In the case of Peptide-3, the docking results indicated that Peptide-3 also binds to the active site of RNase A, as shown in FIG. 30. The amino acids of RNase A that interact hydrophobically with Peptide-3 were His¹², Lys⁶⁶, and His¹¹⁹, and Arg¹⁰, Lys³⁷, Asp³⁸, Lys⁴¹, Thr⁴⁵, and Lys⁶⁶ of RNase A formed hydrogen bonds with Peptide-3. Additionally, Asp³⁸ and Lys⁴¹ of RNase A formed electrostatic interactions with Peptide-3. The docking energy (ΔG) between Peptide-3 and RNase A was estimated to be -8.5 kcal/mol. The interactions between RNase A and the three synthetic peptides (Peptide-1, Peptide-2, and Peptide-3) are summarized in Table 5.

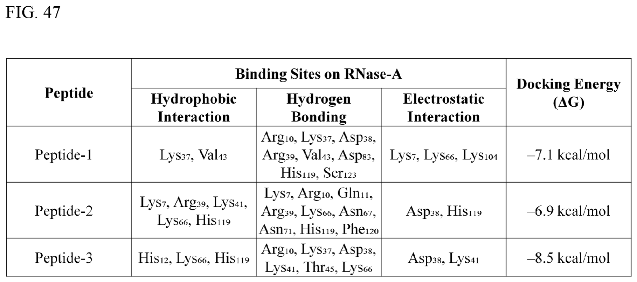

Table 5 is shown in FIG. 47.

Target Fv variants with specific affinity for RNase A were screened from an Fv library that is surface-expressed on the outer membrane of *E. coli.* The target clones were isolated from the Fv library using external magnets, and the magnetic beads were cultured on agar plates. Ultimately, two variants were determined to be target variants with CDR3 sequences different from the template sequence. Based on fitting analysis using an adsorption model, the binding affinities (KD) of the Fv antibodies for the three screened clones were 23.8 ± 2.9 (n=3) µM, 29.4 ± 4.7 (n=3), and 28.7 ± 3.1 (n=3) µM. The CDR3 regions of these three screened Fv antibodies were synthesized as peptides, and the amino acid sequences were determined from the nucleotide sequences of the CDR3 regions. The analysis of RNase A inhibitors was performed using a modified Taq-man probe assay. Compared to the non-inhibitory condition (100%) without inhibitors, RNase A activity was reduced by 54.4% in peptide-1 (53.2% in peptide-2 and 50.3% in peptide-3) (the concentrations of peptide-1, peptide-2, and peptide-3 were 10 µM). These results demonstrated that the three synthetic peptides can specifically bind to the active site of RNase A. In competitive binding analysis using ssDNA (51 bp) as a model RNA, the synthetic peptides (peptide-1, peptide-2, and peptide-3) exhibited binding affinities for RNase A, which were significantly higher than that of the substrate RNA for RNase A. The RNase inhibitory activity of the synthetic peptides was estimated using RNA samples extracted from HeLa cells, and peptide-1 was estimated to be more favorable as an RNase A inhibitor for the total RNA of HeLa cells compared to peptide-2. The RNase inhibitory activity of the synthetic peptides was also estimated using RNA samples extracted from *E. coli* after the transformation of SARS-CoV-2 NP (nucleocapsid protein), and peptide-2 was estimated to be more favorable as an RNase A inhibitor for the total RNA of *E. coli* compared to peptide-1.

As such, the peptide as an RNase A inhibitor according to the present invention can be used in various analytical methods related to RNA. For example, methods for RNA extraction, RNA purification, PCR (polymerase chain reaction), cDNA (complementary DNA) synthesis, and DNA expression methods are included, but are not limited to these.

Unless otherwise defined, all technical and scientific terms used in this specification shall have the same meaning as commonly understood by a person skilled in the art to which the present invention pertains. Generally, the nomenclature used in this specification and the experimental methods described herein are well known and commonly used in the technical field.

The term "antibody" used in this specification encompasses all forms of antibodies known to date, in the broadest sense. That is, as long as an antibody exhibits antigen-binding activity, it includes, without limitation, various antibody structures such as monoclonal antibodies, polyclonal antibodies, multi-specific antibodies (for example, bispecific antibodies), full-length antibodies, and their antigen-binding fragments. The term "antibody" also includes conventional tetravalent antibodies, single-domain antibodies, and their antigen-binding fragments.

The antibody according to the present invention may be in the form of a single-domain antibody (sdAb). A single-domain antibody can include a heavy chain variable domain derived from a heavy-chain-only antibody (for example, VHH from camelids), a light chain derived from a conventional four-chain antibody, a binding molecule that naturally lacks a single domain (for instance, VH or VL), a humanized heavy-chain-only antibody, a human single-domain antibody produced by gene-engineered mice or rats expressing human heavy chain segments, and manipulated domains and single-domain scaffolds derived from sources other than antibodies, without limitation. The sdAb can be derived from any species, including but not limited to mouse, rat, human, camel, llama, eel, fish, shark, goat, rabbit, and bovine. It may also include naturally occurring single-domain antibodies from species other than camelids.

Furthermore, the single-domain antibody is derived from a known naturally occurring single-domain antigen-binding molecule that is a heavy-chain antibody lacking a light chain. Such single-domain molecules are disclosed, for example, in WO 94/04678 and Hamers-Casterman et al., (1993) Nature 363:446-448. The variable domain derived from a heavy-chain molecule that is naturally devoid of a light chain is referred to as VHH in this specification, distinguishing it from the conventional VH of a 4-chain immunoglobulin. Such VHH molecules can be derived from antibodies produced by camelid species, such as camels, llamas, vicuñas, dromedaries, alpacas, and guanacos. Other species outside of camelids may also produce heavy-chain molecules that are naturally devoid of a light chain, and such VHHs are also included within the scope of the present invention.

Furthermore, the single-domain antibody according to the present invention may be a chimeric antibody. Specific chimeric antibodies are described, for example, in U.S. Patent No. 4,816,567; and Morrison et al., Proc. Natl. Acad. Sci. USA, 81:6851-6855 (1984). In some embodiments, the chimeric antibody may include a non-human variable region (for example, a variable region derived from a camelid species, such as a llama) and a human constant region. Additionally, the chimeric antibody may be humanized. Typically, a non-human antibody is humanized to reduce immunogenicity in humans while maintaining the specificity and affinity of the parent non-human antibody. Generally, a humanized antibody includes one or more variable domains in which the CDRs (or portions thereof) are derived from the non-human antibody, and the FRs (or portions thereof) are derived from human antibody sequences. The humanized antibody will optionally include at least a portion of the human constant region. In some embodiments, certain FR residues in the humanized antibody may be substituted with corresponding residues from the non-human antibody (for example, the antibody from which the CDR residues are derived) to restore or improve antibody specificity or affinity.

The term "Fv (Variable region fragment)" used in this specification refers to the antigen-binding region of immunoglobulin G, which consists of three CDR regions (CDR1, CDR2, and CDR3) and a framework region (FR) between the CDR regions, making it the minimal antibody fragment that contains only the heavy chain variable region and/or the light chain variable region. The recombinant techniques for producing Fv are described in international patent application WO88/10649, among others.

The term "CDR (complementarity determining region)" used in this specification refers to the amino acid residues of the antibody variable domain that are necessary for antigen binding. Each variable domain typically has three CDR regions identified as CDR1, CDR2, and CDR3. Additionally, the CDR of the heavy chain variable region may be denoted as HCDR.

The term "auto display" used in this specification refers to a technique for expressing (displaying) variant polypeptides as fusion proteins with at least a portion of a coat protein on the surface of an organism, such as a phage or Escherichia coli. The utility of surface display lies in the ability to rapidly and efficiently screen a large library of randomized protein variants to identify sequences that bind to a target antigen with high affinity. The expression of peptide and protein libraries on the surface of an organism has been used to screen millions of polypeptides to identify those with specific binding characteristics. In particular, it is important to generate diverse libraries of antibodies or antigen-binding proteins for the isolation of high-affinity antibodies. The CDR3 region has been found to often participate in antigen binding. The CDR3 region on the heavy chain is quite diverse in terms of size, sequence, and structural conformation, allowing for the production of various libraries using this region.

"Nucleic acid" encompasses DNA (gDNA and cDNA) and RNA molecules, and the basic building blocks of nucleic acids, nucleotides, include not only natural nucleotides but also analogues with modified sugar or base moieties. The sequence of the nucleic acid coding for the heavy chain and light chain variable regions of the present invention may be modified. Such modifications include the addition, deletion, or non-conservative or conservative substitution of nucleotides.

The amino acid sequence of the antibody or an antigen-binding fragment of the present invention, or the nucleic acid coding for it, is interpreted to include sequences that exhibit substantial identity to the sequence listed in SEQ ID NO:. The term "substantial identity" refers to sequences that, when aligned to maximize correspondence with the sequence of the present invention and analyzed using algorithms commonly used in the art, show 90% or greater homology, more preferably 95% or greater homology, 96% or greater, 97% or greater, 98% or greater, and 99% or greater homology.

Based on this, the antibody or its antigen-binding fragment of the present invention may have 90%, 91%, 92%, 93%, 94%, 95%, 96%, 97%, 98%, 99%, or greater homology compared to the specified sequence or the entirety described in the specification. Such homology can be determined by sequence comparison and/or alignment using methods known in the art. For example, the percentage sequence homology of the nucleic acid or protein of the present invention can be determined using sequence comparison algorithms (i.e., BLAST or BLAST 2.0), manual alignment, or visual inspection.

The DNA encoding the aforementioned antibody can be easily isolated or synthesized using conventional methods (for example, by using an oligonucleotide probe that can specifically bind to the DNA encoding the heavy and light chains of the antibody). Many vectors are available. The vector components generally include, but are not limited to, one or more of a signal sequence, a replication origin, one or more marker genes, enhancer elements, promoters, and transcription termination sequences.

The term "vector" used in this specification includes plasmid vectors, cosmid vectors, bacteriophage vectors, adenovirus vectors, retrovirus vectors, and adeno-associated virus vectors, which are means for expressing a target gene in a host cell. The nucleic acid coding for the antibody in the vector is operatively linked to a promoter. "Operatively linked" refers to a functional connection between a nucleic acid expression regulatory sequence (e.g., promoter, signal sequence, or an array of transcription factor binding sites) and another nucleic acid sequence, whereby the regulatory sequence regulates the transcription and/or translation of the other nucleic acid sequence.

In the case of using prokaryotic cells as hosts, it is common to include a strong promoter capable of driving transcription (for example, the tac promoter, lac promoter, lacUV5 promoter, 1pp promoter, rac5 promoter, amp promoter, recA promoter, SP6 promoter, trp promoter, and T7 promoter), a ribosome binding site for the initiation of translation, and transcription/translation termination sequences. Additionally, for example, when using eukaryotic cells as hosts, promoters derived from the genome of mammalian cells (e.g., metallothionein promoter, actin promoter, human hemoglobin promoter, and human muscle creatine promoter) or promoters derived from mammalian viruses (e.g., adenovirus late promoter, vaccinia virus 7.5K promoter, SV40 promoter, cytomegalovirus (CMV) promoter, HSV tk promoter, mouse mammary tumor virus (MMTV) promoter, HIV LTR promoter, Moloney virus promoter, Epstein-Barr virus (EBV) promoter, and Rous sarcoma virus (RSV) promoter) can be utilized, and they generally have a polyadenylation sequence as a transcription termination sequence.

In some cases, the vector may be fused with another sequence to facilitate the purification of the antibody expressed therefrom. The fused sequence may include, for example, glutathione S-transferase (Pharmacia, USA), maltose-binding protein (NEB, USA), FLAG (IBI, USA), and 6x His (hexahistidine; Quiagen, USA).

The vector includes an antibiotic resistance gene that is commonly used in the industry as a selectable marker, such as resistance genes for ampicillin, gentamicin, carbenicillin, chloramphenicol, streptomycin, kanamycin, geniticin, neomycin, and tetracycline.

The present invention, from another perspective, relates to transformed cells using the aforementioned vector. The cells used to generate the antibody of the present invention may be prokaryotic, yeast, or higher eukaryotic cells, but are not limited thereto.

Prokaryotic host cells such as strains of the genus *Bacillus,* including *Escherichia coli, Bacillus subtilis,* and *Bacillus thuringiensis,* as well as *Streptomyces, Pseudomonas, Proteus mirabilis,* and *Staphylococcus,* can be used.

The present invention, from another aspect, relates to a method for producing an antibody or its antigen-binding fragment, comprising the steps of (a) culturing cells; and (b) recovering the antibody or its antigen-binding fragment from the culture medium and/or the cells.

The cells can be cultured in various media. Any commercially available medium can be used as a culture medium without limitation. Additionally, all other necessary supplements known to those skilled in the art may be included at appropriate concentrations. Culture conditions, such as temperature and pH, have already been used in conjunction with the selected host cells for expression, which will be apparent to those skilled in the art.

The term "transformation" used in this specification refers to the introduction of a vector containing nucleic acid that encodes a target protein into a host cell, allowing the protein encoded by the nucleic acid to be expressed within the host cell. Transformed nucleic acids include all forms, whether inserted into the chromosomes of the host cell or located outside the chromosomes, as long as they can be expressed within the host cell. Furthermore, the nucleic acid includes DNA and RNA that encode the target protein. The nucleic acid can be introduced into the host cell in any form, as long as it can be expressed. For example, the nucleic acid can be introduced into the host cell in the form of an expression cassette, which is a gene construct that contains all the elements necessary for its own expression. The expression cassette typically includes a promoter, transcription termination signal, ribosome binding site, and translation termination signal that are operably linked to the nucleic acid. The expression cassette may be in the form of a self-replicating expression vector. Additionally, the nucleic acid may be introduced into the host cell in its own form, operably linked to the sequences necessary for expression in the host cell.

The recovery of the antibody or its antigen-binding fragment can be achieved by removing impurities, for example, through centrifugation or ultrafiltration, and the resulting product can be purified using methods such as affinity chromatography. Additional purification techniques, such as anion or cation exchange chromatography, hydrophobic interaction chromatography, and hydroxylapatite chromatography, may also be employed.

### < Peptide or antibody specifically binding to RNase A >

In the present invention, the RI with specific binding affinity for the active site of RNase A was screened from an Fv-antibody library that is surface-expressed on the outer membrane of Escherichia coli. As shown in FIG. 1, the Fv-antibody consists of the variable region of the IgG heavy chain, which is composed of three complementarity-determining regions (CDRs) and framework regions (FR) between the CDRs. To form the Fv-antibody library, the CDR3 region, which has 11 amino acid residues, was randomized by site-directed mutagenesis.

As previously reported, the Fv-antibody library was expressed on the outer membrane of *E. coli* using surface display technology, achieving a diversity of approximately 10⁶ clones/library or more. The surface display technology transports the translated protein through the beta-barrel of AIDA-1 to the outer membrane of *E. coli* to express the target protein, and the expression yield of surface display is estimated to be about 10⁵ proteins/E. *coli* or more. This surface display technology has been used for the expression of Z-domain, streptavidin, lipase, Ro/SSA antigen, casein, and others. Using surface display technology, the Fv-antibody library was densely expressed on the outer membrane of *E. coli* (*>* 10⁵ proteins/E. *coli*)*.* When fluorescently labeled antigens (target antigens) are treated with the surface-displayed Fv-antibody library, *E. coli* with target Fv-variants generate sufficiently strong fluorescence to be efficiently isolated using flow cytometry without the need for repetitive panning processes.

In the present invention, the surface-expressed Fv-antibody library was used for the screening of Fv-variants that specifically bind to RNase A. The antibody library was prepared by introducing site-specific mutations in the CDR3 region of the Fv-antibody. Subsequently, fluorescently labeled RNase A was treated to screen *E. coli* for the target Fv-variants, and then the specific binding affinity of the Fv-variants on the *E. coli* outer membrane was estimated using flow cytometry. The screened Fv-variants were then expressed as soluble Fv antibodies and synthesized into peptides to have the amino acid sequence of the CDR3 region of the screened Fv-variants. The binding affinity constant (KD) of the synthesized peptides containing the CDR3 region of the expressed Fv-antibody and the screened Fv-variants was estimated using an SPR biosensor that included immobilized RNase A. The activity of the Fv-antibody and the RI generated from the synthesized peptides was measured through a cleavage assay of the phosphodiester bond of RNA and an RNase activity assay using RNA probes labeled with a fluorophore and a quencher, and compared with two commercial RIs. Finally, the activity of the RI made from the expressed Fv-antibody and the synthesized peptides was demonstrated by an RNA cleavage assay using total RNA from HeLa cells.

### Example 1. Materials

Ribonuclease A (Type II-A) from bovine pancreas, cytidine 2':3'-cyclic monophosphate monosodium salt (2',3'-cCMP), and bovine serum albumin were purchased from Sigma-Aldrich Korea (Seoul, Korea). Luria-Bertani (LB) broth and agar media were obtained from Duchefa (Haarlem, Netherlands). Synthetic peptides (Peptide-1, 2, and 3) with 90% purity (Table 2) were purchased from Peptron Co. (Daejeon, Korea). RNaseOUT^{™} recombinant ribonuclease inhibitor, dynabeads^{™} M-280 tosylactivated, Phusion High-Fidelity DNA polymerase, and other PCR reagents were obtained from Thermo Fisher Scientific Inc. (Waltham, MA, USA). Biofact^{™} RNase inhibitor was purchased from Biofact Co. (Daejeon, Korea). Primers for the FV-antibody library construction were custom synthesized by Bionics (Seoul, Korea). Klenow DNA polymerase and NEBuffer^{™} 2 were obtained from New England Biolabs (Ipswich, MA, USA). PCR purification mini kits and plasmid extraction kits were purchased from Favorgen Biotech Corp. (Pingtung, Taiwan). A 100 kDa Amicon ultra centrifugal filter was obtained from Millipore (MA, USA). BK7 glass for SPR analysis was purchased from iCLUEBiO (Gyeonggido, Korea). TdTomato-labeled Fv-antibody fusion protein plasmids were custom synthesized by Cosmogenetech (Seoul, Korea).

### Example 2. Preparation of Fv-Antibody Library

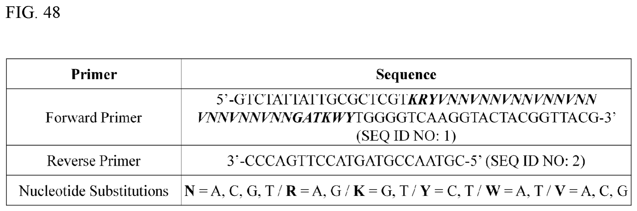

Table 6 is shown in FIG. 48.

A synthesized single-stranded DNA primer containing a randomized CDR3 sequence (75 bp, 0.5 µM) was first mixed with a reverse primer (22 bp) and NEBuffer2TM, and the primer hybridization was performed by heating (95°C for 5 minutes) and cooling (36°C for 1 minute). The primer sequences used for constructing the Fv-antibody library are summarized in Table 6. After hybridization, primer polymerization was performed as follows: Klenow (exo-) DNA polymerase (3 µL), NEBuffer2TM (10 µL), dNTPs (10 mM, 8 µL), and the hybridized primers were mixed with deionized water (DW) to a total volume of 200 µL. Primer polymerization was carried out (37°C for 15 minutes) followed by enzyme inactivation (75°C for 20 minutes). Subsequently, the Fv-antibody library primers with the randomized CDR3 sequence were collected using a PCR purification mini kit. The Fv-antibody library plasmids containing the randomized CDR3 sequence were synthesized through PCR. The template plasmid (pST009, 150 ng), dNTPs (10 mM, 8 µL), HF buffer (10 µL), purified Fv-antibody library primers (150 ng), and Phusion high-fidelity DNA polymerase (0.5 µL) were mixed, and PCR was performed under the following conditions: (1) initial denaturation (98°C for 1 minute), (2) denaturation (98°C for 30 seconds), (3) annealing (68°C for 1 minute), (4) elongation (72°C for 5 minutes), (5) 30 cycles (repeating steps (2) to (4)), and (6) final extension (72°C for 10 minutes). After PCR, DpnI enzyme was added to digest the template plasmid. The Fv-antibody library plasmids containing the randomized CDR3 sequence were finally purified using a 100 kDa Amicon Ultra centrifugal filter. The surface expression of the Fv-antibody library on the outer membrane of Escherichia coli BL21(DE3) was prepared by transforming the purified Fv-antibody library plasmids into electrocompetent cells. Electroporation was performed for bacterial transformation. The electroporated *E. coli* cells were cultured overnight in LB medium (10 mL) containing 50 µg/mL kanamycin (37°C, 16 hours, 150 rpm). To express the Fv-antibody library with the randomized CDR3 sequence on the outer membrane of *E. coli* cells, the overnight cultured cells were re-cultured in LB medium (10 mL) containing kanamycin (50 µg/mL), 99% β-mercaptoethanol (8 µL), and ethylenediaminetetraacetic acid (5 µM) (37°C, 2.5 hours, 150 rpm). Isopropyl thio-β-galactoside (IPTG) induction was performed as follows to express the Fv-antibody library with the randomized CDR3 sequence on the outer membrane of *E. coli* cells (Fv-variants): 1 mM IPTG was added and cultured (30°C, 3 hours, 120 rpm).

### Example 3. Screening of Fv-antibodies specifically binding to RNase A

Before screening for FV antibodies against RNase A, RNase was first immobilized on magnetic beads. The immobilization of RNase on the magnetic beads was performed by mixing M-280 tosylactivated magnetic beads (5 mg, 165 µL), RNase A (100 µg), 0.1 M borate buffer (pH 9.5, 150 µL), and 3 M ammonium sulfate (100 µL) (37°C, 16 hours, 120 rpm). The washing step with PBS was carried out using an external magnet, and the immobilized RNase A-magnetic beads were collected and resuspended in a PBS solution.

The screening of Fv variants that specifically bind to RNase A was performed as follows: (1) Fv variants (200 µL, OD600 nm = 0.5) and immobilized RNase A-magnetic beads (10 mg/mL, 10 µL) were mixed at room temperature and 15 rpm for 1 hour, (2) an external magnet was used to collect Fv variants that specifically bind to the RNase A-immobilized magnetic beads, followed by 10 washing steps with 0.01% PBST and PBS, (3) the isolated Fv variant-bound RNase A magnetic beads were resuspended in 100 µL PBS, and (4) the resuspended magnetic beads were spread on an agar plate to obtain Fv variants that specifically bind to RNase A, as illustrated in FIG. 3.

### Example 4. Flow Cytometry

To select Fv variants that specifically bind to RNase A, 44 Fv variant clones selected on an agar plate were cultured and mixed with 5 µM of FITC-labeled RNase A (RT, 1h, 15 rpm).

FITC-labeled RNase A was synthesized by mixing FITC (0.5 mg) and RNase A (1 mg) in 2 ml of PBS buffer under incubation conditions (RT, 24h, 15 rpm), and the FITC-labeled RNase A was purified using a 10 kDa Amicon Ultra centrifugal filter. After binding the Fv variant to the FITC-labeled RNase A, the Fv variant cells were washed three times with PBS buffer by centrifugation (3,000 x g, 3 minutes) and finally resuspended in PBS. The fluorescence intensity of the Fv variant was measured using a FACSCalibur flow cytometer (Becton-Dickinson, Franklin Lakes, NJ, USA) at an excitation wavelength of 485 nm and an emission wavelength of 535 nm, as shown in FIG. 3.

### Example 5. Expression of Antibody

The recombinant plasmids (pJS001, pJS002, and pJS003) encoding the open reading frames of the screened Fv variants with binding activity to RNase A, TdTomato, and His-tag fusion proteins were custom synthesized by Cosmogenetech as shown in FIG. 8. The three plasmids were transformed into electrocompetent cells via electroporation, and Fv antibodies (His-tag-TdTomato-VH) were expressed in the transformed *E. coli* cells. The three transformed *E. coli* cells were cultured overnight in 20 ml of high-salt LB medium containing 1 mM IPTG and 30 µg/ml carbenicillin (30°C, 16 hours, 150 rpm). The cultured *E. coli* pellet was collected by centrifugation (3,000 xg, 3 minutes) and then resuspended in 30 ml of 3M urea containing binding buffer (1M NaCl, 5 mM Tris-HCl, 0.5 mM EDTA). The resuspended *E. coli* cells were lysed using an ultrasonic processor (Vibra cell VCX-130, Sonics, USA), and the lysate containing the Fv antibodies was collected by centrifugation (25,000 xg, 10 minutes, 4°C). The TdTomato-labeled Fv antibodies were purified using a His-tag purification column (Roche, Basel, Switzerland) and elution buffer (3M urea binding buffer with 100 mM imidazole). Dialysis (4°C, 16 hours, 100 rpm) was performed to remove urea and imidazole from the purified Fv antibody protein solution.

### Example 6. SPR Biosensor Analysis

The analysis of RNase A activity inhibition based on 2',3'-cyclic CMP (cCMP) was performed by measuring the absorbance of cCMP at a wavelength of 286 nm. A mixture was prepared by combining Tris-acetate buffer (500 mM, pH = 6.5, 200 µL), EDTA (5 mM, 200 µL), 2',3'-cCMP (10 mM, 100 µL), RNase A (10 µg/mL, 100 µL), Fv variant (or synthetic peptides in a concentration range of 0.1 nM - 1 µM), and DW (filled up to 1000 µL). The absorbance at 286 nm was measured using a Lamda35 UV-VIS spectrophotometer (Perkin-Elmer, MA, USA). The reaction was initiated by adding RNase A to the mixture, and the absorbance was measured every 3 minutes for a total of 30 minutes.

Binding analysis of the expressed Fv antibody and synthesized peptides against RNase A was performed using an SPR (Surface Plasmon Resonance) biosensor from I-Cluebio (Seongnam, Korea). RNase A was immobilized on the gold surface of the SPR chip used in the SPR biosensor to conduct the binding analysis. For the manufacture of the SPR chip, a 2 nm thick titanium adhesive layer and a 48 nm thick gold surface were consecutively sputter-coated onto BK-7 glass (1×1 cm²). As shown in FIG. 11, RNase A (100 µg/ml) from bovine pancreas was incubated on the gold surface SPR chip at 4°C for 16 hours. To analyze the binding of the expressed Fv antibody to RNase A, Fv antibodies in the concentration range of 0.37 - 100 nM were reacted using PBS buffer as the cell through association (10 minutes), equilibrium (5 minutes), and dissociation (10 minutes) steps. The washing buffer and total SPR measurements were performed at a flow rate of 15 µL/min. For the binding analysis of the synthesized peptides against RNase A, peptides in the concentration range of 0.37 - 100 nM were reacted using PBS buffer as the washing buffer through association (10 minutes), equilibrium (5 minutes), and dissociation (10 minutes) steps, and total SPR measurements were conducted at a flow rate of 15 µL/min.

The analysis of RNase inhibitory activity of Fv antibodies and synthetic peptides was performed using RNA samples extracted from HeLa cells as follows: (1) A mixture of RNA samples extracted from HeLa cells (600 ng) was prepared at concentrations of 87.5 nM - 5.6 µM and incubated at 37°C for 30 minutes; (2) Electrophoresis was conducted in a 1% agarose gel under 90 V for 40 minutes. The estimation of RNase inhibitory activity was carried out by gel imaging of the cleaved RNA using a ChemiDoc XPS (Bio-Rad, CA, USA).

### Example 7. Screening of Fv antibodies with affinity for RNase

Target Fv variants with specific affinity for RNase A were screened from an Fv library that is surface-expressed in the outer membrane of *E. coli.* The Fv-antibody library was composed of three CDR regions (CDR1, CDR2, CDR3), and the CDR3 region, which has 11 amino acid residues, was designed to have a randomized amino acid sequence as shown in FIG. 1. After surface expression of the Fv-antibody library, the diversity of the Fv-antibody library was estimated to be over 10⁶ clones/library through random clone selection and genetic analysis of the CDR3 region. To screen for targeted Fv variants with affinity for RNase A, fluorescently labeled RNase A was treated with the Fv-antibody library.

As shown in FIG. 2, the Fv-variants were observed to have significantly higher fluorescence intensity compared to mutant strains possessing Fv-antibodies composed solely of natural *E. coli* and CDR1&2, where the Fv-antibody was not expressed on the surface. The Fv-variants in the dotted rectangular area of the Fv-antibody library (in red) were indicated as target clones with Fv-antibodies that have specific affinity for RNase A. It was observed that the ratio of clones at the same fluorescence level was significantly higher. Compared to natural *E. coli* (5.2%) and mutant strains (13.2%), the ratio of clones in the Fv-antibody library (66.5% of the total population) was observed to be significantly higher. These results imply that the Fv-antibody library contains target Fv-variants with high affinity for RNase A and that these target variants were isolated from the Fv-antibody library using magnetic beads with immobilized RNase A.

To isolate the target Fv variant for RNase A, RNase A was covalently immobilized on tosyl-activated magnetic beads, and the Fv library was cultured together with the magnetic beads as shown in FIG. 3. The target clones were separated from the Fv library using an external magnet, and the magnetic beads were cultured on an agar plate. Randomly selected clones were analyzed using a flow cytometer with fluorescently labeled RNase A.

As shown in FIG. 4, using fluorescently labeled RNase A, three clones and nine variants (clones 5, 14, 21, 23, 26, 29, 30, 39) exhibited significantly high fluorescence signals in flow cytometry analysis. Among these nine variants, six were confirmed to have non-targeted CDR3 sequences. Finally, three variants were determined to be targeted variants with CDR3 sequences different from the template sequence. Based on these results, clones 5, 23, and 39 were selected as targeted Fv antibodies.

The sequence of the heavy chain variable region of clone 5 (Fv-1) is as follows.
CDR1: TYGIQ (SEQ ID NO: 3)
CDR2: WIHAGTGGTKYSRKFQG (SEQ ID NO: 4)
CDR3: CGPDSDVMTDF (SEQ ID NO: 5)
VH:

EVQLVESAAEVRRPGASVKITCKASGYSFSTYGIQWMRQAPGQRPEWLGWIHAGTGGT
KYSRKFQGRITITRDTSANTVYLDLNSLTSEDTAVYYCARCGPDSDVMTDFWGQGTTVT
VSS (SEQ ID NO: 8)

The sequence of the heavy chain variable region of clone 23 (Fv-2) is as follows.
CDR1: TYGIQ (SEQ ID NO: 3)
CDR2: WIHAGTGGTKYSRKFQG (SEQ ID NO: 4)
CDR3: YGPVGRVKRDD (SEQ ID NO: 6)
VH:

EVQLVESAAEVRRPGASVKITCKASGYSFSTYGIQWMRQAPGQRPEWLGWIHAGTGGT
KYSRKFQGRITITRDTSANTVYLDLNSLTSEDTAVYYCARYGPVGRVKRDDWGQGTTVT
VSS (SEQ ID NO: 9)

The sequence of the heavy chain variable region of clone 39 (Fv-3) is as follows.
CDR1: TYGIQ (SEQ ID NO: 3)
CDR2: WIHAGTGGTKYSRKFQG (SEQ ID NO: 4)
CDR3: DGTAKKSRKDD (SEQ ID NO: 7)
VH:

EVQLVESAAEVRRPGASVKITCKASGYSFSTYGIQWMRQAPGQRPEWLGWIHAGTGGT
KYSRKFQGRITITRDTSANTVYLDLNSLTSEDTAVYYCARDGTAKKSRKDDWGQGTTVT
VSS (SEQ ID NO: 10)

The affinity of the Fv antibodies screened from clone 5, clone 23, and clone 39 was analyzed by treatment with RNase A at varying concentrations: the screened variant (clone 5), a mutant strain with only CDR1 & CDR2, and a natural *E. coli* strain. In the case of the variant with only CDR1 & CDR2 and the natural *E. coli* strain, no significant differences were observed in the baseline and fluorescence signals. For the screened Fv variant (clone 5), it was observed that the fluorescence signal quantitatively increased according to the concentration of the fluorescently labeled RNase A, indicating specific binding of RNase A to the screened Fv antibody. Based on fitting analysis using an adsorption model, the binding affinity (KD) of the Fv antibody for clone 5 was estimated to be 23.8+2.9 (n=3) µM. The same experiment was conducted for the other screened clones (clone 23 and clone 39), and it was observed that the fluorescence signal quantitatively increased according to the concentration of the fluorescently labeled RNase A.

In FIGs. 6 and 7, the mutant strains containing only CDR1 and CDR2 showed no significant difference in baseline and fluorescence signal compared to the natural *E. coli* strain. These results indicate that the specific binding of RNase A occurred with the screened clones (clones 23 and 39) of the Fv antibody, and the binding affinities (KD) of the Fv antibodies from clone 23 and clone 39 were estimated to be 29.4 ± 4.7 µM (n=3) and 28.7 ± 3.1 µM (n=3), respectively. From these results, it is presumed that the binding of fluorescently labeled RNase A occurs in the CDR3 region of the screened Fv antibodies, as the mutant strains with only CDR1 and CDR2 and the natural *E. coli* strain were determined not to have specific binding affinity. However, it was confirmed that CDR1 and CDR2 do not directly participate in the binding of RNase but cause steric hindrance within the active site of RNase. Additionally, this effect was found to occur even in cases other than SEQ ID NO: 3 and SEQ ID NO: 4. In other words, since CDR3 was directly involved in binding to RNase, CDR1 and CDR2 acted similarly in cases with different amino acid sequences.

### Example 8. Inhibitory activity of synthesized peptides and expressed Fv-antibodies on RNase A.

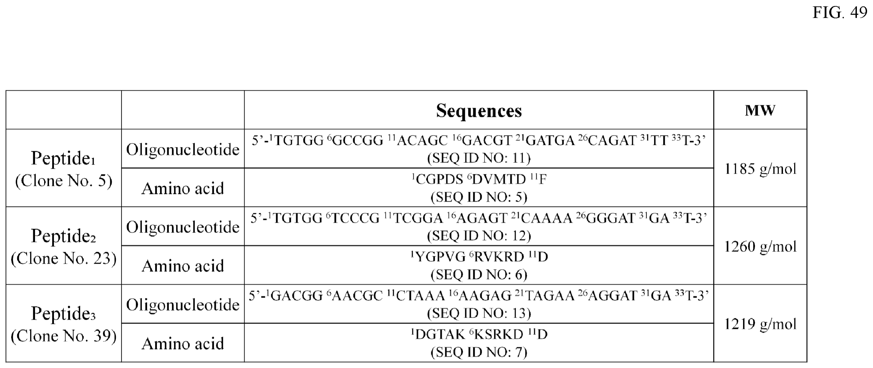

Table 7 is shown in FIG. 49.

Using the screened CDR3 amino acid sequences, the CDR3 regions of the screened Fv antibodies were synthesized as peptides, and the amino acid sequences were determined from the nucleotide sequences of the CDR3 regions as summarized in Table 7. The clones were internally expressed using recombinant plasmids as shown in FIG. 8. These synthetic peptides (Peptide-1, Peptide-2, Peptide-3) and Fv antibodies (Fv-1, Fv-2, Fv-3) were identical to the amino acid sequences of the CDR3 regions of the screened clones. The expression of the Fv antibodies was confirmed using SDS-PAGE, and the molecular weight of the Fv antibodies was estimated to be 69.2 kDa as shown in FIG. 9. The affinity constant (KD) of the synthetic peptides and the expressed Fv antibodies against RNase A was estimated using an SPR biosensor. As shown in FIG. 10, RNase was immobilized on the Au chip of the SPR biosensor, and as shown in FIG. 11, synthetic peptides in the concentration range of 0.37-10 µM were reacted through binding, equilibrium, and dissociation steps. The SPR signal was plotted according to the concentration of the synthetic peptides, and the affinity constants (KD) of these synthetic peptides were calculated through a fitting process. The affinity constants (KD) were calculated to be 1.3+ 0.1 µM for Peptide-1, 1.3+ 0.3 µM for Peptide-2, and 3.7± 1.1 µM for Peptide-3. These results indicate that the three synthetic peptides can specifically bind to the active site of RNase A.

Using an Au chip with immobilized RNase A, the same SPR measurements were performed for the expressed Fv-antibodies in the concentration range of 3.70 - 100 nM. As shown in FIG. 12, the affinity constants (KD) were calculated to be 17.5 ± 4.10 nM for Fv-1, 28.8 ± 9.7 nM for Fv-2, and 33.9 ± 8.9 nM for Fv-3. These results indicate that the expressed Fv-antibodies can have significantly higher binding affinity for RNase A compared to synthetic peptides with the same amino acid sequence in the CDR3 region.

RNase A is known to degrade RNA by cleaving the phosphodiester bonds between nucleotides. The cleavage of the phosphodiester bond occurs through the following two-step reaction: (1) the formation of a 5'-phosphoryl transfer state and (2) the subsequent degradation of a 2',3'-cyclic phosphate intermediate. During this RNA cleavage reaction, histidine residues (His12 and His119) are known to participate in the electron transfer reaction. In the homology of this RNA cleavage reaction, the hydrolysis of 2',3'-cyclic CMP (cCMP) to 3'-CMP has been used to estimate RNase A activity. Specifically, it was utilized that the absorbance at 286 nm quantitatively increases as 3'-CMP increases due to the hydrolysis of cCMP, as shown in FIG. 13. In this invention, the active unit of the inhibitor (1 unit) is defined as the 50% inhibition of RNase A (5 ng) using 0.2 mM cCMP in 100 nM Tris-acetate (pH 6.5) for 10 minutes at 25°C. To estimate the RNase A inhibitory activity of the expressed Fv antibody using the cCMP assay, the expressed Fv antibody (Fv-1) was used as an inhibitor of RNase A in a concentration range of 0.1 nM to 1 µM, and cCMP was observed at 286 nm using a Lamda35 UV-VIS spectrophotometer (Perkin-Elmer, MA, USA). As shown in FIG. 14, the absorbance increased with reaction time, reaching saturation after 18 minutes, confirming that the inhibitory activity increased quantitatively with the concentration of the Fv antibody. The same experiment was performed for other Fv antibodies (Fv-2 and Fv-3) to observe the inhibitory activity of the Fv antibodies. The inhibition curves of the Fv antibodies were plotted linearly, and the IC₅₀ values were calculated by linear regression, as shown in FIG. 15. The IC₅₀ values were estimated to be 194 nM for Fv-1, 236 nM for Fv-2, and 196 nM for Fv-3.

The same cCMP-based analysis was performed on synthesized peptides in the concentration range of 0.1 nM to 1 µM. As shown in FIG. 16, the absorbance increased with reaction time, reaching saturation after 18 minutes, and it was confirmed that the inhibitory activity quantitatively increased according to the concentration of the Fv antibody. The IC₅₀ values were estimated to be 607 nM for peptide-1, 373 nM for peptide-2, and 356 nM for peptide-3, as shown in FIG. 17. The RNase inhibitory constants (IC₅₀) for the synthesized peptides and the expressed Fv antibodies are summarized in Table 8.

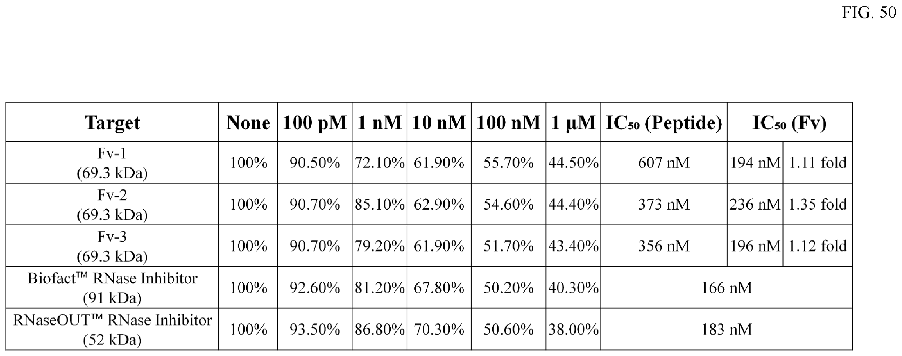

Table 8 is shown in FIG. 50.

The activity of RNase A, which degrades RNA, was evaluated using specially designed RNA probes. This RNase A analysis was performed using a commercial RNase activity test kit based on a modified Taq-man probe analysis (ab273299) from Abcam (Cambridge, UK). For the comparison of inhibitory activity, three synthetic peptides (Peptide-1, Peptide-2, Peptide-3) and three expressed Fv-antibodies (Fv-1, Fv-2, and Fv-3) were compared using two types of commercially available RNase inhibitors from BiofactTM (Daejeon, South Korea) and two types of RNaseOUTTM from Thermofisher (USA). The probes for the RNase inhibition analysis contain RNA probes with fluorescent dyes and fluorescent quenchers at each end of the RNA nucleotide sequence.

As shown in FIG. 18, after the degradation of the RNA backbone, the fluorescent dye was released, generating a fluorescent signal. When RNase A was completely inhibited, the RNA backbone was maintained, and the fluorescent signal was completely absorbed by the fluorescent quencher. To estimate the RNase A inhibitory activity of the three synthesized peptides, each peptide was treated at a concentration of 1 µM using an assay kit, and the RNase activity was evaluated. The positive control was prepared to have an RNase reaction without the inhibitor, while the negative control was prepared to have an RNase reaction without the RNA probe.

As shown in FIG. 19, an increase in fluorescence signal was observed over the treatment time in the RNase reaction with the peptides and positive control, reaching saturation after approximately 60 minutes. When the fluorescence signal of the positive control was set to 100%, the relative inhibition levels after 60 minutes were estimated to be 54.4% for Peptide-1, 53.2% for Peptide-2, and 50.3% for Peptide-3. Additionally, the inhibition levels of commercial inhibitors were estimated to be 35.8% for Inhibitor-1 (Biofact Co) and 27.2% for Inhibitor-2 (RNaseOUTTM RNase inhibitor). These results indicate that the optimal time for the inhibition test was determined to be 60 minutes, and the relative inhibitory activity of the synthesized peptides can be evaluated by comparing them to the positive control (no inhibitor). The RNase A inhibitory activity of the three synthesized peptides was analyzed after treating each peptide at a concentration range of 100 pM to 1 µM with the RNase A from the assay kit.

As shown in FIG. 20, in the case of the non-inhibitory condition (positive control) without any RNase A inhibitor, a fluorescence signal of 100% was observed. When treated with peptide-1, peptide-2, and peptide-3, a quantitative decrease in the fluorescence signal was observed, indicating that RNase A activity was inhibited depending on the concentrations of peptide-1, peptide-2, and peptide-3. Compared to the non-inhibitory condition (100%), RNase A activity decreased by 54.4% with peptide-1, 53.2% with peptide-2, and 50.3% with peptide-3.

As shown in FIG. 21, in the case of commercial RNase inhibitors, the activity of RNase A was reduced by 35.8% with Inhibitor-1 (Biofact Co) and by 27.2% with Inhibitor-2 (referred to as RNaseOUTTM RNase inhibitor) at the same concentration of 1 µM.

The IC₅₀ values estimated by linear regression were 8.1 µM for peptide-1, 3.6 µM for peptide-2, and 0.4 µM for peptide-3. These results demonstrate that the three synthetic peptides can specifically bind to the active site of RNase A and exhibit a similar level of inhibition compared to commercial RNase inhibitors.

The RNase A inhibitory activity of three expressed Fv-antibodies was analyzed after treating each peptide at a concentration range of 100 pM to 1 µM against RNase A in the analytical kit. In the analysis of RNase activity, the IC₅₀ values were calculated to be 90.2 nM for Fv-1, 65.3 nM for Fv-2, and 98.8 nM for Fv-3. The RNase inhibition constants (IC₅₀) for the synthetic peptides and the expressed Fv antibodies are summarized in Table 9. These results demonstrated that the Fv antibodies could significantly increase the inhibitory activity against RNase A compared to the same synthetic peptides. As evidenced by the RNase A binding analysis using the Fv-antibody library (FIG. 2) and the affinity constants (KD) from SPR analysis (FIG. 12), the amino acid sequence of the CDR3 region was confirmed to have a high affinity for RNase A. In contrast, mutant strains with CDR1 and CDR2 did not exhibit a high affinity for RNase A (FIG. 2). These results indicate that the inhibition of RNase requires specific affinity for the active site of RNase A, along with steric effects.

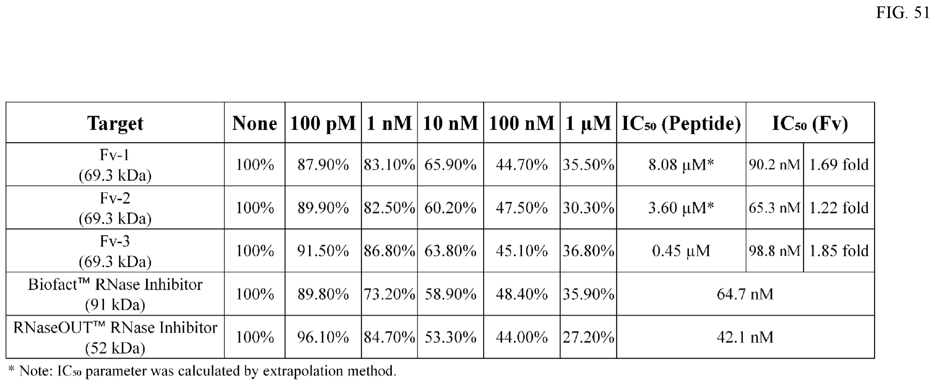

Table 9 is shown in FIG. 51.

### Example 9. Application of synthetic peptides and Fv-antibodies as RNase A inhibitors.

Using RNA samples extracted from HeLa cells, the RNase inhibitory activity of the synthesized peptides was estimated. The RNase inhibitory activity was assessed using agarose gel imaging of the cleaved RNA. To optimize the RNase concentration for the RNase inhibition assay, total RNA was treated with RNase A (600 ng), with RNase A concentrations ranging from 5.7 ng/ml to 3.6 µg/ml, and the cleaved RNA bands were observed after treatment with ETBR (ethidium bromide). As shown in FIG. 22, it was observed that the entire RNA band was cleaved at RNase concentrations of 0.7 µg/ml or higher, confirming that the full RNA bands (1500 bp and 600 bp) were completely cleaved into smaller bands of 1500 bp and 600 bp. The RNase concentration for inhibition analysis was determined to be 0.7 µg/ml, with RNase concentrations above 3.6 µg/ml. The inhibition analysis was performed by mixing total RNA from HeLa cells with RNase (0.7 µg/ml) and Fv-antibody at a concentration of 10 µM, resulting in the observation that the entire RNA was completely cleaved into small RNA bands in the absence of the Fv-antibody. However, as shown in FIG. 23, when Fv antibody (at a concentration of 10 µM) and two types of commercial inhibitors (at a concentration of 10 µM) were mixed with the total RNA, it was observed that the cleavage reaction of RNase A was completely inhibited. The optimal concentration of the Fv-antibody for RNase inhibition was determined by the cleavage reaction with total RNA in the range of 87.5 nM to 5.6 µM for Fv-antibodies (Fv-1, Fv-2, Fv-3). As shown in FIG. 24, the RNase inhibitory activity was estimated to be over 50% at Fv-antibody concentrations of 350 nM or higher. The estimated IC₅₀ values for RNase A activity inhibition, derived from dose-response curve fitting based on agarose gel image analysis, were 234 µM for Fv-1, 366 µM for Fv-2, and 436 µM for Fv-3. As shown in FIG. 25, these values are similar to those of commercial inhibitors (257 nM for Inhibitor-1 and 381 nM for Inhibitor-2).

The same RNase inhibition analysis was performed using three synthetic peptides. As shown in FIG. 26, the optimal concentration of the Fv-antibody for RNase inhibition was determined by the cleavage reaction with total RNA using the three synthetic peptides (Peptide-1, Peptide-2, Peptide-3) in a concentration range of 87.5 nM to 5.6 µM. It was estimated from the agarose gel image that the RNase inhibition activity was over 50% at a concentration of synthetic peptides greater than 1.0 µM. As shown in FIG. 27, the IC₅₀ values for the inhibition of RNase A activity were estimated to be 995 nM for Peptide-1, 658 nM for Peptide-2, and 464 nM for Peptide-3, which showed results relatively similar to commercial inhibitors (257 nM for Inhibitor-1 and 381 nM for Inhibitor-2). These results demonstrated that the Fv-antibody can be effectively used to inhibit RNase activity, and it was estimated that the inhibitory activity of the Fv-antibody is similar to that of commercial RNase A inhibitors.

To investigate the interaction between two synthetic peptides (Peptide-1 and Peptide-2) and RNase A, docking simulations were performed using the AutoDock Vina software from Scripps Research. The interactions were visualized and analyzed using version 0.99rc6 of the Pymol software from DeLano Scientific LLC (South San Francisco, CA, USA) and version v21 of the Discovery Studio Visualizer. The structure of RNase A (PDB ID: 1jvt) was obtained from the PDB (www.rcsb.org), and the interactions of Peptide-1 were analyzed as shown in FIG. 28. The docking energies between three synthetic peptides (Peptide-1, Peptide-2, and Peptide-3) and RNase A were estimated using the AutoDock Vina docking program. The docking energy was estimated by the change in Gibbs free energy (ΔG), which is one of the thermodynamic parameters. The docking results showed that the inhibitor binds to the active site of RNase A, blocking interactions with the RNA substrate. The amino acids of RNase A that interact hydrophobically with Peptide-1 were Lys³⁷ and Val⁴³, while Arg¹⁰, Lys³⁷, Asp³⁸, Arg³⁹, Val⁴³, Asp⁸³, His¹¹⁹, and Ser¹²³ of RNase A formed hydrogen bonds with Peptide-1. Additionally, Lys⁷, Lys⁶⁶, and Lys¹⁰⁴ of RNase A formed electrostatic interactions with Peptide-1. The change in Gibbs free energy (ΔG) between Peptide-1 and RNase A was estimated to be -7.1 kcal/mol. When Peptide-2 interacted with RNase A, the docking results showed that Peptide-2 also binds to the active site of RNase A (FIG. 29). The amino acids of RNase A that interact hydrophobically with Peptide-2 were Lys⁷, Arg³⁹, Lys⁴¹, Lys⁶⁶, and His¹¹⁹, and Lys⁷, Arg¹⁰, Gln¹¹, Arg³⁹, Lys⁶⁶, Asn⁶⁷, Asn⁷¹, His¹¹⁹, and Phe¹²⁰ of RNase A formed hydrogen bonds with Peptide-2. Furthermore, Asp³⁸ and His¹¹⁹ of RNase A formed electrostatic interactions with Peptide-2. The binding energy (ΔG) between Peptide-2 and RNase A was estimated to be -6.9 kcal/mol. In the case of Peptide-3, the docking results indicated that Peptide-3 also binds to the active site of RNase A, as shown in FIG. 30. The amino acids of RNase A that interact hydrophobically with Peptide-3 were His¹², Lys⁶⁶, and His¹¹⁹ , and Arg¹⁰, Lys³⁷, Asp³⁸, Lys⁴¹, Thr⁴⁵, and Lys⁶⁶ of RNase A formed hydrogen bonds with Peptide-3. Additionally, Asp³⁸ and Lys⁴¹ of RNase A formed electrostatic interactions with Peptide-3. The docking energy (ΔG) between Peptide-3 and RNase A was estimated to be -8.5 kcal/mol. The interactions between RNase A and the three synthetic peptides (Peptide-1, Peptide-2, and Peptide-3) are summarized in Table 10.

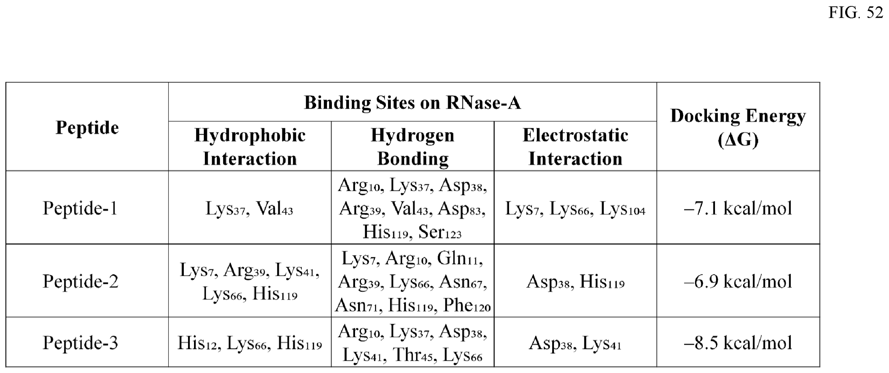

Table 10 is shown in FIG. 52.

The target Fv variant with specific affinity for RNase A was screened from an Fv library that is surface-expressed on the outer membrane of *E. coli.* The target clones were isolated from the Fv library using external magnets, and the magnetic beads were cultured on an agar plate. Finally, two variants were determined to be target variants with CDR3 sequences different from the template sequence. Based on fitting analysis using an adsorption model, the binding affinities (KD) of the Fv antibodies for the three screened clones were 23.8 ± 2.9 (n=3) µM, 29.4 ± 4.7 (n=3), and 28.7 ± 3.1 (n=3) µM. The CDR3 regions of these three screened Fv antibodies were synthesized as peptides, and the amino acid sequences were determined from the nucleotide sequences of the CDR3 regions. The analysis of RNase A inhibitors was performed using a modified Taq-man probe assay. Compared to the non-inhibitory condition (100%) without inhibitors, RNase A activity was reduced by 54.4% in peptide-1 (53.2% in peptide-2 and 50.3% in peptide-3) (the concentrations of peptide-1, peptide-2, and peptide-3 were 10 µM). These results demonstrated that the three synthetic peptides can specifically bind to the active site of RNase A. In competitive binding analysis using ssDNA (51 bp) as a model RNA, the synthetic peptides (peptide-1, peptide-2, and peptide-3) exhibited binding affinities for RNase A, and the binding affinities were significantly higher than that of the substrate RNA for RNase A. The RNase inhibitory activity of the synthetic peptides was estimated using RNA samples extracted from HeLa cells, and peptide-1 was estimated to be more favorable as an RNase A inhibitor for the total RNA of HeLa cells compared to peptide-2. The RNase inhibitory activity of the synthetic peptides was also estimated using RNA samples extracted from *E. coli* after the transformation of SARS-CoV-2 NP (nucleocapsid protein), and peptide-2 was estimated to be more favorable as an RNase A inhibitor for the total RNA of *E. coli* compared to peptide-1.

On the other hand, for actual samples of serum, pig swab, and saliva swab, 50 µL of each was diluted 8-fold with 350 µL of a reagent containing 500 mM Tris-acetate buffer, 5 mM EDTA, 10 mM 2',3'-cCMP, and 1 µM inhibitor. The absorbance over time at 288 nm corresponding to the 3'-CMP converted by 2',3'-cCMP and RNase A in a two-step catalytic reaction (hydrolysis reaction) was measured.

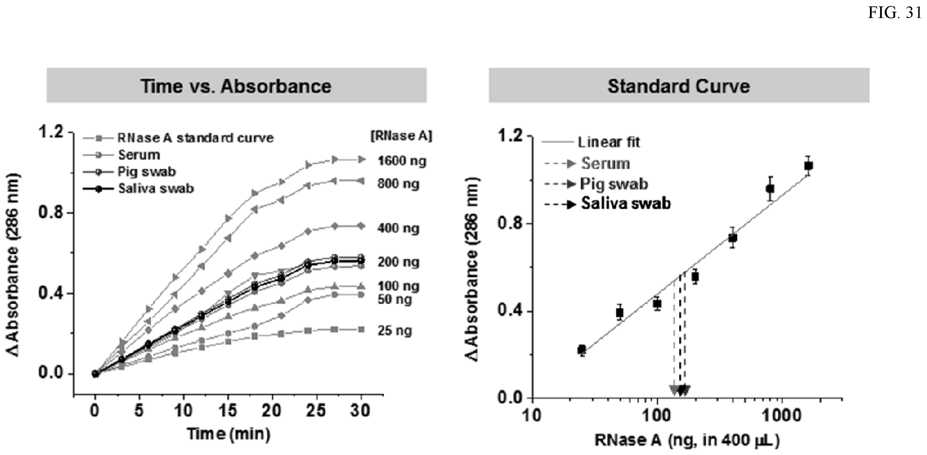

FIG. 31 shows the cCMP analysis results for actual samples of serum, pig swab, and saliva swab.

As shown in FIG. 31, the amounts of RNase A contained in the actual serum sample, pig swab actual sample, and saliva swab actual sample, compared to the standard curve created using commercially available RNase A samples, were 131 ng, 166 ng, and 151 ng, respectively.

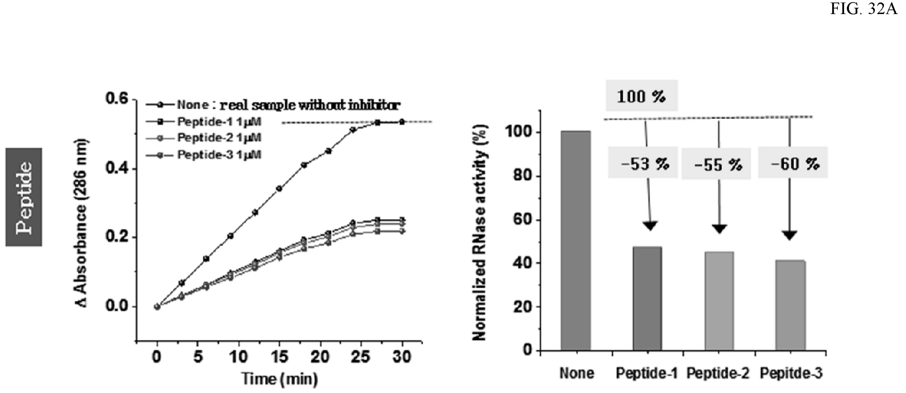

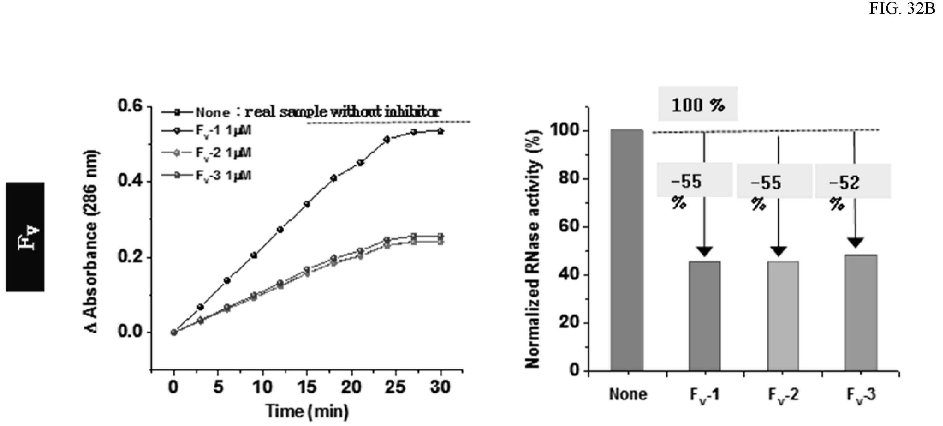

FIGs. 32A and 32B show the cCMP analysis results for serum actual samples treated with peptide-1 to peptide-3 and Fv-1 to Fv-3.

As shown in FIGs. 32A and 32B, in the case of serum actual samples using the peptides-1, peptides-2, and peptides-3 of the present invention as the inhibitor, it was found that after approximately 27 minutes, the RNase A activity decreased by about 53%, 55%, and 60%, respectively, compared to the samples without the inhibitor. Additionally, in the case of serum actual samples using Fv-1, Fv-2, and Fv-3 of the present invention as the inhibitor, it was found that after approximately 27 minutes, the RNase A activity decreased by about 55%, 55%, and 52%, respectively, compared to the samples without the inhibitor.

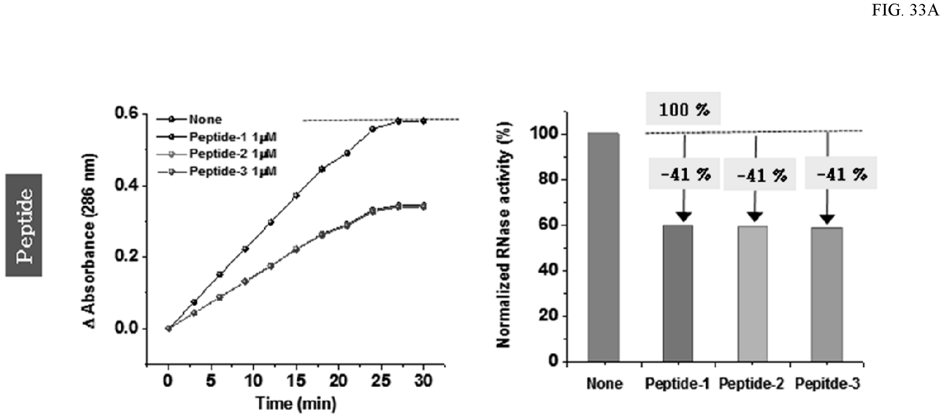

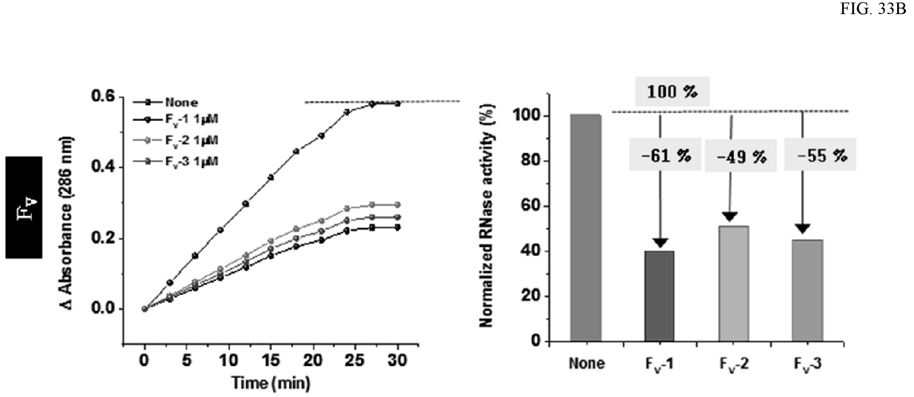

FIGs. 33A and 33B show the cCMP analysis results for the Pig swap actual samples treated with Peptide-1 to Peptide-3 and Fv-1 to Fv-3.

As shown in FIGs. 33A and 33B, in the case of Pig swap actual samples using the peptides-1, peptides-2, and peptides-3 of the present invention as the inhibitors, it was found that after approximately 27 minutes, the RNase A activity decreased by about 41%, 41%, and 41% compared to the samples without the inhibitor. Additionally, in the case of Pig swap actual samples using Fv-1, Fv-2, and Fv-3 of the present invention as the inhibitors, it was found that after approximately 27 minutes, the RNase A activity decreased by about 61%, 49%, and 55% compared to the samples without the inhibitor.

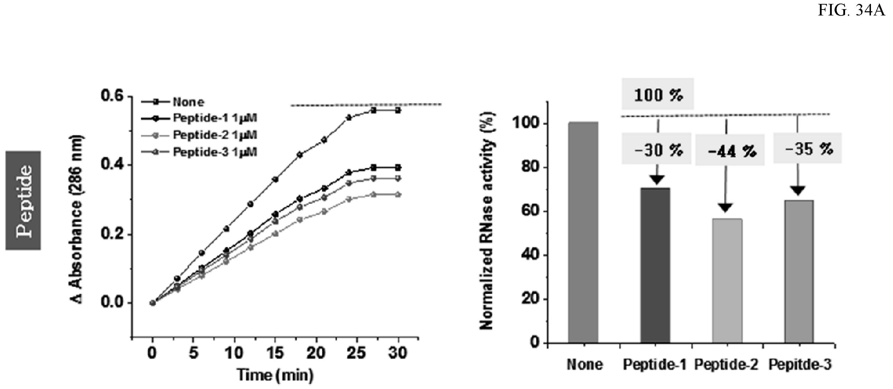

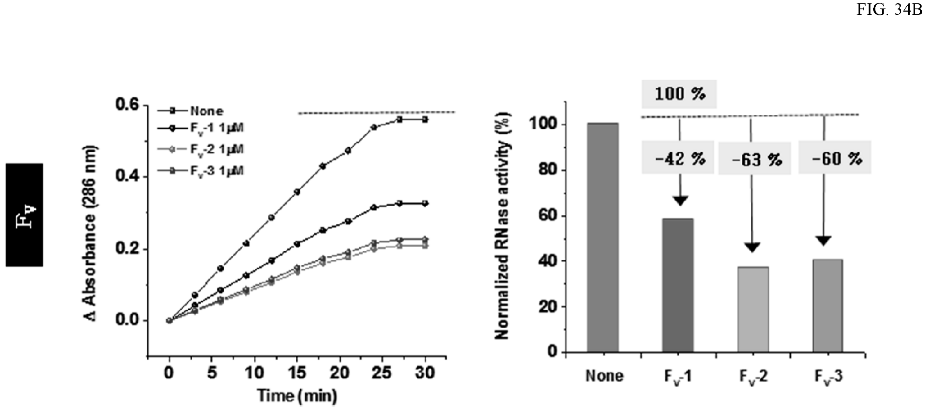

FIGs. 34A and 34B show the cCMP analysis results for actual saliva swap samples treated with peptides 1 to 3 and Fv-1 to Fv-3.

As shown in FIGs. 34A and 34B, in the case of Saliva swap actual samples using the peptides-1, peptides-2, and peptides-3 of the present invention as the inhibitors, it was found that after approximately 27 minutes, the RNase A activity decreased by about 30%, 44%, and 35%, respectively, compared to the samples without the inhibitor. Additionally, in the case of Saliva swap actual samples using Fv-1, Fv-2, and Fv-3 of the present invention as the inhibitors, it was found that after approximately 27 minutes, the RNase A activity decreased by about 42%, 63%, and 60%, respectively, compared to the samples without the inhibitor.

As such, the peptide or antibody as an RNase A inhibitor according to the present invention can be used in various analytical methods related to RNA. For example, methods for RNA extraction, RNA purification, PCR (polymerase chain reaction), cDNA (complementary DNA) synthesis, and DNA expression methods are included, but are not limited to these.

### < RNase A capturing method >

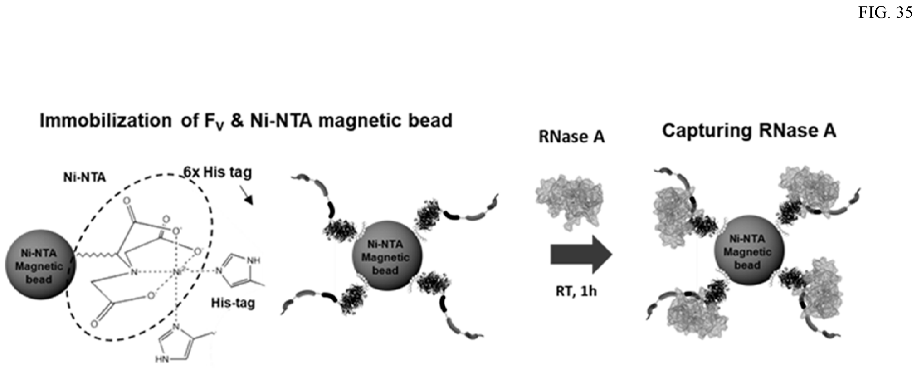

FIG. 35 is a conceptual diagram for explaining the RNase A capturing method according to an embodiment of the present invention.

Referring to FIG. 35, the RNase A capturing method according to an embodiment of the present invention may include the step of mixing beads fixed on the surface with an antibody or peptide that specifically binds to RNase A with a sample containing RNase A to induce a reaction between the RNase A and the antibody or peptide; and the step of collecting the beads from the sample.

In one embodiment, the type of sample containing RNase A is not specifically limited. For example, the sample containing RNase A may include a solution that contains RNA or is used for processing RNA.

In one embodiment, the antibody or peptide that specifically binds to the RNase A may comprise one or more of peptide-1, peptide-2, peptide-3, Fv-1, Fv-2, and Fv-3 according to the invention described above.

In one embodiment, the beads are not specifically limited as long as the antibody or peptide can bind to them. For example, the beads may include inorganic beads such as ceramics or metals, and organic beads such as polymers, without limitation.

In one embodiment, the beads may include magnetic particles containing nickel (Ni), copper (Cu), zinc (Zn), or cobalt (Co) with chelating groups such as NTA (nitrilotriacetic acid) or IDA (iminodiacetic acid) modified on their surface. In this case, the antibody or peptide can be tagged with histidine and then bound to the beads.

In one embodiment, the method of collecting the beads from the sample is not particularly limited. For example, the beads can be collected from the sample using methods such as filtering or centrifugation. Alternatively, if the beads are formed of magnetic material, the beads can be collected from the sample using a magnetic field.

According to the RNase A capturing method of the present invention, RNase A contained in a sample can be simply and easily captured or removed from the sample through an antibody or peptide that is fixed to a bead and specifically binds to RNase A.

### Example 1

(i) A solution containing untreated RNase A (10 ng) ('Before') and (ii) four different concentrations of Fv-bound bead solutions diluted to 1/4 each, were reacted with the RNase A (10 ng) solution at room temperature for 1 hour, after which the beads were removed to obtain the solutions ('After'). Each of these solutions was then mixed with a solution containing 1000 ng of RNA at room temperature for 10 minutes to prepare the samples, and electrophoresis analysis (90V, 40 min) was performed on each of them.

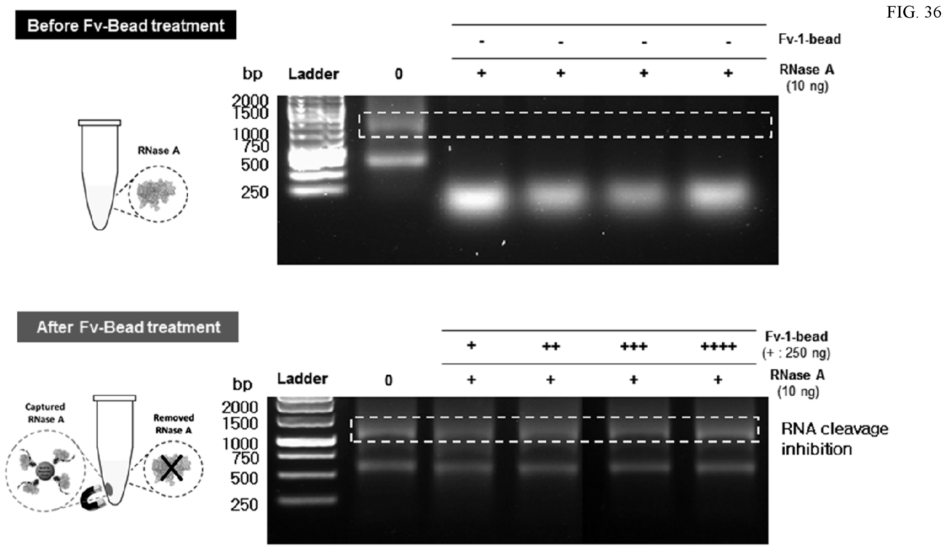

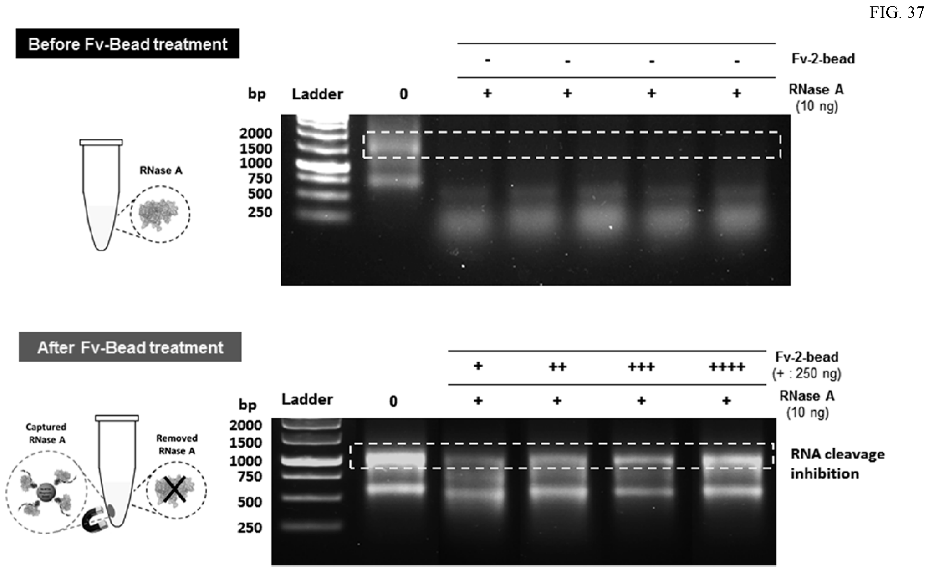

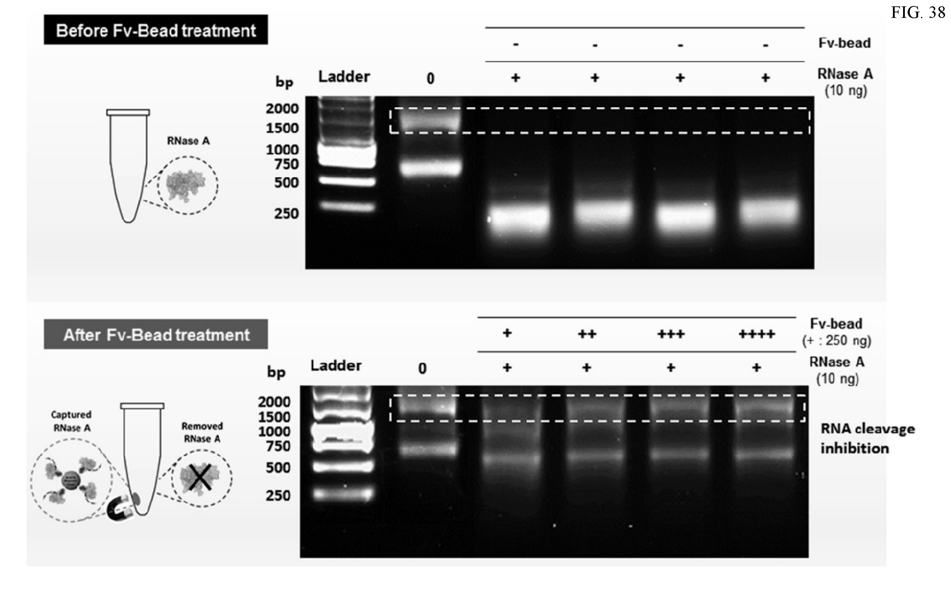

FIGs. 36 to 38 show the results of electrophoresis analysis according to Embodiment 1.

As shown in FIGs. 36, 37, and 38, samples mixed with an RNA-containing solution after treatment with a solution containing RNase A (10 ng) using one of Fv-1, Fv-2, and Fv-3 showed that RNA was maintained without degradation. In contrast, samples mixed with a solution containing RNase A (10 ng) that was not treated with Fv-1, Fv-2, and Fv-3 showed that RNA was degraded and not maintained. This indicates that Fv-1, Fv-2, and Fv-3 can effectively capture RNase A contained in the sample through immobilized beads.

### Example 2

(i) A serum solution containing RNase A (10 ng) that was not treated with Fv-3 and (ii) solutions of Fv-3 fixed beads at four different concentrations, each diluted 1/4, were reacted with the serum solution containing RNase A (10 ng) at room temperature for 1 hour. After removing the beads, solutions were prepared for each, which were then mixed with a solution containing 600 ng of RNA at room temperature for 10 minutes to produce samples. Electrophoresis analysis (90V, 40 min) was performed for each of these samples.

### Example 3

(i) A solution containing RNase A (10 ng) in a pig swab that was not treated with Fv-3, and (ii) solutions of Fv-3 fixed beads at four different concentrations, each diluted 1/4, were reacted with the RNase A (10 ng) containing pig swab solution at room temperature for 1 hour. After removing the beads, these solutions were prepared and mixed with a solution containing 600 ng of RNA at room temperature for 10 minutes to produce samples. Electrophoresis analysis (90V, 40 min) was then performed on each of these samples.

### Example 4

(i) A saliva swab solution containing RNase A (10 ng) that was not treated with Fv-3, and (ii) solutions of Fv-3 fixed beads at four different concentrations, each diluted to 1/4, were reacted with the saliva swab solution containing RNase A (10 ng) at room temperature for 1 hour. After removing the beads, solutions were prepared, and each of these was mixed with a solution containing 600 ng of RNA and incubated at room temperature for 10 minutes to produce samples. Electrophoresis analysis (90V, 40 min) was then performed on each of these samples.

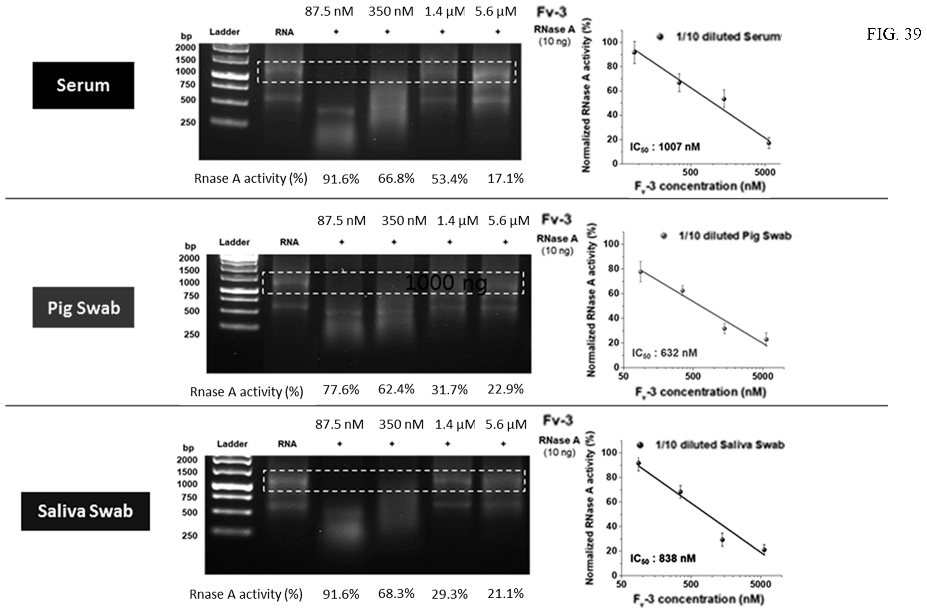

FIG. 39 shows the results of electrophoresis analysis according to Examples 2 to 4.

As shown in FIG. 39, it was found that as the concentration of the Fv-3 fixed beads-containing solution increased, the RNase A activity in the samples treated with serum, pig swab, and saliva swab solutions decreased. In particular, it was confirmed that when the concentration of Fv-3 was 1.4 µM or higher, the inhibitory effect on RNase A activity exceeded 50%.

### < RNase detection immunosensor or method >

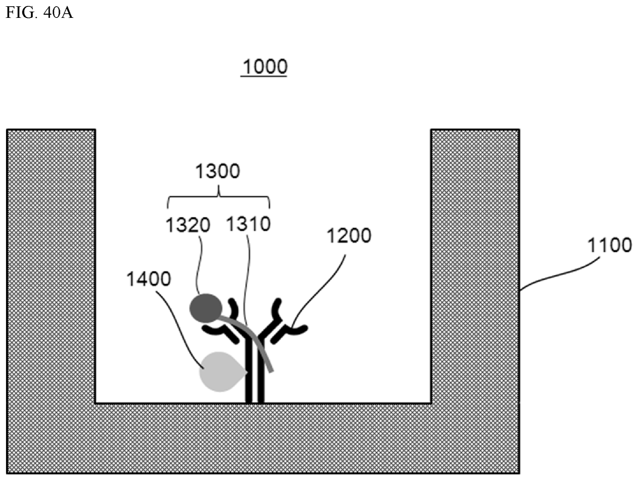

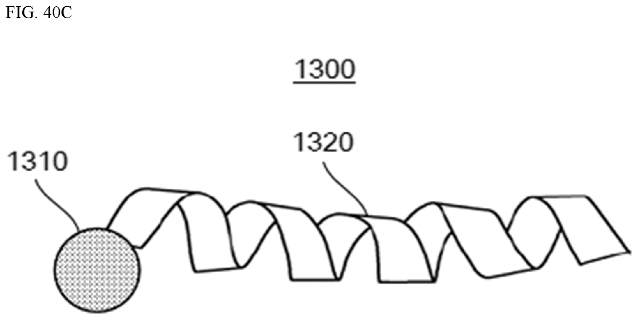

FIGs. 40A illustrate the RNase detection immunosensor according to one embodiment of the present invention and the RNase detection mechanism thereof, and FIG. 40C is a diagram for explaining the switching peptide depicted in FIG. 40A.

Referring to FIGs. 40A to 40C, the RNase detection immunoassay device (1000) according to an embodiment of the present invention includes a substrate (1100) having a reaction space that can accommodate a detection sample solution to be analyzed, a binding antibody (1200) that is located within the reaction space, fixed to the substrate (1100), and specifically binds to the RNase, a switching peptide (1300) that is reversibly bound to the Fab region of the binding antibody (1200), and a quenching material (1400) that is arranged adjacent to a fluorescent label (1320) of the switching peptide (1300) and quenches the fluorescence from the fluorescent label (1320).

The substrate (1100) is not particularly limited in its structure or shape as long as it has a reaction space that can accommodate the detection sample solution. However, the substrate (1100) can be formed of a transparent material that can transmit light, such as a transparent polymer material, glass material, or metal oxide material.

The binding antibody (1200) is substantially identical to the antibody that specifically binds to the RNase according to the present invention described above, and therefore, a redundant detailed description thereof is omitted. The binding antibody (1200) can be fixed to the substrate (1100) by directly binding to the surface of the substrate (1100) or by binding to the surface of the substrate (1100) through a separate linker compound.

On the other hand, the variable regions of the heavy chain and light chain of the binding antibody (1200) may each have a structure in which three complementarity-determining regions (CDRs), which are hypervariable regions (HVR), and four framework regions (FRs) that structurally support the complementarity-determining regions are arranged alternately. The complementarity-determining regions have different amino acid sequences for each antibody, allowing them to specifically bind to each antigen, while the framework regions have conserved amino acid sequences according to the subfamilies of organisms, meaning that antibodies from organisms belonging to the same subfamily have identical or similar amino acid sequences. For the convenience of explanation, in the variable region of the light chain of the binding antibody (1200), the four framework regions that are sequentially spaced from the N-terminus are referred to as the first to fourth light chain framework regions (VL-FR1, VL-FR2, VL-FR3, and VL-FR4), and the three complementarity-determining regions that are located between the first to fourth light chain framework regions (VL-FR1, VL-FR2, VL-FR3, and VL-FR4) and are sequentially arranged from the N-terminus are referred to as the first to third light chain complementarity-determining regions (VL-CDR1, VL-CDR2, and VL-CDR3). Additionally, in the variable region of the heavy chain of the binding antibody (1200), the four framework regions that are sequentially spaced from the N-terminus are referred to as the first to fourth heavy chain framework regions (VH-FR1, VH-FR2, VH-FR3, and VH-FR4), and the three complementarity-determining regions that are located between the first to fourth heavy chain framework regions (VH-FR1, VH-FR2, VH-FR3, and VH-FR4) and are sequentially arranged from the N-terminus are referred to as the first to third heavy chain complementarity-determining regions (VH-CDR1, VH-CDR2, and VH-CDR3).

The switching peptide (1300) may include a peptide compound (1310) and a fluorescent label (1320). The switching peptide (1300) can reversibly bind to the binding antibody (1200). For example, when the RNase binds to the binding antibody (1200), the switching peptide (1300) may be quantitatively released from the binding antibody (1200), resulting in a change in the electrochemical properties of the test sample containing the target antigen, mediated by the fluorescent label (1320) of the switching peptide (1300) released from the binding antibody (1200) through a redox reaction.

The peptide compound (1310) may have an amino acid sequence that can selectively and reversibly bind to the Fab (Fragment antigen-binding) region of the binding antibody (120).

In one embodiment, the peptide compound (1310) may have an amino acid sequence that can specifically and reversibly bind to one or more of the four framework regions (FR) of the light chain or heavy chain of the binding antibody (1200). For example, the peptide compound (1310) may include an amino acid sequence that has 90% or more, for instance, 95% or more homology with one or more of the framework regions of the light chain or heavy chain of the binding antibody (1200). In this case, the peptide compound (1310) can reversibly bind to one or more of the first to fourth framework regions (FR1, FR2, FR3, and FR4) of the light chain or heavy chain. Accordingly, when RNase A binds to the binding antibody (1200), the peptide compound (1310) bound to the binding antibody (1200) can be quantitatively released from the binding antibody (1200) depending on the amount of RNase A bound to the binding antibody (1200).

In one embodiment, the peptide compound (110) may comprise a first peptide compound (L1) having a first amino acid sequence that has homology with the amino acid sequence of the second light chain framework region (VL-FR2) of the binding antibody (1200), a second peptide compound (L2) having a second amino acid sequence that has homology with the amino acid sequence of the third or fourth light chain framework region (VL-FR3, VL-FR4), a third peptide compound (H1) having a third amino acid sequence that has homology with the amino acid sequence of the second heavy chain framework region (VH-FR2), and a fourth peptide compound (H2) having a fourth amino acid sequence that has homology with the amino acid sequence of the third or fourth heavy chain framework region (VH-FR3, VH-FR4), wherein one or more of these compounds may be selected.

The binding antibody (1200) has its second light chain framework region (VL-FR2) and the third or fourth heavy chain framework region (VH-FR3, VH-FR4) positioned adjacent to each other to interact, and the third or fourth light chain framework region (VL-FR3, VL-FR4) and the second heavy chain framework region (VH-FR2) are also positioned adjacent to each other to interact. Therefore, the first peptide compound (L1) having a first amino acid sequence that is homologous to the second light chain framework region (VL-FR2) of the binding antibody (1200) can selectively interact with the third or fourth heavy chain framework region (VH-FR3, VH-FR4) of the binding antibody (1200). The second peptide compound (L2) having a second amino acid sequence that has homology with the third or fourth light chain framework region (VL-FR3, VL-FR4) of the binding antibody (1200) can selectively interact with the second heavy chain framework region (VH-FR2) of the binding antibody (1200). The third peptide compound (H1) having a third amino acid sequence that has homology with the second heavy chain framework region (VH-FR2) of the binding antibody (1200) can selectively interact with the third or fourth light chain framework region (VL-FR3, VL-FR4) of the binding antibody (1200). The fourth peptide compound (H2) having a fourth amino acid sequence that has homology with the third or fourth heavy chain framework region (VH-FR3, VH-FR4) of the binding antibody (1200) can selectively interact with the second light chain framework region (VL-FR2) of the binding antibody (1200).

In one embodiment, when the binding antibody (1200) is derived from an organism belonging to the subfamilies that include humans, the peptide compound (1310) may comprise approximately 10 to 25 amino acids and have a molecular weight of about 1600 to 3000 Da. For example, the peptide compound (1310) may include approximately 14 to 20 amino acids and have a molecular weight of about 1650 to 2500 Da. In one embodiment, the first to fourth peptide compounds (L1, L2, H1, H2) may include the amino acid sequences as described in Table 11 below.

**Table 11**

| Switching peptide | Amino acid sequence | Molecular weight | Gibbs free energy (Kcal/mol) |
|---|---|---|---|
| H1 | SYWIH WVKQR PGQGL EWIGE | 2469.7 | -17.91 |
| H2 | VYYCA REPTG TGIYF DVWGK | 2325.6 | -17.19 |
| L1 | TYLEW YPQKP GQSPK LLIYK | 2452.8 | -15.37 |
| L2 | VYYCF QGSHV PFTK | 1675.9 | -13.05 |

On the other hand, since the framework regions of the light chain and heavy chain of antibodies generally possess conserved amino acid sequences according to the subfamilies of organisms, the peptide compound (1310) can be commonly applied to antibodies derived from other species belonging to the same subfamilies. Therefore, even if the peptide compound (1310) is synthesized from antibodies of species such as chickens, mice, or rabbits, it can exhibit the same actions and effects as described earlier for human antibodies.

The fluorescent label (1320) can bind to the peptide compound (1310) and can emit fluorescence. If it can bind to the peptide compound (1310) and emit fluorescence, the fluorescent label (1320) is not specifically limited. For example, the fluorescent label (1320) may include one or more selected from the group consisting of rhodamine and its derivatives, fluorescein and its derivatives, coumarin and its derivatives, acridine and its derivatives, pyrene and its derivatives, erythrosin and its derivatives, eosin and its derivatives, 4-acetamido-4'-isothiocyanatostilbene-2,2'-disulfonic acid, fluorescein isothiocyanate (FITC), Oregon green, Alexafluor, carboxyfluorescein (FAM), 6-carboxy-4',5'-dichloro-2',7'-dimethoxyfluorescein (JOE), carboxy-X-rhodamine (ROX), 6-carboxy-2',4,4',5',7,7'-hexachlorofluorescein (HEX), Texas Red (sulforhodamine 101 acid chloride), 6-carboxy-2',4,7',7-tetrachlorofluorescein (TET), tetramethylrhodamine isothiocyanate (TRITC), carboxytetramethylrhodamine (TAMRA), cyanine series dyes, and cyadicarbocyanine dyes.

The quenching material (1400) is arranged adjacent to the fluorescent label (1320) when the switching peptide (1300) is bound to the binding antibody (1200), allowing it to absorb fluorescent energy from the fluorescent label (1320) and quench the fluorescence from the fluorescent label (1320). If the fluorescence from the fluorescent label (1320) can be quenched, the material of the quenching material (1400) is not specifically limited.

As an embodiment, the quenching material (1400) may comprise one or more selected from the group consisting of 4-(dimethylamino)azobenzene-4-carboxylic acid (DABCYL), 4-(dimethylamino)azobenzenesulfonic acid (DABSYL), blackhole quencher (BHQ), blackberry quencher (BBQ), ECLIPSE quencher, and Tide quencher.

In a different embodiment, the quenching material (1400) may include carbon nanomaterials or manganese dioxide nanomaterials. The carbon nanomaterials may comprise one or more selected from graphene and its derivatives, graphene oxide (NGO) and its derivatives, reduced graphene oxide and its derivatives, graphene oxide nanocolloids (GON), and the like. The nanomaterials may be in sheet form or particle form. The sheet may be composed of a single layer or multiple layers, and the shape of the sheet may exist in various forms, including flat or curved surfaces. Additionally, the shape of the particles may include various forms such as spherical, oval, rod-like, and polygonal shapes.

The quenching material (1400) can absorb the light or fluorescent energy emitted from the fluorescent label (1320) due to its interaction with the fluorescent label (1320), thereby quenching the fluorescence from the fluorescent label (1320).

In one embodiment, the quenching material (1400) can be arranged adjacent to the fluorescent label (1320) by being bound to the binding antibody (1200). For example, the quenching material (1400) can bind to the Fc region of the binding antibody (1200). In another embodiment, the quenching material (1400) can be arranged adjacent to the fluorescent label (1320) by being bound to the substrate (1100).

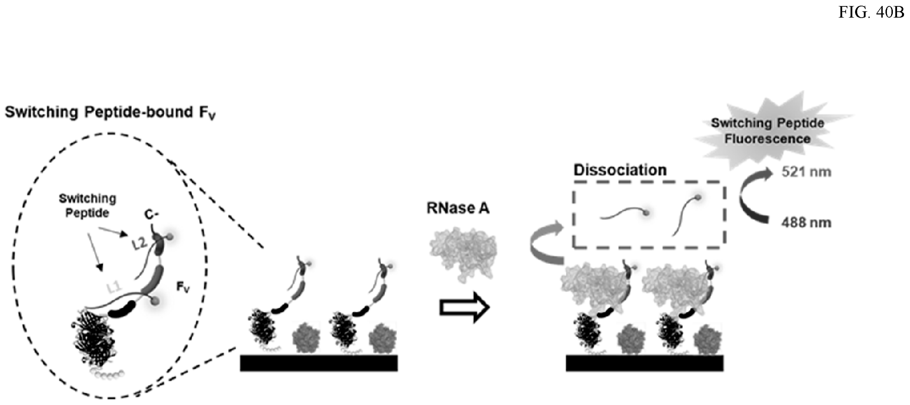

According to the immunoassay device of the present invention, as shown in FIG. 40B, when a detection sample solution containing RNase A that specifically reacts with the binding antibody (1200) is injected into the reaction space of the substrate (1100), the RNase A will react with the binding antibody (1200). Consequently, the amount of RNase A that reacts with the binding antibody (1200) allows the switching peptide (1300) to be quantitatively released from the binding antibody (1200). Furthermore, the fluorescent label (13200) of the switching peptide (1300) released from the binding antibody (1200) moves away from the quenching material (1400), and the fluorescence generated from this is not quenched and can be emitted externally. In this invention, by quantitatively analyzing the switching peptide (1300) released from the binding antibody (1200) through this fluorescence, quantitative analysis of the target antigen contained in the detection sample solution can be performed. In this case, since a washing process to remove unreacted RNase A is not required, quantitative analysis of RNase A can be performed in a one-step manner after reacting the detection sample solution with the binding antibody.

On the other hand, although not illustrated in the drawings, in order to generate fluorescence from the fluorescent label (1320) and to quantitatively analyze the switching peptide (1300) released from the binding antibody (1200) through this fluorescence, the immunoassay device (1000) may further include a light source and an image analysis device.

The light source can irradiate the analytical sample solution accommodated in the reaction space of the substrate (1100), and the fluorescent label (1320) of the switching peptide (1300) can absorb the light energy irradiated from the light source to generate fluorescence. In one embodiment, the light source can generate short-wavelength light in the visible or ultraviolet region to irradiate the analytical sample solution accommodated in the reaction space of the substrate (1100).

The image analysis device can accommodate the fluorescence generated from the switching peptide (1300) released from the binding antibody (1200) and can generate an image thereof. By analyzing the image, it is possible to quantify the amount of the switching peptide (1300) released from the binding antibody (1200). For example, the image analysis device may include an image generation unit that images the fluorescent signal, an image processing unit that processes the image generated by the image generation unit, and an image analysis unit that analyzes the image processed by the image processing unit.

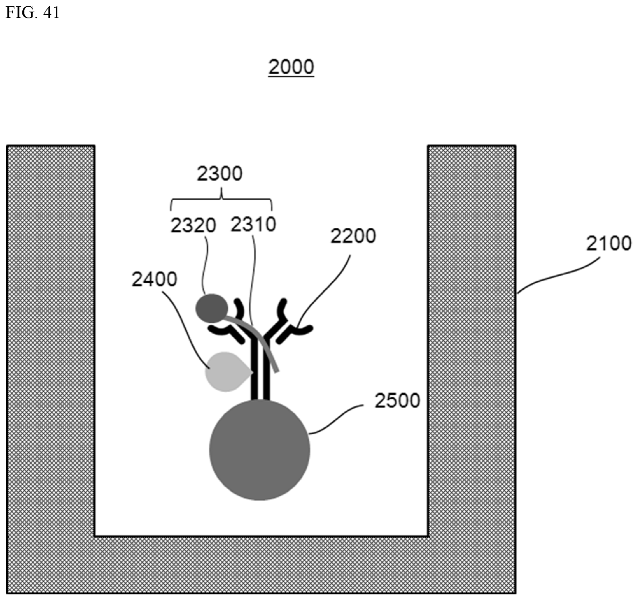

FIG. 41 is a diagram illustrating an RNase detection immunosensor according to another embodiment of the present invention.

Referring to FIG. 41, the immunoanalysis device (2000) according to another embodiment of the present invention includes a substrate (2100), a binding antibody (2200), a switching peptide (2300), a quenching material (2400), and a support (2500).

The RNase detection immunoassay device (2000) of this embodiment is substantially identical or similar to the RNase detection immunoassay device (1000) described with reference to FIG. 40A, except that the binding antibody (2200) is fixed to the support (2500) rather than the substrate (2100). Therefore, the following detailed description will omit redundant information.

The support (2500) may have a particle form made of inorganic or organic material, and the binding antibody (2200) can be bound to the support (2500). Additionally, the quenching material (2400) can be bound to the binding antibody (2200) or the support (2500), and may be arranged adjacent to the fluorescent label (2320) of the switching peptide (2300) bound to the binding antibody (2200).

On the other hand, in FIG. 41, the support (2500) and the quenching material (2400) are shown as separate independent components; however, in one embodiment of the present invention, the quenching material is applied to the support (2500), and the support (2500) and the quenching material (2400) may be the same component. In this case, a carbon material such as graphene may be used as the support (2500).

According to the RNase detection immunoassay device of this embodiment, the binding antibody (2200) is bound to the support (2500) rather than the substrate (2100), allowing it to move within the solution. Therefore, in addition to the advantages of the RNase detection immunoassay device (1000) illustrated in FIG. 40A, it can further enhance the reaction rate between the binding antibody (2300) and RNase A in the detection sample solution.

Hereinafter, the immunoassay method according to an embodiment of the present invention using the RNase detection immunoassay device shown in FIG. 40A or the RNase detection immunoassay device shown in FIG. 41 will be described in detail.

Referring to FIGs. 40A to 40C, the immunoassay method according to an embodiment of the present invention includes the following steps: a first step of fixing a binding antibody (1200) to a substrate (1100) with a switching peptide (1300) bound to the binding antibody (1200), or fixing the binding antibody (1200) to the substrate (1100) and then binding the switching peptide (1300) to the binding antibody, and binding a quenching material (1400) to the binding antibody (1200) or the substrate (1100); a second step of treating the binding antibody (1200) with a detection sample solution; and a third step of irradiating the treated detection sample solution with light and quantitatively analyzing RNase A in the detection sample solution through the fluorescence generated from the fluorescent label (1320) of the switching peptide (1300) released from the binding antibody (1200).

In the first step, the switching peptide (1300) may include a peptide compound (1310) having an amino acid sequence that can selectively and reversibly bind to the Fab (Fragment antigen-binding) region of the binding antibody (1200), and a fluorescent label (1320) that is bound to it and can emit fluorescence in the detection sample solution. In one embodiment, the switching peptide (1300) and the binding antibody (1200) are those described with reference to FIGS. 40A to 40C, and therefore, a redundant detailed description of these is omitted.

In one embodiment, for the binding antibody (1200) and the switching peptide (1300), the binding antibody (1200) is first immobilized on the substrate (1100), after which the switching peptide (1300) may bind to the immobilized binding antibody (1200). Alternatively, the switching peptide (1300) may be bound to the binding antibody (1200) first, and then the binding antibody (1200) may be immobilized on the substrate (1100).

The method of fixing the binding antibody (1200) to the substrate (1100) is not specifically limited. For example, the binding antibody (1200) may bind directly to the substrate (1100), or it may be fixed to the substrate (1100) via a linker compound.

The switching peptide (1300) can selectively and reversibly bind to the Fab (Fragment antigen-binding) region of the binding antibody (1200), as described above.

The quenching material (1400) can be coupled to the surface within the reaction space of the Fc region of the binding antibody (1200) or the substrate (1100). At this time, the quenching material (1400) can be coupled to the binding antibody (1200) or the substrate (1100) so as to be positioned adjacent to the fluorescent label (1320) of the switching peptide (1300) bound to the binding antibody (1200).

In the second step, the detection sample solution can be injected into the reaction space of the substrate (1100) to react the binding antibody (1200) with the RNase A in the detection sample solution.

In one embodiment, when RNase A, which specifically reacts with the binding antibody (1200), is present in the detection sample solution, injecting the detection sample solution into the reaction space allows the RNase A to bind to the binding antibody (1200). In this case, the switching peptide (1300) can be quantitatively released from the binding antibody (1200) depending on the amount of RNase A that has reacted with the binding antibody (1200).

In the third step, the detection sample solution can be injected into the reaction space of the substrate (1100), and after a certain period of time, light can be irradiated onto the detection sample solution. In this case, the fluorescence generated from the fluorescent label (1320) of the switching peptide (3100) bound to the binding antibody (1200) is quenched by the quenching material (1400) arranged adjacent to it and is not emitted externally. In contrast, the fluorescence generated from the fluorescent label (1320) of the switching peptide (1300) released from the binding antibody (1200) is spatially separated from the quenching material (1400) and can be emitted externally without being quenched.

Then, using the image analysis device, an image of the fluorescence generated from the fluorescent label (1320) of the switching peptide (1300) released from the binding antibody (1200) is created, and by analyzing the image, quantitative analysis of the switching peptide (1300) released from the binding antibody (1200) is performed. As a result, the amount of RNase A included in the detection sample solution can be quantitatively calculated. The quantitative analysis of the amount of RNase A, as described above, is possible because the switching peptide (1300) bound to the binding antibody (1200) is quantitatively released from the binding antibody (1200) depending on the amount of RNase A that reacts with the binding antibody (1200).

On the other hand, referring to FIG. 41, the quantitative analysis method according to another embodiment of the present invention includes a first step of fixing a binding antibody (2200) to a support (2500) or fixing the binding antibody (2200) to the support (2500) and then binding a switching peptide (2300) to the binding antibody (2200), and coupling a quenching material (2400) to the binding antibody (2200) or the support (2500); a second step of injecting the support (2500) with the bound binding antibody (2200) and a detection sample solution into the reaction space of a substrate (2100); and a third step of quantitatively analyzing RNase A in the detection sample solution through fluorescence generated from a fluorescent label (2320) of the switching peptide (2300) released from the binding antibody (2200) after irradiating the detection sample solution with light following the treatment.

In the first step, the binding antibody (2200) can be bound to the support (2500) rather than the substrate (2100), and the binding antibody (2200) bound to the support (2500) in the second step can flow within the detection sample solution. Meanwhile, since the remaining steps are substantially the same as the immunoassay method using the RNase detection immunoassay device illustrated in FIG. 40A, a detailed redundant description of these steps is omitted.

According to the RNase detection immunoassay device and immunoassay method of the present invention, the bound switching peptide of the binding antibody can be quantitatively released from the binding antibody depending on the amount of RNase A that reacted with the binding antibody. The fluorescent label of the switching peptide released from the binding antibody generates fluorescence externally, allowing for quantitative analysis of RNase A in the detection sample to be performed in a one-step manner without the need for washing processes, such as removing unreacted target substances, which are generally required in conventional immunoassay methods.

### Example 1

A solution containing Fv-1 at a concentration of 50 µg/ml was used to coat MaxiSorp plates with high protein binding capacity, followed by washing with PBS solution. Subsequently, a solution containing BSA at a concentration of 5 mg/ml was applied and then washed with PBS solution. Next, a 100 nM solution of the switching peptide was applied and maintained at room temperature for 1 hour to bind the switching peptide to Fv-1, followed by washing with PBS solution. Then, sample solutions containing RNase A at concentrations of 0, 1 pg/ml, 10 pg/ml, 100 pg/ml, 1 ng/ml, 10 ng/ml, 100 ng/ml, and 1 µg/ml, each in 100 µL, were applied and reacted at room temperature for 1 hour before measuring fluorescence.

### Example 2

Except for coating Fv-2 on a MaxiSorp plate using a solution containing Fv-2 at a concentration of 50 µg/ml, the experiment was conducted in the same manner as in Example 1, and fluorescence was measured.

### Example 3

Except for coating Fv-3 on a MaxiSorp plate using a solution containing Fv-3 at a concentration of 50 µg/ml, the experiment was conducted in the same manner as in Example 1, and fluorescence was measured.

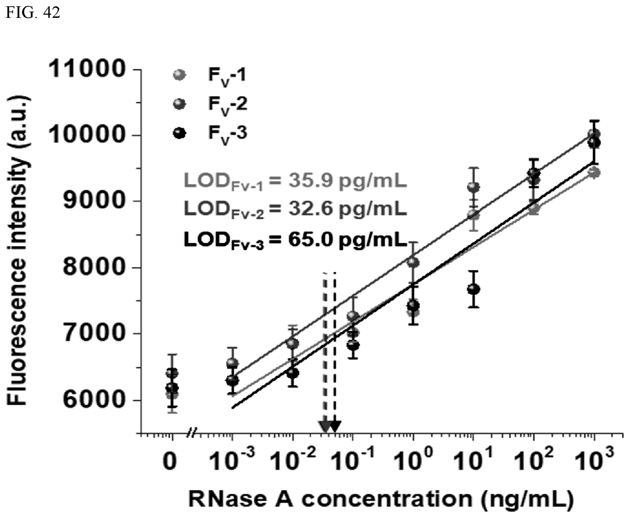

FIG. 42 shows the fluorescence measurement results according to Embodiments 1 to 3.

As shown in FIG. 42, it was found that the fluorescence intensity increases linearly with the concentration of RNase A included in the sample. This indicates that it is possible to quantitatively analyze the RNase A contained in the sample using the binding antibody coupled with the switching peptide.

Although the above has been described with reference to a preferred embodiment of the present invention, those skilled in the art will understand that the present invention can be variously modified and changed without departing from the spirit and scope of the invention as set forth in the following patent claims.

### [Description of Symbols]

1000, 2000: RNase detection immunoassay device
1100, 2100: Substrate
1200, 2200: Binding antibody
1300, 2300: Switching peptide

## Claims

1. An antibody or an antigen-binding fragment thereof that specifically binds to RNase A, comprising a CDR3 selected from the group consisting of a CDR3 comprising the amino acid sequence of SEQ ID NO: 5, a CDR3 comprising the amino acid sequence of SEQ ID NO: 6, and a CDR3 comprising the amino acid sequence of SEQ ID NO: 7.

2. The antibody or antigen-binding fragment thereof that specifically binds to RNase A of claim 1, wherein the antibody or antigen-binding fragment comprises a heavy chain variable region selected from the group consisting of:
a heavy chain variable region comprising a CDR1 comprising the amino acid sequence of SEQ ID NO: 3, a CDR2 comprising the amino acid sequence of SEQ ID NO: 4, and a CDR3 comprising the amino acid sequence of SEQ ID NO: 5;
a heavy chain variable region comprising a CDR1 comprising the amino acid sequence of SEQ ID NO: 3, a CDR2 comprising the amino acid sequence of SEQ ID NO: 4, and a CDR3 comprising the amino acid sequence of SEQ ID NO: 6; and
a heavy chain variable region comprising a CDR1 comprising the amino acid sequence of SEQ ID NO: 3, a CDR2 comprising the amino acid sequence of SEQ ID NO: 4, and a CDR3 comprising the amino acid sequence of SEQ ID NO: 7.

3. The antibody or antigen-binding fragment that specifically binds to RNase A of claim 2, wherein the antibody or antigen-binding fragment comprises a heavy chain variable region selected from the group consisting of:
a heavy chain variable region comprising the amino acid sequence of SEQ ID NO: 8;
a heavy chain variable region comprising the amino acid sequence of SEQ ID NO: 9; and
a heavy chain variable region comprising the amino acid sequence of SEQ ID NO: 10.

4. The antibody or antigen-binding fragment that specifically binds to RNase A of claim 1, wherein the antibody or antigen-binding fragment is in the form of a single domain antibody.

5. A peptide that specifically binds to RNase A, comprising an amino acid sequence selected from the group consisting of SEQ ID NO: 5, SEQ ID NO: 6, and SEQ ID NO: 7.

6. A nucleic acid encoding the antibody, antigen-binding fragment thereof, or peptide of any one of claims 1 to 5.

7. A recombinant expression vector comprising the nucleic acid according to claim 6.

8. A cell transformed with the recombinant expression vector according to claim 7.

9. The cell of claim 8, wherein the cell is selected from the group consisting of an animal cell, a plant cell, yeast, *E. coli,* and an insect cell.

10. An RNase A inhibitor comprising a peptide selected from the group consisting of the amino acid sequences of SEQ ID NO: 5, SEQ ID NO: 6, and SEQ ID NO: 7.

11. An analytical method involving RNA, comprising the step of treating RNA with the RNase A inhibitor according to claim 10.

12. The analytical method involving RNA of claim 11, wherein the analytical method involving RNA is selected from the group consisting of an RNA extraction method, an RNA purification method, polymerase chain reaction (PCR), a complementary DNA (cDNA) synthesis method, and a DNA expression method.

13. An RNase detection immunoassay device, comprising:
a substrate having a reaction space capable of accommodating a detection sample solution;
a binding antibody located within the reaction space and fixed to the substrate;
a switching peptide comprising a peptide compound reversibly bound to a Fab region of the binding antibody and a fluorescent label bound to the peptide compound; and
a quenching material disposed adjacent to the fluorescent label to quench fluorescence from the fluorescent label,
wherein the binding antibody specifically binds to RNase A and comprises a CDR3 selected from the group consisting of a CDR3 comprising the amino acid sequence of SEQ ID NO: 5, a CDR3 comprising the amino acid sequence of SEQ ID NO: 6, and a CDR3 comprising the amino acid sequence of SEQ ID NO: 7.

14. An RNase detection immunoassay device, comprising:
a substrate having a reaction space capable of accommodating a detection sample solution;
a support located within the reaction space;
a binding antibody coupled to the support;
a switching peptide comprising a peptide compound reversibly bound to a Fab region of the binding antibody and a fluorescent label coupled to the peptide compound; and
a quenching material disposed adjacent to the fluorescent label to quench fluorescence from the fluorescent label,
wherein the binding antibody specifically binds to RNase A and comprises a CDR3 selected from the group consisting of a CDR3 comprising the amino acid sequence of SEQ ID NO: 5, a CDR3 comprising the amino acid sequence of SEQ ID NO: 6, and a CDR3 comprising the amino acid sequence of SEQ ID NO: 7.

15. The RNase detection immunoassay device of claim 13 or 14, wherein the binding antibody comprises a heavy chain variable region selected from the group consisting of:
a heavy chain variable region comprising a CDR1 comprising the amino acid sequence of SEQ ID NO: 3, a CDR2 comprising the amino acid sequence of SEQ ID NO: 4, and a CDR3 comprising the amino acid sequence of SEQ ID NO: 5;
a heavy chain variable region comprising a CDR1 comprising the amino acid sequence of SEQ ID NO: 3, a CDR2 comprising the amino acid sequence of SEQ ID NO: 4, and a CDR3 comprising the amino acid sequence of SEQ ID NO: 6; and
a heavy chain variable region comprising a CDR1 comprising the amino acid sequence of SEQ ID NO: 3, a CDR2 comprising the amino acid sequence of SEQ ID NO: 4, and a CDR3 comprising the amino acid sequence of SEQ ID NO: 7.

16. The RNase detection immunoassay device of claim 15, wherein the peptide compound has an amino acid sequence capable of specifically and reversibly binding to one or more of the first to fourth framework regions of a light chain or a heavy chain of the binding antibody.

17. The RNase detection immunoassay device of claim 15, wherein the binding antibody comprises a heavy chain variable region selected from the group consisting of:
a heavy chain variable region comprising the amino acid sequence of SEQ ID NO: 8;
a heavy chain variable region comprising the amino acid sequence of SEQ ID NO: 9; and
a heavy chain variable region comprising the amino acid sequence of SEQ ID NO: 10.

18. The RNase detection immunoassay device of claim 13 or 14, wherein the binding antibody is in the form of a single domain antibody.

19. The RNase detection immunoassay device of claim 13 or 14, further comprising:
a light source for irradiating the analytical sample solution in the reaction space of the substrate with light; and
an image analysis device for receiving fluorescence generated from the switching peptide released from the binding antibody, generating an image thereof, and analyzing the image to quantify the amount of switching peptide released from the binding antibody.

20. The RNase detection immunoassay device of claim 13 or 14, wherein the fluorescent label comprises one or more selected from the group consisting of rhodamine and its derivatives, fluorescein and its derivatives, coumarin and its derivatives, acridine and its derivatives, pyrene and its derivatives, erythrosine and its derivatives, eosin and its derivatives, 4-acetamido-4'-isothiocyanatostilbene-2,2'disulfonic acid, fluorescein isothiocyanate (FITC), Oregon Green, Alex Fluor, carboxyfluorescein (FAM), 6-carboxy-4',5'-dichloro-2',7'-dimethoxyfluorescein (JOE), carboxy-X-rhodamine (ROX), 6-carboxy-2',4,4',5',7,7'-hexachlorofluorescein (HEX), Texas Red (sulforhodamine 101 acid chloride), 6-carboxy-2',4,7',7-tetrachlorofluorescein (TET), tetramethylrhodamine-isothiocyanate (TRITC), carboxytetramethylrhodamine (TAMRA), cyanine dyes, and thiadicarbocyanine dyes.

21. The RNase detection immunoassay device of claim 20, wherein the quenching material comprises one or more selected from the group consisting of 4-(dimethylamino)azobenzene-4-carboxylic acid (DABCYL), 4-(dimethylamino)azobenzene sulfonic acid (DABSYL), blackhole quencher (BHQ), blackberry quencher (BBQ), ECLIPSE quencher, Tide quencher, carbon nanomaterials, and manganese dioxide nanomaterials.

22. An RNase detection immunoassay method, comprising:
a first step of fixing a binding antibody having a switching peptide coupled thereto to a substrate, or fixing the binding antibody to the substrate and then coupling the switching peptide to the binding antibody, and coupling a quenching material to the binding antibody or the substrate;
a second step of treating the binding antibody with a detection sample solution; and
a third step of, after the treating, irradiating the detection sample solution with light and quantitatively analyzing a target antigen in the detection sample solution via fluorescence generated from a fluorescent label of the switching peptide released from the binding antibody,
wherein the binding antibody specifically binds to RNase A and comprises a CDR3 selected from the group consisting of a CDR3 comprising the amino acid sequence of SEQ ID NO: 5, a CDR3 comprising the amino acid sequence of SEQ ID NO: 6, and a CDR3 comprising the amino acid sequence of SEQ ID NO: 7, and
wherein the switching peptide comprises a peptide compound having an amino acid sequence capable of selectively and reversibly binding to a Fab (Fragment antigen-binding) region of the binding antibody, and a fluorescent label coupled thereto that emits fluorescence.

23. An RNase detection immunoassay method, comprising:
a first step of fixing a binding antibody having a switching peptide coupled thereto to a support, or fixing the binding antibody to the support and then coupling the switching peptide to the binding antibody, and coupling a quenching material to the binding antibody or the support;
a second step of injecting the support having the binding antibody coupled thereto and a detection sample solution into a reaction space of a substrate; and
a third step of, after irradiating the detection sample solution with light, quantitatively analyzing a target antigen in the detection sample solution via fluorescence generated from a fluorescent label of the switching peptide released from the binding antibody,
wherein the binding antibody specifically binds to RNase A and comprises a CDR3 selected from the group consisting of a CDR3 comprising the amino acid sequence of SEQ ID NO: 5, a CDR3 comprising the amino acid sequence of SEQ ID NO: 6, and a CDR3 comprising the amino acid sequence of SEQ ID NO: 7, and
wherein the switching peptide comprises a peptide compound having an amino acid sequence capable of selectively and reversibly binding to a Fab (Fragment antigen-binding) region of the binding antibody, and a fluorescent label coupled thereto that emits fluorescence.

24. The RNase A detection immunoassay method of claim 22 or 23, wherein, when RNase A is present in the detection sample solution in the second step, the switching peptide is quantitatively released from the binding antibody dependent on the amount of the RNase A reacted with the binding antibody when the RNase A binds to the binding antibody.

25. A method for capturing RNase A, comprising:
mixing an RNase A-containing sample with beads having an antibody or peptide that specifically binds to RNase A fixed to a surface thereof to induce a reaction between the RNase A and the antibody or peptide; and
collecting the beads from the sample;
wherein the binding antibody specifically binds to RNase A and comprises a CDR3 selected from the group consisting of a CDR3 comprising the amino acid sequence of SEQ ID NO: 5, a CDR3 comprising the amino acid sequence of SEQ ID NO: 6, and a CDR3 comprising the amino acid sequence of SEQ ID NO: 7.

26. The method for capturing RNase A of claim 25, wherein the beads comprise nickel (Ni), copper (Cu), zinc (Zn), or cobalt (Co) containing magnetic particles, the surface of which is modified with a chelating group such as nitrilotriacetic acid (NTA) or iminodiacetic acid (IDA).

27. The method for capturing RNase A of claim 26, wherein the beads are formed of a magnetic material, and the beads are collected from the sample using a magnetic field.
